# EUROPEAN PATENT APPLICATION

(11) **EP 4 721 685 A1**
(43) Date of publication of application: **08.04.2026**
(21) Application number: 24815878.4
(22) Date of filing: 30.05.2024
(51) Int. Cl.: A61B 18/14, A61B 17/29, A61B 34/30, A61B 18/00

(54) **INSTRUMENT FOR ELECTROCAUTERY SURGERY**

(30) Priority: 02.06.2023 KR 20230071954; 04.04.2024 KR 20240046142
(71) Applicant: Livsmed Inc., Seongnam-si, Gyeonggi-do 13516 (KR)
(72) Inventor: LEE, Jae Yeong, Seongnam-si, Gyeonggi-do 13504 (KR); KIM, Hee Jin, Seoul 06998 (KR); KIM, Zwa Nyun, Yongin-si, Gyeonggi-do 16826 (KR); KIM, Byung Ho, Seongnam-si, Gyeonggi-do 13376 (KR)
(74) Representative: AWA Sweden AB
(86) International application number: PCT/KR2024/007406
(87) International publication number: WO 2024/248504

(57) **Abstract**

Provided is a surgical instrument, and more particularly, a surgical instrument for electrocautery with improved insulation performance, capable of being mounted on a robot arm or operated manually for use in laparoscopic surgery or various other surgeries.

## Description

### CROSS-REFERENCE TO RELATED APPLICATION

This application is based on and claims priority under 35 U.S.C. §119 to Korean Patent Application No. 10-2023-0071954, filed on, Month Day 2025, in the Korean Intellectual Property Office, the disclosure of which is incorporated by reference herein in its entirety.

### BACKGROUND

### 1. Field

The present disclosure relates to a surgical instrument, and more particularly, to a surgical instrument for electrocautery with improved insulation performance, capable of being mounted on a robot arm or operated manually for use in laparoscopic surgery or various other surgeries.

### 2. Description of the Related Art

Surgical procedures in many cases require cutting and joining of body tissues including organs, muscular tissues, connective tissues, and blood vessels. Over the centuries, sharp blades and sutures have been used for cutting and joining. However, bleeding occurs when cutting body tissues, in particular, relatively highly vascularized tissue during surgical procedures. Accordingly, surgeons have sought surgical instruments and methods that can slow down or reduce bleeding during surgical procedures.

Recently, it has become possible to use electrosurgical instruments that use electrical energy to perform certain surgical tasks. For example, electrosurgical instruments equipped with one or more electrodes configured to supply electrical energy have been developed for use with surgical tools such as graspers, scissors, forceps, blades, needles, and hooks. Electrical energy supplied through the electrodes can be used to coagulate, join, or cut the patient's body tissues. In particular, when electrical energy is used, cutting and hemostasis may be performed at the same time.

Electrosurgical instruments are typically classified into two types, monopolar and bipolar. In a monopolar electrosurgical instrument, electrical energy of a specific polarity is supplied to one or more electrodes of the instrument. In an embodiment, electricity of different polarity is electrically connected to a patient. In a bipolar electrosurgical instrument, one or more electrodes are electrically connected to a first polarity electrical energy source, and one or more other electrodes are electrically connected to a second polarity electrical energy source opposite to the first polarity electrical energy source.

The background art described above is technical information retained by the present inventors in order to derive the present disclosure or obtained by the present inventors in the process of deriving the present disclosure, and thus is not necessarily known art disclosed to the general public before the filing of the present application.

### SUMMARY

The present disclosure is directed to providing a surgical instrument for electrocautery that may be mounted on a robotic arm or operated manually for use in laparoscopic surgery or various other surgeries, and includes an end tool that is rotatable in two or more directions and operates in a way that intuitively matches a motion of a manipulation part.

According to an aspect of the present disclosure, there is provided a surgical instrument including an end tool including a first jaw and a second jaw, each configured to be rotatable, the end tool being configured to be rotatable in two or more directions, a manipulation part configured to control the rotation of the end tool in the two or more directions, a power transmission part including a first jaw wire connected to the manipulation part and configured to transmit a rotation of the manipulation part to the first jaw, and a second jaw wire connected to the manipulation part and configured to transmit the rotation of the manipulation part to the second jaw, and a connection part configured to extend in a first direction (X-axis) and having one end portion to which the end tool is coupled and another end portion to which the manipulation part is coupled, thereby connecting the manipulation part to the end tool, wherein the end tool further includes a first jaw pulley coupled to the first jaw and configured to be rotatable around a first rotation shaft, a second jaw pulley coupled to the second jaw and configured to be rotatable around a shaft that is substantially identical to or parallel to the first rotation shaft, and a blade assembly including a blade that moves between a proximal end and a distal end of the end tool, and positioned adjacent to the first jaw pulley or the second jaw pulley, the power transmission part further includes a blade wire, at least a portion of which is in contact with the blade assembly, to transmit to the blade a driving force required to move the blade, the manipulation part includes a cutting-manipulation portion including a cutting lever configured to rotate around a rotation shaft, and opposite end portions of the blade wire are respectively connected to the cutting-manipulation portion and the blade, such that a rotational motion of the cutting-manipulation portion is converted into a linear motion of the blade through the blade wire.

An embodiment of the present disclosure provides a surgical instrument including an end tool including a first jaw and a second jaw, each configured to be rotatable, the end tool being configured to be rotatable in two or more directions, a manipulation part configured to control the rotation of the end tool in the two or more directions, a power transmission part including a first jaw wire connected to the manipulation part and configured to transmit a rotation of the manipulation part to the first jaw, and a second jaw wire connected to the manipulation part and configured to transmit the rotation of the manipulation part to the second jaw, and a connection part configured to extend in a first direction (X-axis) and having one end portion to which the end tool is coupled and another end portion to which the manipulation part is coupled, thereby connecting the manipulation part to the end tool, wherein the end tool further includes a first jaw pulley coupled to the first jaw and configured to be rotatable around a first rotation shaft, a second jaw pulley coupled to the second jaw and configured to be rotatable around a shaft that is substantially identical to or parallel to the first rotation shaft, and a blade assembly including a blade that moves between a proximal end and a distal end of the end tool, and positioned adjacent to the first jaw pulley or the second jaw pulley, the power transmission part further includes a blade wire, at least a portion of which is in contact with the blade assembly, to transmit to the blade a driving force required to move the blade, the manipulation part includes a cutting-manipulation portion including a cutting lever configured to rotate around a rotation shaft, and opposite end portions of the blade wire are respectively connected to the cutting-manipulation portion and the blade, such that a rotational motion of the cutting-manipulation portion is converted into a linear motion of the blade through the blade wire.

In an embodiment of the present disclosure, the cutting-manipulation portion may include a cutting-relay member configured to rotate around a rotation shaft different from the rotation shaft around which the cutting lever rotates and connected to the cutting lever by a cutting-lever wire, and a cutting-relay assembly configured to be connected to the cutting-relay member and to convert a rotational motion of the cutting-relay member into a linear motion, and the rotational motion of the cutting-manipulation portion may be converted into a linear motion of the blade wire through the cutting-relay assembly.

In an embodiment of the present disclosure, the cutting-lever wire may generate the rotational motion of the cutting-relay member by transmitting a rotation of the cutting lever to the cutting-relay member.

In an embodiment of the present disclosure, the cutting-manipulation portion may further include a cutting-lever tube configured to internally accommodate at least a portion of the cutting-lever wire and to be bendable to a certain extent.

In an embodiment of the present disclosure, the cutting-lever tube may have one end portion fixed to one side of the cutting lever, and another end portion fixed to one side of the cutting-relay member, and the cutting-lever wire may be further configured to be movable along and within the cutting-lever tube.

In an embodiment of the present disclosure, the cutting-lever wire may include a first cutting-lever wire and a second cutting-lever wire, the first cutting-lever wire may be wound in the first direction around a cutting-lever rotation shaft and then wound in the first direction around the cutting-relay member, and the second cutting-lever wire may be wound in a second direction, which is a direction opposite to the first direction, around the cutting-lever rotation shaft and then wound in the second direction around the cutting-relay member.

In an embodiment of the present disclosure, according to an operation of the cutting lever, when the first cutting-lever wire is wound around the cutting-relay member, the second cutting-lever wire may be unwound from the cutting-relay member, and when the first cutting-lever wire is unwound from the cutting-relay member, the second cutting-lever wire may be wound around the cutting-relay member.

In an embodiment of the present disclosure, the cutting-relay assembly may include a cutting-relay wire connected to the cutting-relay member, and a cutting-relay block coupled to the cutting-relay wire at at least one region, and one end portion of the cutting-relay wire may be fixed to the cutting-relay member, and at least one region of the cutting-relay wire may be connected to a pulley, thereby forming a loop-shaped path.

In an embodiment of the present disclosure, the cutting-relay wire may have a movement path including at least one linear section along which the cutting-relay wire moves in a straight-line direction.

In an embodiment of the present disclosure, the cutting-relay block may move within the linear section toward either a distal end side or a proximal end side of the manipulation part.

In an embodiment of the present disclosure, the blade assembly may further include a guide tube positioned in the connection part and configured to be bendable to a certain extent, and at least a portion of the blade wire is accommodated in the guide tube.

In an embodiment of the present disclosure, the blade wire may pass through an interior of the guide tube, and may have one end portion connected to one surface of the cutting-relay block at the distal end side and another end portion connected to the blade.

In an embodiment of the present disclosure, a movement path of the blade wire may be guided by the guide tube.

In an embodiment of the present disclosure, the guide tube may have opposite ends respectively fixed to the end tool and the manipulation part, and is bendable between the opposite ends.

In an embodiment of the present disclosure, the blade may move linearly in a direction in which the connection part extends.

In an embodiment of the present disclosure, the end tool may be configured, in response to a control of the manipulation part, to perform a pitch motion, a yaw motion, and an actuation motion, and to linearly move the blade.

In an embodiment of the present disclosure, the end tool may be configured to perform at least one motion among the pitch motion, the yaw motion, and the actuation motion, and to linearly move the blade either independently of or simultaneously with the at least one motion.

In an embodiment of the present disclosure, the surgical instrument may further include a guide tube configured to internally accommodate at least a portion of the blade wire and to be bendable to a certain extent, and an actuation hub positioned between the first jaw and the second jaw, coupled to the first jaw and the second jaw, and having a hollow provided therein to which one end portion of the guide tube is coupled.

In an embodiment of the present disclosure, the guide tube and the at least a portion of the blade wire accommodated therein may be configured to pass through the hollow provided in the actuation hub.

In an embodiment of the present disclosure, the guide tube may not be directly coupled to the first jaw or the second jaw.

In an embodiment of the present disclosure, the first jaw and the second jaw may be positioned to cross each other about a first point serving as a rotation center, and rotation centers of the first jaw pulley and the second jaw pulley may be provided at a second point that is positioned to be spaced apart from the first point.

In an embodiment of the present disclosure, the first point may be positioned more distally than the second point on the first jaw and the second jaw.

In an embodiment of the present disclosure, the first rotation shaft may include a first sub-shaft and a second sub-shaft, the first sub-shaft and the second sub-shaft may be positioned to be spaced apart from each other to a certain extent, and the blade assembly may be partially accommodated between the first sub-shaft and the second sub-shaft.

In an embodiment of the present disclosure, the first jaw and the second jaw may perform an actuation motion by rotating around an actuation rotation shaft of the end tool, and the manipulation part may further include an actuation-manipulation portion configured to control an actuation motion of the end tool, wherein the actuation-manipulation portion may include an actuation pulley configured to rotate around one actuation rotation shaft, and an actuation lever coupled to the actuation pulley and configured to rotate integrally with the actuation pulley, and the one actuation rotation shaft of the manipulation part may not be parallel to the actuation rotation shaft of the end tool.

In an embodiment of the present disclosure, the first jaw wire and the second jaw wire may be wound around and fixed to the actuation pulley, and the first jaw and the second jaw may rotate in opposite directions in response to the rotation of the actuation pulley.

In an embodiment of the present disclosure, each of the first jaw wire and the second jaw wire may include a pair of wire strands, one of the pair of wire strands of the first jaw wire may have one end portion wound in the first direction around the actuation pulley and another end portion wound in a third direction around the first jaw pulley, the other of the pair of wire strands of the first jaw wire may have one end portion wound in a second direction, which is opposite to the first direction, around the actuation pulley and another end portion wound in a fourth direction, which is opposite to the third direction, around the first jaw pulley, one of the pair of wire strands of the second jaw wire may have one end portion wound in the second direction around the actuation pulley and another end portion wound in the third direction around the second jaw pulley, and the other of the pair of wire strands of the second jaw wire may have one end portion wound in the first direction around the actuation pulley and another end portion wound in the fourth direction around the second jaw pulley.

In an embodiment of the present disclosure, the manipulation part may further include a sealing-manipulation portion provided on one side of the cutting-manipulation portion and configured to be rotatable around one rotation shaft.

In an embodiment of the present disclosure, the sealing-manipulation portion may include a sealing button configured to protrude from one end portion thereof, and when the sealing button is pressed, a contact portion provided at another end portion of the sealing-manipulation portion may come into contact with another contact portion within the manipulation part, thereby transmitting an electrical signal.

In an embodiment of the present disclosure, a first electrode may be provided on a surface of the first jaw facing the second jaw, and a second electrode may be provided on a surface of the second jaw facing the first jaw.

In an embodiment of the present disclosure, cauterization of a tissue may be performed as a current flows through the first electrode and the second electrode.

In an embodiment of the present disclosure, after completion of the cauterization, the blade wire may move, and accordingly, the blade may move from a proximal end side toward a distal end side of the first jaw to cut the tissue.

In an embodiment of the present disclosure, at least a portion of the manipulation part may extend toward the end tool.

In an embodiment of the present disclosure, when the manipulation part is rotated in the two or more directions, the end tool may rotate in directions substantially the same as manipulation directions of the manipulation part.

In an embodiment of the present disclosure, a direction in which the end tool is provided at the one end portion of the connection part and a direction in which the manipulation part is provided at the other end portion of the connecting part may be identical directions with respect to an extending axis (X-axis) of the connection part.

In an embodiment of the present disclosure, the manipulation part may be configured to extend in a direction away from a user who grips the surgical instrument.

In an embodiment of the present disclosure, an end portion of the manipulation part may be formed toward the end tool so that an end portion of a finger of a user gripping the manipulation part faces the end tool.

In an embodiment of the present disclosure, the connection part may include a bent portion that is configured to be bent once or more while connecting the end tool to the manipulation part.

In an embodiment of the present disclosure, the bent portion may have a cross section provided as an approximately semi-circular shape, and a direction in which the manipulation part is provided at an end portion of the bent portion and a direction in which the end tool is provided at a point where the connection part and the end tool are connected may be provided to be substantially identical.

In an embodiment of the present disclosure, at least a portion of the manipulation part may be configured to be accommodated in the bent portion during at least one movement of the manipulation part.

An embodiment of the present disclosure provides a manipulation part of a surgical instrument, the manipulation part including a handle, an actuation-manipulation portion provided at one side of the handle and configured to control an actuation motion of an end tool including a first jaw and a second jaw, a cutting-manipulation portion provided adjacent to the actuation-manipulation portion and including a cutting lever rotatable around a cutting-lever rotation shaft, a cutting-relay member configured to rotate around a rotation shaft different from the cutting-lever rotation shaft and connected to the cutting lever via a cutting-lever wire, a cutting-relay assembly configured to be connected to the cutting-relay member and to convert a rotational motion of the cutting-relay member into a linear motion, and a blade wire configured to transmit a driving force to a blade, wherein a rotational motion of the cutting-manipulation portion is converted into a linear motion of the blade wire through the cutting-relay assembly.

In an embodiment of the present disclosure, the cutting-relay member may rotate in response to the rotation of the cutting lever and may mediate between the rotational motion of the cutting lever and the motion of the cutting-relay assembly.

In an embodiment of the present disclosure, the cutting-lever wire may generate the rotational motion of the cutting-relay member by transmitting the rotation of the cutting lever to the cutting-relay member.

In an embodiment of the present disclosure, the cutting-manipulation portion may include a cutting-lever tube configured to internally accommodate at least a portion of the cutting-lever wire and to be bendable to a certain extent.

In an embodiment of the present disclosure, the cutting-lever wire may be connected to the cutting-relay member by passing through an interior of the cutting-lever tube.

In an embodiment of the present disclosure, when the cutting-lever tube is bent to a certain extent, the cutting-lever wire inside the cutting-lever tube may also be bent together with the cutting-lever tube.

In an embodiment of the present disclosure, the cutting-lever wire may be configured to be movable along and within the cutting-lever tube.

In an embodiment of the present disclosure, one end portion of the cutting-lever tube may be fixed to one side of the cutting lever, and another end portion of the cutting-lever tube may be fixed to one side of the cutting-relay member.

In an embodiment of the present disclosure, the manipulation part may further include a yaw-manipulation portion configured to be connected to the handle, to rotate around a yaw rotation shaft by an operation of the handle, and to control a yaw motion of the end tool, and the cutting-lever tube may be provided as a pair of cutting-lever tubes, which may cross each other below the yaw rotation shaft.

In an embodiment of the present disclosure, the cutting lever may rotate around the cutting-lever rotation shaft, and may have one end portion with respect to the cutting-lever rotation shaft and another end portion connected to the cutting-lever wire, the one end portion serving as a body portion for manipulating the cutting lever.

In an embodiment of the present disclosure, the cutting-lever wire may include a first cutting-lever wire and a second cutting-lever wire, the first cutting-lever wire may be wound in a first direction around the cutting lever and then wound in the first direction around the cutting-relay member, and the second cutting-lever wire may be wound in a second direction opposite to the first direction around the cutting lever and then wound in the second direction around the cutting-relay member.

In an embodiment of the present disclosure, one end portion of the first cutting-lever wire may be fixed to one side surface of the cutting lever with respect to a plane perpendicular to the cutting-lever rotation shaft, another end portion of the first cutting-lever wire may be fixed to another side surface of the cutting-relay member, one end portion of the second cutting-lever wire may be fixed to another side surface of the cutting lever with respect to the plane perpendicular to the cutting-lever rotation shaft, and another end portion of the second cutting-lever wire may be fixed to one side surface of the cutting-relay member.

In an embodiment of the present disclosure, according to an operation of the cutting lever, when the first cutting-lever wire is wound around the cutting-relay member the second cutting-lever wire may be unwound from the cutting-relay member, and when the first cutting-lever wire is unwound from the cutting-relay member, the second cutting-lever wire may be wound around the cutting-relay member.

In an embodiment of the present disclosure, the cutting relay member may include a first cutting-relay pulley, a second cutting-relay pulley, and a third cutting-relay pulley that rotate around the same rotational shaft, wherein the third cutting-relay pulley may be positioned at the center of the cutting-relay member, and the first cutting-relay pulley and the second cutting-relay pulley may be positioned on opposite sides with respect to the third cutting-relay pulley.

In an embodiment of the present disclosure, the first cutting-relay pulley, the second cutting-relay pulley, and the third cutting-relay pulley may be integrally provided as one body and rotatable in the same direction.

In an embodiment of the present disclosure, the cutting-relay assembly may include a cutting-relay wire connected to the cutting-relay member, and a cutting-relay block coupled to at least one region of the cutting-relay wire, and the cutting-relay wire may have one end portion fixed to the cutting-relay member and at least one region connected to a pulley, thereby forming a loop-shaped path.

In an embodiment of the present disclosure, the cutting-relay wire may be a single wire, both end portions of which are connected to the cutting-relay member, and the cutting-relay block may be coupled to a portion of the cutting-relay wire.

In an embodiment of the present disclosure, the cutting-relay wire may include a first cutting-relay wire and a second cutting-relay wire, wherein the first cutting-relay wire may have one end portion connected to the cutting-relay member and another end portion connected to one side surface of the cutting-relay block, and the second cutting-relay wire may have one end portion connected to the cutting-relay member and another end portion is connected to the opposite side surface of the cutting-relay block.

In an embodiment of the present disclosure, the cutting-relay wire may have a movement path including at least one linear section along which the cutting-relay wire moves in a straight-line direction.

In an embodiment of the present disclosure, the cutting-relay block may move within the linear section toward either a distal end side or a proximal end side of the manipulation part.

In an embodiment of the present disclosure, one end portion of the blade wire may be connected to one surface of the cutting-relay block at the distal end side, and at least a portion of the blade wire may be accommodated within a guide tube.

In an embodiment of the present disclosure, a movement path of the blade wire may be guided by the guide tube.

In an embodiment of the present disclosure, the guide tube may be fixed in position by being coupled to one or more fixing members configured to fix the guide tube, and may form a linear section within a region coupled to the one or more fixing members.

In an embodiment of the present disclosure, the cutting-relay assembly may further include a cutting restoring elastic member having one end portion fixed to the cutting-relay block, and the cutting restoring elastic member may apply a force that pulls the cutting-relay block toward a proximal end side.

An embodiment of the present disclosure provides a manipulation part of a surgical instrument, the manipulation part including a handle, an actuation-manipulation portion provided at one side of the handle and configured to control an actuation motion of an end tool including a first jaw and a second jaw, and a cutting-manipulation portion positioned adjacent to the actuation-manipulation portion and including a cutting lever rotatable around a rotation shaft, wherein the actuation-manipulation portion includes an actuation pulley rotatable around one actuation rotation shaft, and an actuation lever coupled to and configured to rotate integrally with the actuation pulley, the actuation rotation shaft of the manipulation part is not parallel to an actuation rotation shaft of the end tool, and a rotation of the actuation pulley in one direction by the actuation lever allows opening and closing motions of the first jaw and the second jaw.

In an embodiment of the present disclosure, the manipulation part may further include a yaw-manipulation portion configured to be connected to the handle and to control a yaw motion of the end tool, and a pitch-manipulation portion configured to be connected to the yaw-manipulation portion and to control a pitch motion of the end tool, wherein the yaw-manipulation portion may rotate around a yaw rotation shaft in response to a manipulation of the handle, the pitch-manipulation portion may rotate around a pitch rotation shaft in response to the manipulation of the handle, and when the manipulation part is rotated in two or more directions, the end tool may rotate in a direction substantially identical to a manipulation direction of the manipulation part.

In an embodiment of the present disclosure, a rotation shaft of the actuation pulley may be parallel to the pitch rotation shaft, and the actuation lever may rotate in a direction toward or away from the handle.

In an embodiment of the present disclosure, the first jaw and the second jaw of the end tool may be opened or closed as the actuation lever rotates toward or away from the handle.

In an embodiment of the present disclosure, the actuation-manipulation portion may include a first jaw wire configured to transmit the rotation of the actuation-manipulation portion to the first jaw, and a second jaw wire configured to transmit the rotation of the actuation-manipulation portion to the second jaw, and the first jaw wire and the second jaw wire may be wound around and fixed to the actuation pulley.

In an embodiment of the present disclosure, the first jaw wire and the second jaw wire may each include a pair of wires, one of which is wound around the actuation pulley in a first direction, and another one of which is wound in a second direction opposite to the first direction around the actuation pulley.

In an embodiment of the present disclosure, the first jaw wire may include a pair of wires including a first strand and a second strand, and the second jaw wire may include a pair of wires including a third strand and a fourth strand, wherein the first strand may be wound in a first direction around the actuation pulley, the second strand may be wound in a second direction opposite to the first direction around the actuation pulley, the third strand may be wound in the first direction around the actuation pulley, and the fourth strand may be wound in the second direction opposite to the first direction around the actuation pulley.

In an embodiment of the present disclosure, the first strand of the first jaw wire and the fourth strand of the second jaw wire may be positioned on one side with respect to a plane perpendicular to a rotation shaft of the actuation pulley, and the second strand of the first jaw wire and the third strand of the second jaw wire may be disposed on another side with respect to the plane perpendicular to the rotation shaft of the actuation pulley.

In an embodiment of the present disclosure, the actuation-manipulation portion may further include an actuation restoring elastic member, and the actuation restoring elastic member may apply a force to the actuation lever so that the actuation lever moves away from the handle.

Other aspects, features, and advantages other than those described above will become apparent from the following drawings, claims, and detailed description of the disclosure.

### BRIEF DESCRIPTION OF THE DRAWINGS

The above and other aspects, features, and advantages of certain embodiments of the disclosure will be more apparent from the following description taken in conjunction with the accompanying drawings, in which:

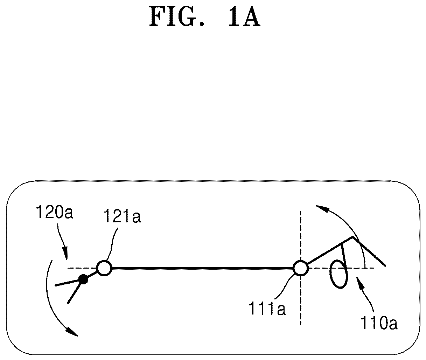

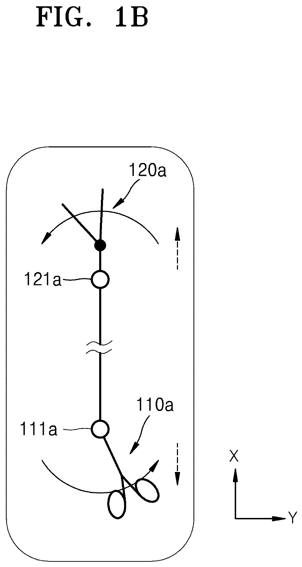

FIG. 1A is a conceptual diagram of a pitch motion of a conventional surgical instrument, and FIG. 1B is a conceptual diagram of a yaw motion;

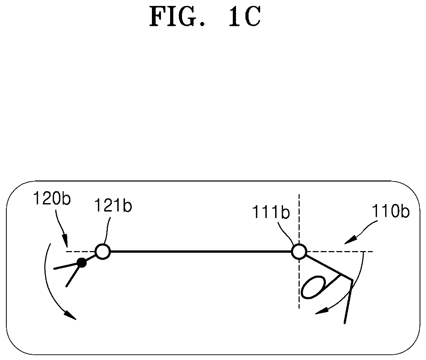

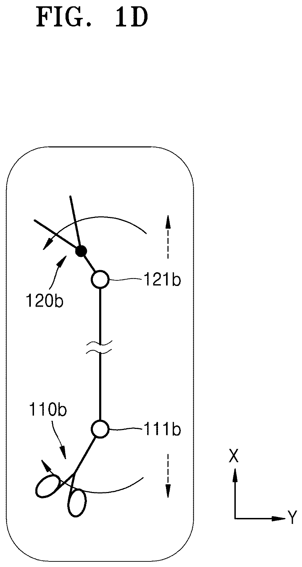

FIG. 1C is a conceptual diagram of a pitch motion of another conventional surgical instrument, and FIG. 1D is a conceptual diagram of a yaw motion;

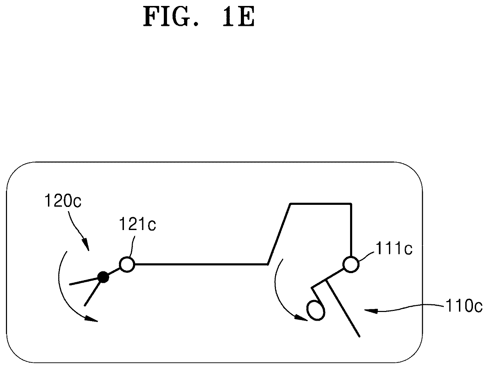

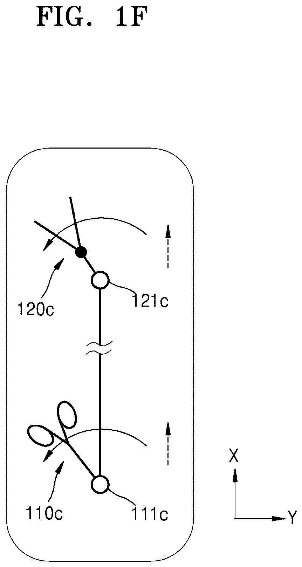

FIG. 1E is a conceptual diagram of a pitch motion of a surgical instrument according to the present disclosure, and FIG. 1F is a conceptual diagram of a yaw motion;
FIG. 2 is a perspective view illustrating a surgical instrument for electrocautery according to an embodiment of the present disclosure;
FIGS. 3 to 8 are views illustrating an end tool of the surgical instrument for electrocautery of FIG. 2;
FIG. 9 is a perspective view illustrating an end tool hub of the surgical instrument for electrocautery of FIG. 2;
FIGS. 10 and 11 are cutaway perspective views of the end tool hub of FIG. 9;
FIGS. 12 and 13 are perspective views illustrating the end tool hub of FIG. 9;
FIG. 14 is a side view illustrating the end tool hub of FIG. 9 and a guide tube;
FIG. 15 is a plan view illustrating the end tool hub of FIG. 9 and the guide tube;
FIG. 16 is a set of a perspective view and a cutaway perspective view illustrating an actuation hub of the surgical instrument for electrocautery of FIG. 2;
FIG. 17 is a view illustrating a state in which the guide tube, a blade wire, and a blade are mounted on the actuation hub illustrated in the cutaway perspective view of FIG. 16;
FIG. 18 is an exploded perspective view illustrating the end tool of the surgical instrument for electrocautery of FIG. 2;
FIG. 19 is a perspective view illustrating a first jaw of the end tool of the surgical instrument for electrocautery of FIG. 2;
FIG. 20 is a perspective view illustrating a second jaw of the end tool of the surgical instrument for electrocautery of FIG. 2;
FIG. 21 is a perspective view illustrating a first jaw pulley of the surgical instrument for electrocautery of FIG. 2;
FIG. 22 is a plan view illustrating an opening and closing motion of the first jaw of the end tool of the surgical instrument for electrocautery of FIG. 2;
FIG. 23 is a plan view illustrating an opening and closing motion of the second jaw of the end tool of the surgical instrument for electrocautery of FIG. 2;
FIG. 24 is a plan view illustrating an opening and closing motion of the first jaw and the second jaw of the end tool of the surgical instrument for electrocautery of FIG. 2;
FIGS. 25 and 26 are plan views illustrating an opening and closing motion of the end tool of the surgical instrument for electrocautery of FIG. 2;
FIGS. 27 to 29 are partial cross-sectional views illustrating the operation of the blade of the end tool of the surgical instrument for electrocautery of FIG. 2;
FIGS. 30 and 31 are views illustrating a process of performing an opening and closing motion in a state in which the end tool of the surgical instrument for electrocautery of FIG. 2 is yaw-rotated by -90°;
FIGS. 32 and 33 are views illustrating a process of performing an opening and closing motion in a state in which the end tool of the surgical instrument for electrocautery of FIG. 2 is yaw-rotated by +90°;
FIGS. 34 and 35 are views illustrating a path of the guide tube and a movement path of a blade during a cutting motion in a state in which the end tool of the surgical instrument for electrocautery of FIG. 2 is yaw-rotated;
FIGS. 36 and 37 are views illustrating a process of performing an opening and closing motion in a state in which the end tool of the surgical instrument for electrocautery of FIG. 2 is pitch-rotated by +90°;
FIGS. 38 and 39 are views illustrating a process of performing an opening and closing motion in a state in which the end tool of the surgical instrument for electrocautery of FIG. 2 is pitch-rotated by -90°;
FIG. 40 is a view illustrating a path of the guide tube in a state in which the end tool of the surgical instrument for electrocautery of FIG. 2 is pitch-rotated by -90°;
FIGS. 41 and 42 are views illustrating a path of the guide tube and a movement path of the blade during a cutting motion in a state in which the end tool of the surgical instrument for electrocautery of FIG. 2 is pitch-rotated by -90°;
FIG. 43 is a perspective view illustrating the surgical instrument for electrocautery of FIG. 2 in a pitch- and yaw-rotated state;
FIGS. 44 to 46 are views illustrating the end tool of the surgical instrument for electrocautery of FIG. 2 performing a cutting motion in a state in which the end tool is pitch-rotated by -90° and simultaneously yaw-rotated by +90°;
FIG. 47 is a perspective view illustrating the surgical instrument for electrocautery of FIG. 2;
FIGS. 48 and 49 are perspective views illustrating a manipulation part of the surgical instrument for electrocautery of FIG. 2;
FIG. 50 is a view schematically illustrating only a configuration of pulleys and wires that constitute joints of the surgical instrument for electrocautery of FIG. 2;
FIG. 51 is a perspective view illustrating a yaw motion of the surgical instrument for electrocautery of FIG. 47;
FIGS. 52 and 53 are diagrams illustrating a configuration of pulleys and wires, which are associated with an actuation motion and a yaw motion of the surgical instrument for electrocautery shown in FIG. 2, separately for each of the first jaw and the second jaw;
FIG. 54 is a perspective view illustrating a pitch motion of the surgical instrument for electrocautery of FIG. 2;
FIGS. 55 and 56 are views illustrating a configuration of pulleys and wires relating to a pitch motion of the surgical instrument for electrocautery shown in FIG. 2, separately for each of the first jaw and the second jaw;
FIGS. 57 to 60 are perspective views illustrating the operation of an actuation lever of the surgical instrument for electrocautery shown in FIG. 2;
FIGS. 61 and 62 are views illustrating the movement of wires during the operation of the actuation lever of the surgical instrument for electrocautery shown in FIG. 2;
FIGS. 63 and 64 are partial cross-sectional views illustrating an actuation restoring elastic member of the surgical instrument for electrocautery shown in FIG. 2;
FIGS. 65 and 66 are perspective views illustrating the operation of a sealing button of the surgical instrument for electrocautery shown in FIG. 2;
FIG. 67 is a perspective view illustrating a cutting-relay assembly of the surgical instrument for electrocautery shown in FIG. 2;
FIG. 68 is a perspective view illustrating a cutting-manipulation portion and the cutting-relay assembly of the surgical instrument for electrocautery shown in FIG. 2;
FIGS. 69 and 70 are views for describing the cutting principle of the surgical instrument for electrocautery shown in FIG. 2;
FIGS. 71 and 72 are perspective views illustrating a cutting lever operation of the surgical instrument for electrocautery shown in FIG. 2;
FIGS. 73 and 74 are views illustrating the movement of the cutting-relay assembly and the blade of the end tool during the cutting lever operation of the surgical instrument for electrocautery shown in FIG. 2;
FIGS. 75 and 76 are views schematically illustrating only components of the cutting-relay member and the cutting-relay assembly during the cutting lever operation of the surgical instrument for electrocautery shown in FIG. 2;
FIGS. 77 and 78 are views illustrating the cutting-manipulation portion and wires during the cutting lever operation of the surgical instrument for electrocautery shown in FIG. 2;
FIGS. 79 and 80 are views illustrating a path of the guide tube in the end tool and the connection part of the surgical instrument for electrocautery shown in FIG. 2;
FIG. 81 is a view illustrating a path of the guide tube in the end tool and the manipulation part when the end tool of the surgical instrument for electrocautery shown in FIG. 2 is yaw-rotated by +90°;
FIG. 82 is a view illustrating a path of the guide tube in the end tool and the manipulation part when the end tool of the surgical instrument for electrocautery shown in FIG. 2 is yaw-rotated by -90°;
FIGS. 83 and 84 are perspective views illustrating a pitch motion of the surgical instrument for electrocautery shown in FIG. 2; and
FIG. 85 is a perspective view illustrating a yaw-pitch combined motion of the surgical instrument for electrocautery shown in FIG. 2.

### MODE OF INVENTION

Hereinafter, the following embodiments will be described in detail with reference to the accompanying drawings. When describing with reference to the drawings, identical or corresponding components will be assigned the same reference numerals and duplicate descriptions thereof will be omitted.

Since various transformations can be made to these embodiments, specific embodiments will be illustrated in the drawings and described in detail in the detailed description. The effects and features of the present embodiments and the accompanying methods thereof will become apparent from the following description of the contents, taken in conjunction with the accompanying drawings. However, the present embodiments are not limited to the embodiments disclosed below, but may be implemented in various forms.

In describing the present disclosure, a detailed description of known related arts will be omitted when it is determined that the gist of the present disclosure may be unnecessarily obscured.

In the following embodiments, singular forms are intended to include plural forms as well, unless the context clearly indicates otherwise. Although terms such as "first," "second," and the like may be used to describe various components, such components should not be limited to the above terms The terms are only used to distinguish one component from another.

In the following embodiments, terms such as "include" or "have" mean that the features or components described in the specification are present, and the possibility that one or more other features or components will be added is not excluded in advance.

In the following embodiments, when a unit, region, or component is referred to as being provided on another unit, region, or component, it can be directly provided on the other unit, region, or component. That is, for example, intervening units, regions, or components may be present.

In the following embodiments, terms such as "connecting" or "coupling" two members do not necessarily mean a direct and/or fixed connection or coupling of the two members, unless the context clearly indicates otherwise, and do not preclude another members from being interposed between the two members.

Sizes of components in the drawings may be exaggerated or reduced for convenience of description. For example, since the size and thickness of each component shown in the drawings are arbitrarily illustrated for convenience of description, the following embodiments are not necessarily limited thereto.

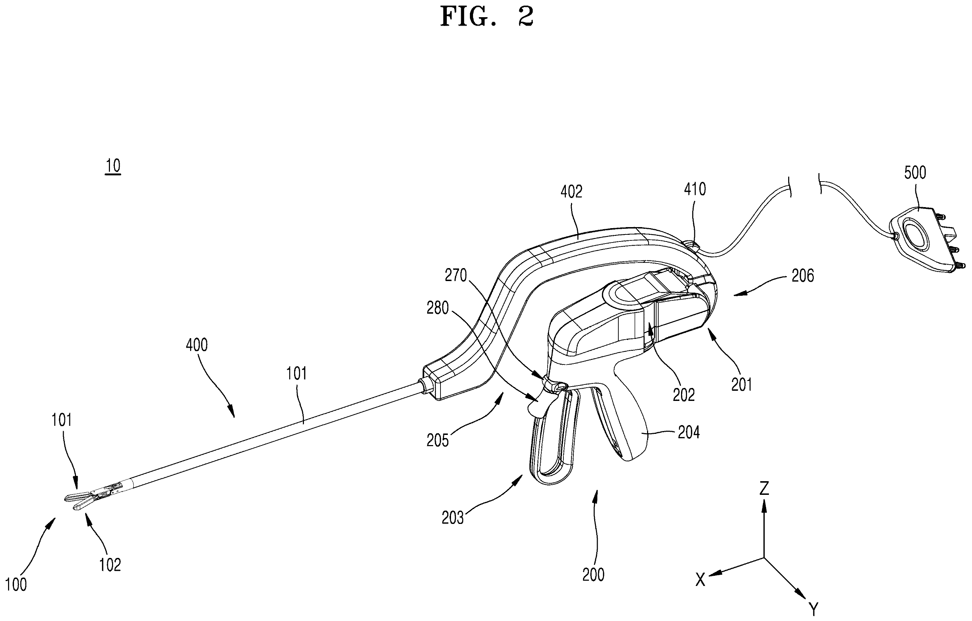

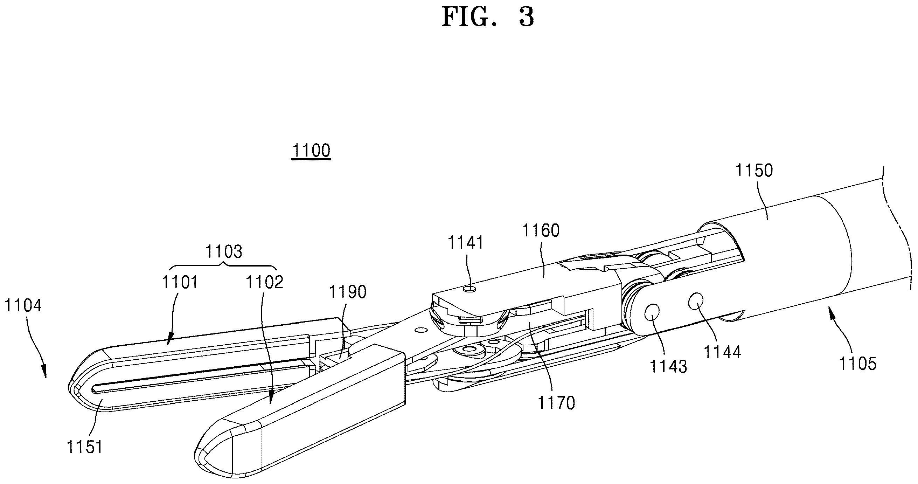

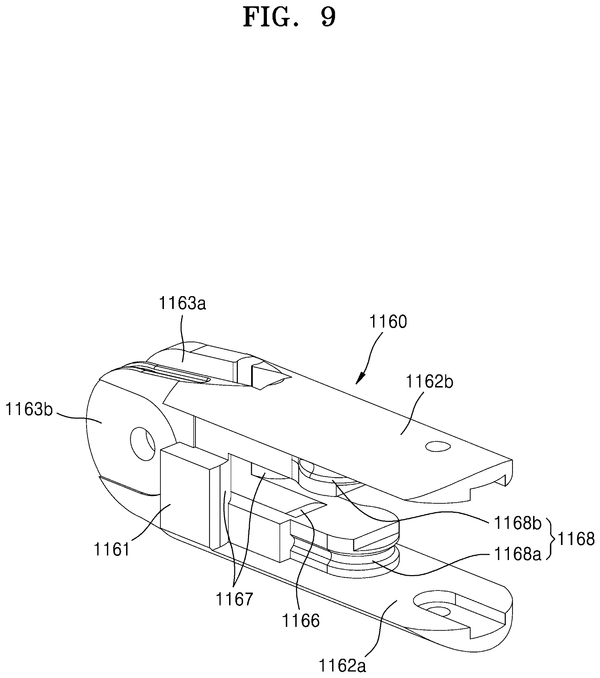

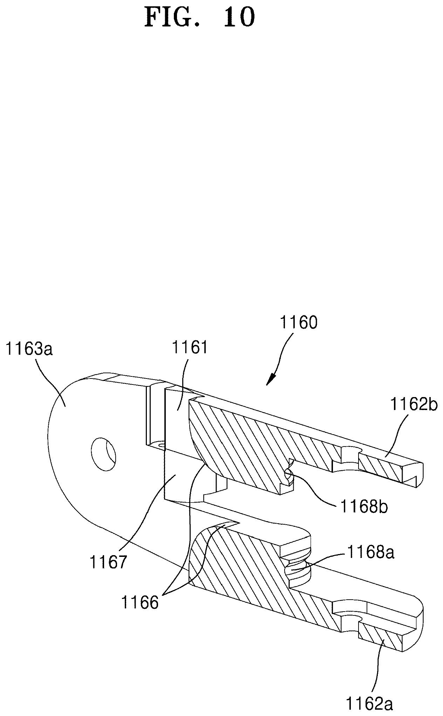

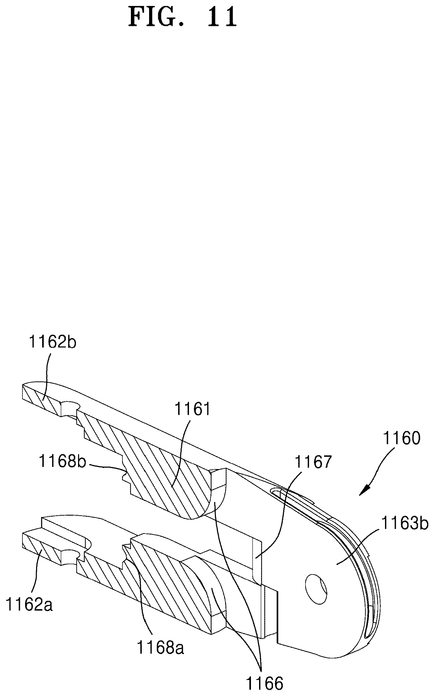

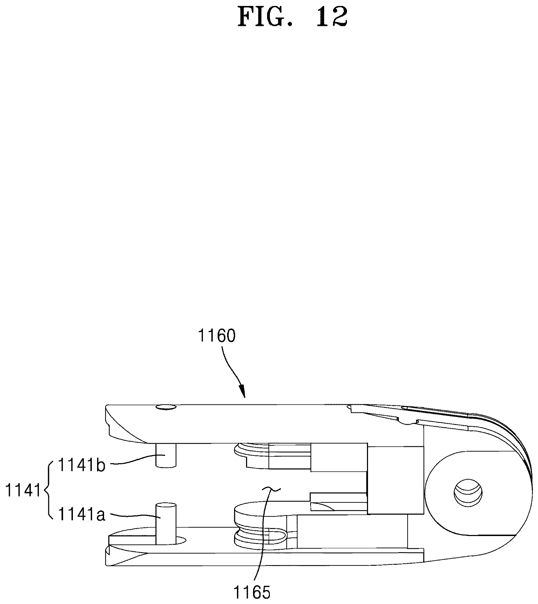

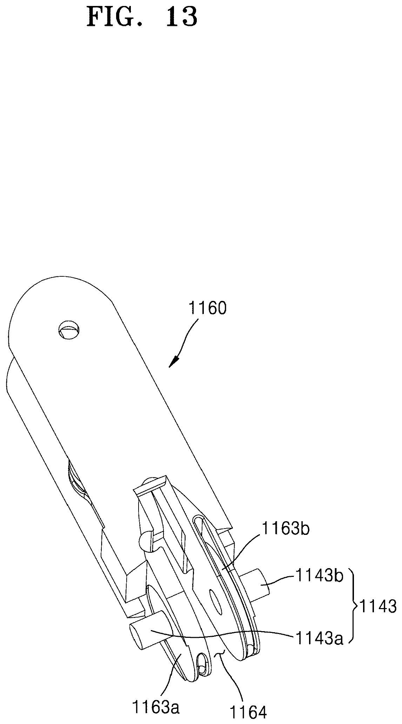

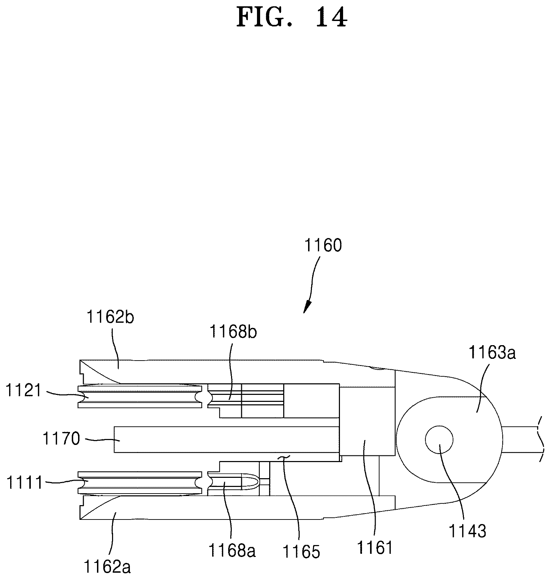

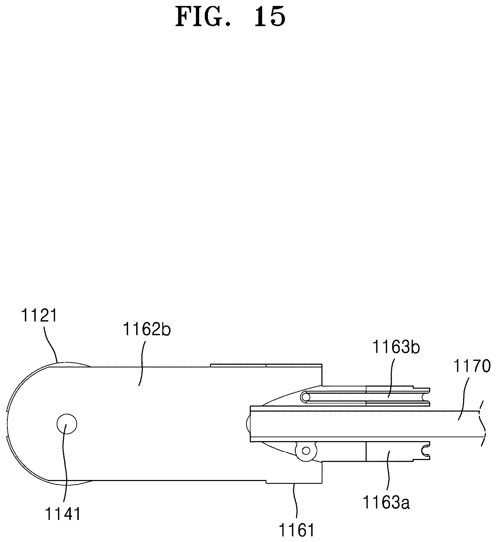

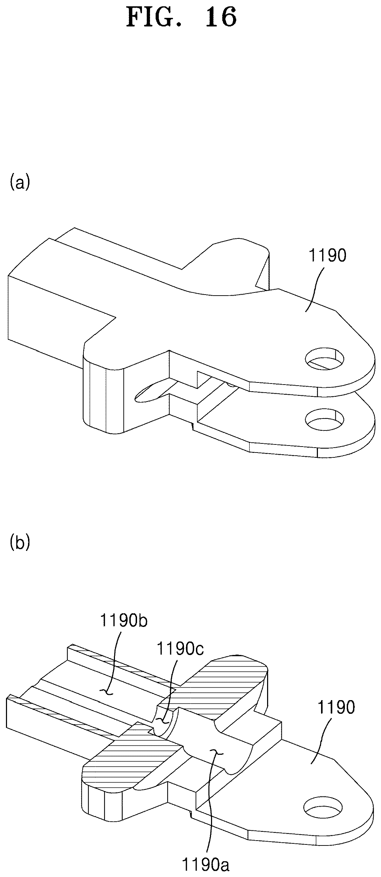

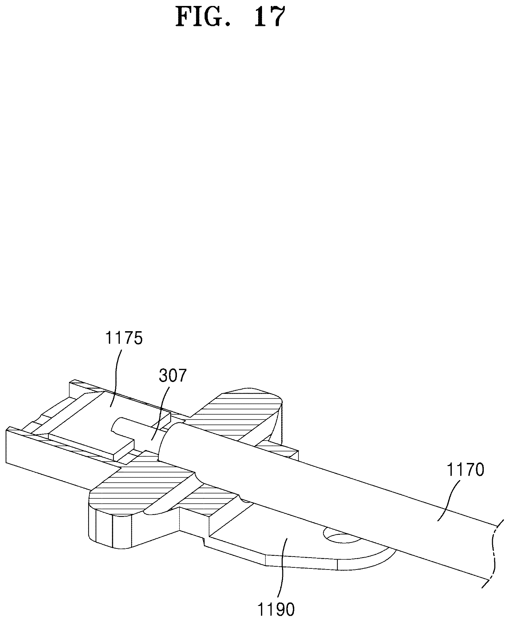

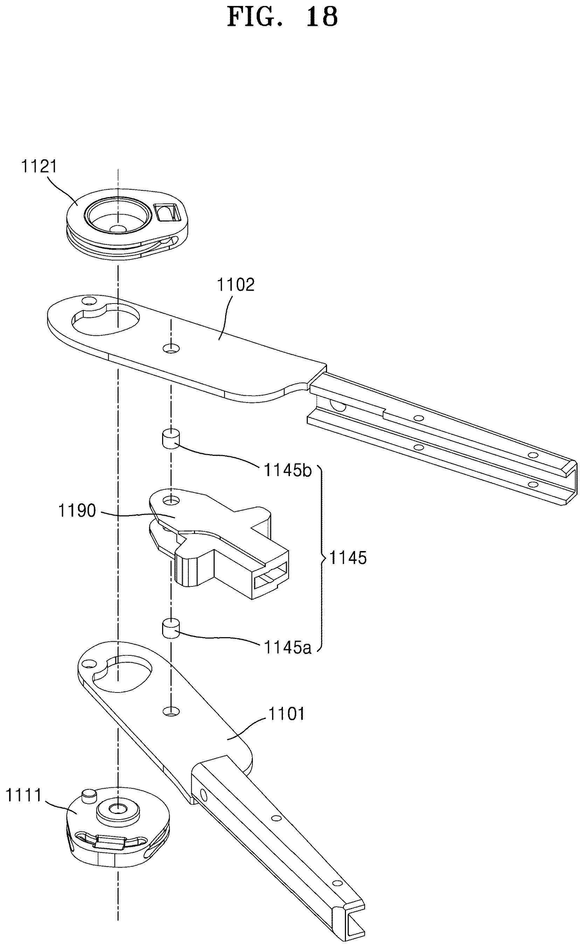

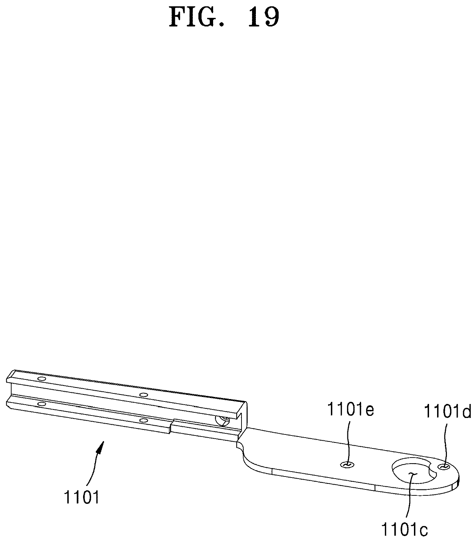

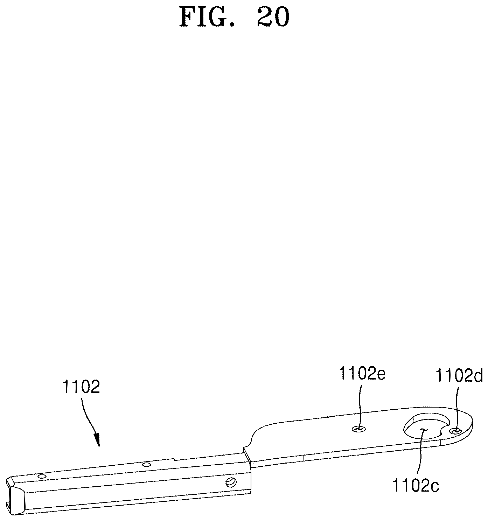

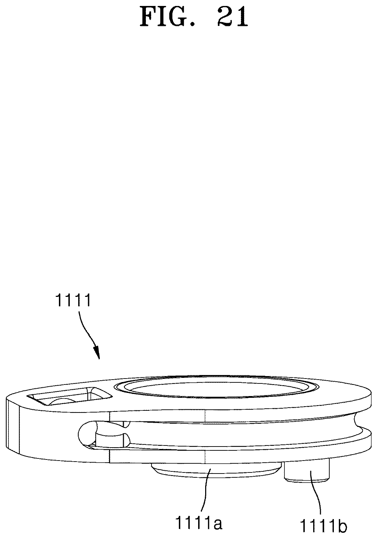

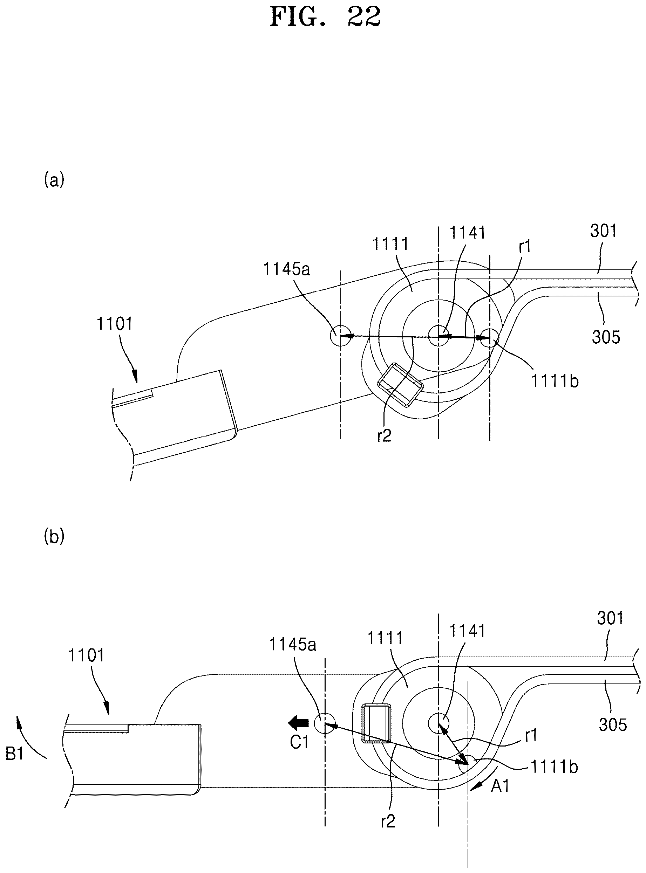

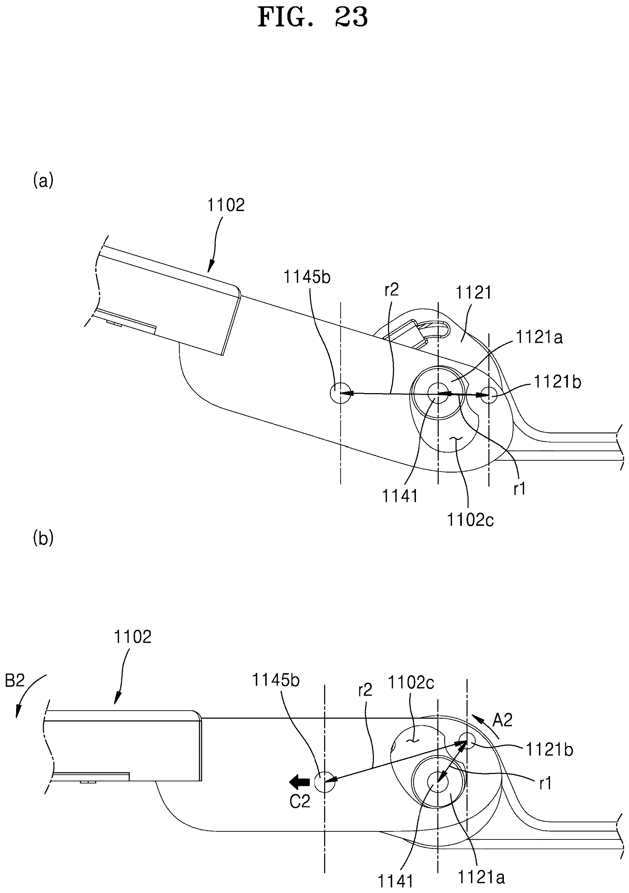

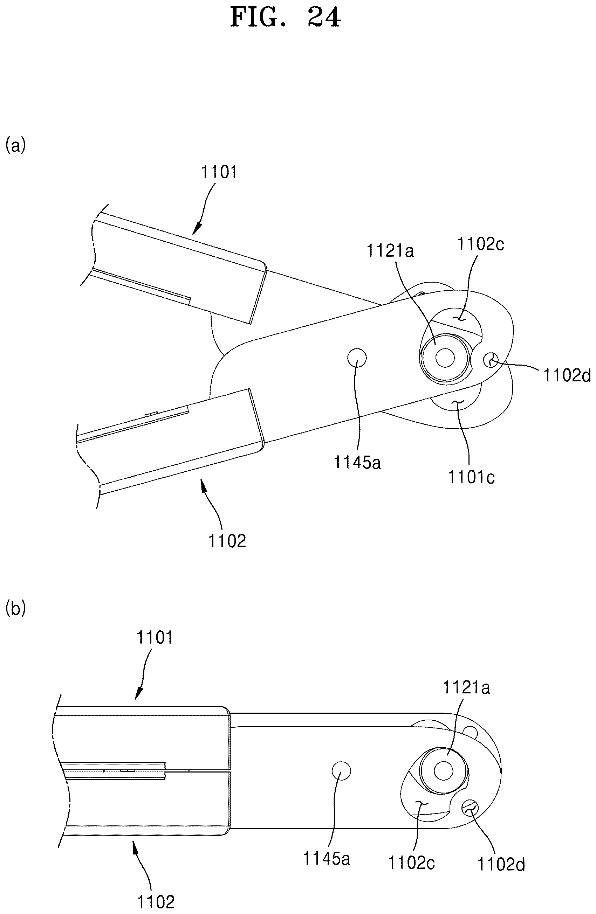

FIG. 2 is a perspective view illustrating a surgical instrument for electrocautery according to an embodiment of the present disclosure. FIGS. 3 to 8 are views illustrating an end tool of the surgical instrument for electrocautery of FIG. 2. FIG. 9 is a perspective view illustrating an end tool hub of the surgical instrument for electrocautery of FIG. 2. FIGS. 10 and 11 are cutaway perspective views of the end tool hub of FIG. 9. FIGS. 12 and 13 are perspective views illustrating the end tool hub of FIG. 9. FIG. 14 is a side view illustrating the end tool hub of FIG. 9 and a guide tube. FIG. 15 is a plan view illustrating the end tool hub of FIG. 9 and the guide tube. FIG. 16 is a perspective view illustrating an actuation hub of the surgical instrument for electrocautery of FIG. 2. FIG. 17 is a cutaway perspective view of the actuation hub of FIG. 16. FIG. 18 is an exploded perspective view illustrating the end tool of the surgical instrument for electrocautery of FIG. 2. FIG. 19 is a perspective view illustrating a first jaw of the end tool of the surgical instrument for electrocautery of FIG. 2. FIG. 20 is a perspective view illustrating a second jaw of the end tool of the surgical instrument for electrocautery of FIG. 2. FIG. 21 is a perspective view illustrating a first jaw pulley of the surgical instrument for electrocautery of FIG. 2. FIG. 22 is a plan view illustrating an opening and closing motion of the first jaw of the end tool of the surgical instrument for electrocautery of FIG. 2. FIG. 23 is a plan view illustrating an opening and closing motion of the second jaw of the end tool of the surgical instrument for electrocautery of FIG. 2. FIG. 24 is a plan view illustrating an opening and closing motion of the first jaw and the second jaw of the end tool of the surgical instrument for electrocautery of FIG. 2.

Referring to FIGS. 2 to 24 and the like, a surgical instrument for electrocautery 10 according to an embodiment of the present disclosure includes an end tool 1100, a manipulation part 200, a power transmission part 300, and a connection part 400.

Here, the connection part 400 is provided in the shape of a hollow shaft, and one or more wires and electric wires may be accommodated therein. The manipulation part 200 is coupled to one end portion of the connection part 400, the end tool 1100 is coupled to another end portion thereof, and the connection part 400 may serve to connect the manipulation part 200 to the end tool 1100. Here, the connection part 400 of the surgical instrument for electrocautery 10 according to an embodiment of the present disclosure includes a straight portion 401 and a bent portion 402, wherein the straight portion 401 is provided at a side coupled to the end tool 1100, and the bent portion 402 is provided at a side to which the manipulation part 200 is coupled. As such, since the end portion of the connection part 400 at the side of the manipulation part 200 is provided to be bent, a pitch-manipulation portion 201, a yaw-manipulation portion 202, and an actuation-manipulation portion 203 may be provided along an extension line of the end tool 1100 or adjacent to the extension line. In other words, it may be said that the pitch-manipulation portion 201 and the yaw-manipulation portion 202 are at least partially accommodated in a concave portion provided by the bent portion 402. Due to the above-described shape of the bent portion 402, the shapes and motions of the manipulation part 200 and the end tool 1100 may be further intuitively matched with each other.

In an embodiment, a plane on which the bent portion 402 is provided may be substantially the same as a pitch plane, that is, an XZ plane of FIG. 2. As such, as the bent portion 402 is provided on substantially the same plane as the XZ plane, interference with the manipulation part may be reduced. For intuitive motions of the end tool and the manipulation part, any form other than the XZ plane may be possible.

In an embodiment, a connector 410 may be provided on the bent portion 402. The connector 410 may be connected to an external power supply (not shown), and the connector 410 may be connected to a jaw 1103 through electric wires 411 and 412 to transfer electrical energy supplied from the external power supply (not shown) to the jaw 1103. Here, the connector 410 may be of a bipolar-type having two electrodes, or the connector 410 may be of a monopolar type having one electrode.

The manipulation part 200 is provided at one end portion of the connection part 400 and provided as an interface to be directly controlled by a medical doctor, for example, a tongs shape, a stick shape, a lever shape, or the like, and when the medical doctor controls the manipulation part 200, the end tool 1100, which is connected to the interface and inserted into the body of a surgical patient, performs a certain motion, thereby performing surgery. Here, the manipulation part 200 is illustrated in FIG. 2 as being provided in a handle shape that is rotatable while the finger is inserted therein, but, the concept of the present disclosure is not limited thereto, and various types of manipulation parts that are connected to the end tool 1100 and manipulate the end tool 1100 may be possible.

The end tool 1100 is provided on another end portion of the connection part 400, and performs necessary motions for surgery by being inserted into a surgical site. In an example of the end tool 1100 described above, as shown in FIG. 2, a pair of jaws 1103 for performing a grip motion may be used. However, the concept of the present disclosure is not limited thereto, and various devices for performing surgery may be used as the end tool 1100. For example, a configuration of a cantilever cautery may also be used as the end tool. The end tool 1100 is connected to the manipulation part 200 by the power transmission part 300, and receives a driving force of the manipulation part 200 through the power transmission part 300 to perform a motion necessary for surgery, such as gripping, cutting, suturing, or the like.

Here, the end tool 1100 of the surgical instrument for electrocautery 10 according to an embodiment of the present disclosure is configured to be rotatable in at least one direction, for example, the end tool 1100 may perform a pitch motion around a Y-axis of FIG. 2 and simultaneously perform a yaw motion and an actuation motion around a Z-axis of FIG. 2.

The power transmission part 300 may connect the manipulation part 200 to the end tool 1100, transmit a driving force of the manipulation part 200 to the end tool 1100, and include a plurality of wires, pulleys, links, sections, gears, or the like.

The end tool 1100, the manipulation part 200, the power transmission part 300, and the like of the surgical instrument for electrocautery 10 of FIG. 2 will be described in detail later.

### (Power transmission part)

Hereinafter, the power transmission part 300 of the surgical instrument for electrocautery 10 of FIG. 2 will be described in more detail.

Referring to FIGS. 2 to 8 and the like, the power transmission part 300 of the surgical instrument for electrocautery 10 according to an embodiment of the present disclosure may include a wire 301, a wire 302, a wire 303, a wire 304, a wire 305, a wire 306, and a blade wire 307.

Here, the wire 301 and the wire 305 may be paired to serve as first jaw wires. The wire 302 and the wire 306 may be paired to serve as second jaw wires. Here, the components encompassing the wires 301 and 305, which are first jaw wires, and the wires 302 and 306, which are second jaw wires, may be referred to as jaw wires. In an embodiment, the wire 303 and the wire 304 may be paired to serve as pitch wires.

In an embodiment, the power transmission part 300 of the surgical instrument for electrocautery 10 according to an embodiment of the present disclosure may include a fastening member 321, a fastening member 322, a fastening member 323, a fastening member 324, a fastening member 326, and a fastening member 327 that are coupled to respective end portions of the wires to respectively couple the wires and the pulleys. Here, each of the fastening members may have various shapes as necessary, such as a ball shape, a tube shape, and the like.

Here, at the end tool 1100 side, the fastening member 321 and the fastening member 322 may serve as pitch wire-end tool fastening members, the fastening member 323 may serve as a first jaw wire-end tool fastening member, and the fastening member 326 may serve as a second jaw wire-end tool fastening member.

In an embodiment, at the manipulation part 200 side, the fastening member 324 may serve as a first jaw wire-manipulation part fastening member, and the fastening member 327 may serve as a second jaw wire-manipulation part fastening member. In an embodiment, although not shown in the drawings, a pitch wire-manipulation part fastening member and a blade wire-manipulation part fastening member may be further provided at the manipulation part 200 side.

The coupling relationship between the wires, the fastening members, and the respective pulleys will be described in detail as follows.

First, the wires 301 and 305, which are first jaw wires, may form a single first jaw wire. The fastening member 323, which is a first jaw wire-end tool fastening member, is inserted at an intermediate point of the first jaw wire, which is a single wire, and the fastening member 323 is crimped and fixed, and then, opposite strands of the first jaw wire centered on the fastening member 323 may be referred to as the wire 301 and the wire 305, respectively.

Alternatively, the wires 301 and 305, which are first jaw wires, may also be provided as separate wires and connected by the fastening member 323.

In an embodiment, by coupling the fastening member 323 to a pulley 1111, the wires 301 and 305 may be fixedly coupled to the pulley 1111. This allows the pulley 1111 to rotate as the wire 301 and the wire 305 are pulled and released.

In an embodiment, the first jaw wire-manipulation part fastening member 324 may be coupled to another end portions of the wires 301 and 305, which are opposite to one end portions to which the fastening member 323 is fastened.

In an embodiment, by coupling the first jaw wire-manipulation part fastening member 324 to a pulley 211, the wires 301 and 305 may be fixedly coupled to the pulley 211. As a result, when the pulley 211 is rotated by a motor or human power, the wire 301 and the wire 305 are pulled and released, allowing the pulley 1111 of the end tool 1100 to rotate.

In the same manner, the wire 302 and the wire 306, which are second jaw wires, are coupled to each of the fastening member 326, which is a second jaw wire-end tool fastening member, and the second jaw wire-manipulation part fastening member 327. In an embodiment, the fastening member 326 is coupled to a pulley 1121, and the second jaw wire-manipulation part fastening member is coupled to a pulley 220. As a result, when the pulley 220 is rotated by a motor or a human power, the pulley 1121 of the end tool 1100 may be rotated as the wire 302 and the wire 306 are pulled and released.

In the same manner, the wire 304, which is a pitch wire, is coupled to the fastening member 321, which is a pitch wire-end tool fastening member, and the pitch wire-manipulation part fastening member (not shown). In an embodiment, the wire 303, which is a pitch wire, is coupled to the fastening member 322, which is a pitch wire-end tool fastening member, and the pitch wire-manipulation part fastening member (not shown).

In an embodiment, the fastening member 321 is coupled to a first pitch pulley portion 1163a of an end tool hub 1160, the fastening member 322 is coupled to a second pitch pulley portion 1163b of the end tool hub 1160, and the pitch wire-manipulation part fastening member (not shown) is coupled to a pulley 231. As a result, when the pulley 231 is rotated by a motor or human power, the wire 303 and the wire 304 are pulled and released, allowing the end tool hub 1160 of the end tool 1100 to rotate.

In describing the present disclosure, the part closer to the user side, i.e., the part closer to the manipulation part 200, is described as a proximal end, and the part farther from the user side, that is, the part closer to the end tool 1100, will be described as a distal end.

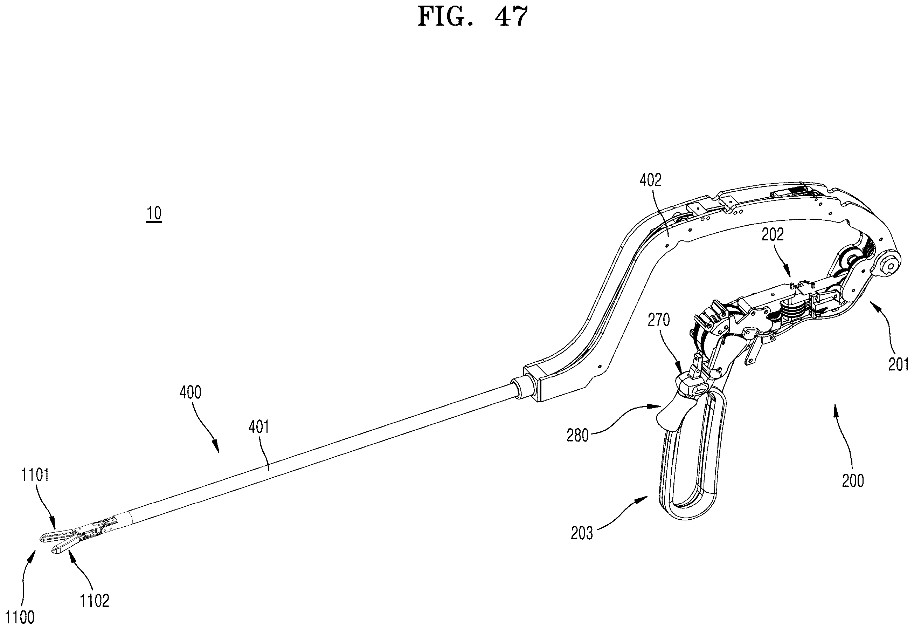

For example, referring to FIGS. 3 and 47, the part of the end tool 1100 closer to the manipulation part 200 will be defined as a proximal end 1105 of the end tool 1100, and the part farther from the manipulation part 200, that is, the part closer to an end portion of the end tool 1100 will be defined and described as a distal end 1104 of the end tool 1100. In other words, the proximal end 1105 of the end tool 1100 may be described as the part closer to the connection part 400, and the distal end 1104 of the end tool 1100 may be described as the part farther from the connection part 400.

In an embodiment, one end portion of the blade wire 307 is coupled to a blade 1175 to be described later, and another end portion thereof is coupled to a cutting-manipulation portion 280 of the manipulation part 200. By the manipulation of the cutting-manipulation portion 280, a cutting motion may be performed as the blade wire 307 moves from the proximal end 1105 toward the distal end 1104 of the end tool 1100, or the blade wire 307 may return from the distal end 1104 toward the proximal end 1105 of the end tool 1100.

At this time, at least a portion of the blade wire 307 may be accommodated in a guide tube 1170 to be described later. Accordingly, when the guide tube 1170 is bent in response to a pitch motion or yaw motion of the end tool 1100, the blade wire 307 accommodated therein may also be bent together with the guide tube 1170. The guide tube 1170 will be described in more detail later.

The blade wire 307 is configured to linearly move in a longitudinal direction of the connection part 400 within the connection part 400. In an embodiment, since one end portion of the blade wire 307 is coupled to the blade 1175, when the blade wire 307 linearly moves in the longitudinal direction of the connection part 400, the blade 1175 connected thereto is also linearly moved. That is, when the blade wire 307 linearly moves in the longitudinal direction of the connection part 400, a cutting motion is performed as the blade 1175 connected thereto moves toward the distal end 1104 or the proximal end 1105 of the end tool 1100. This will be described in more detail later.

In an embodiment, the member that linearly moves in the end tool 1100 by the blade wire 307 is not limited to the blade 1175, and may include various moving members configured to perform different purposes and functions. For example, various moving members that linearly move, such as a wedge of a stapler or other similar members may be included.

In an embodiment, the cutting-manipulation portion of the manipulation part is not limited to controlling linear movement of the blade, and may include, for example, a stapling manipulation portion configured to perform stapling and cutting by linearly moving the moving member along the axis of the connection part (shaft) through the blade wire.

### (End tool)

Hereinafter, the end tool 1100 of the surgical instrument for electrocautery 10 of FIG. 2 will be described in more detail.

FIG. 2 is a perspective view illustrating the surgical instrument for electrocautery according to an embodiment of the present disclosure. FIGS. 3 to 8 are views illustrating the end tool of the surgical instrument for electrocautery of FIG. 2.

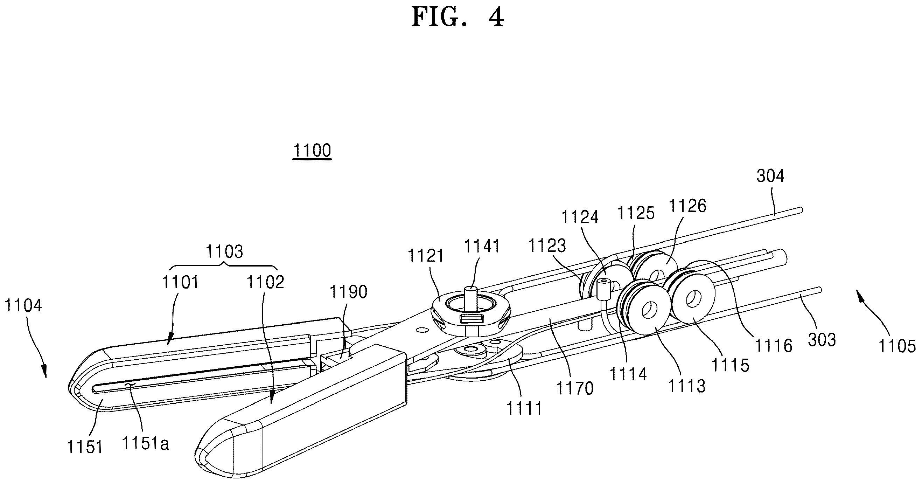

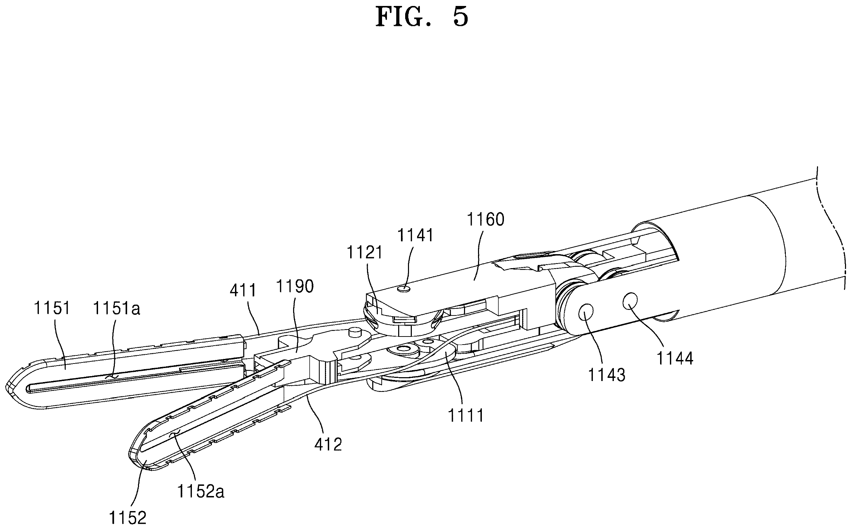

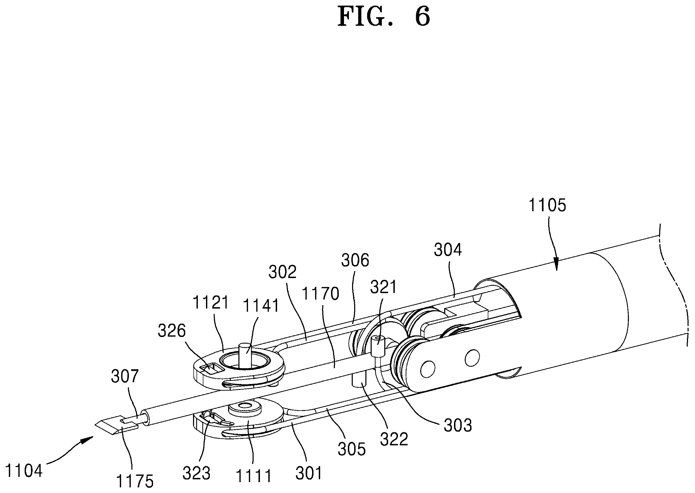

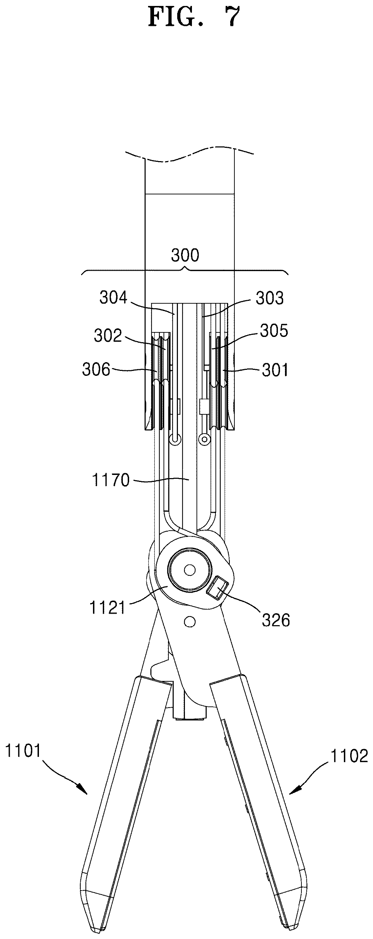

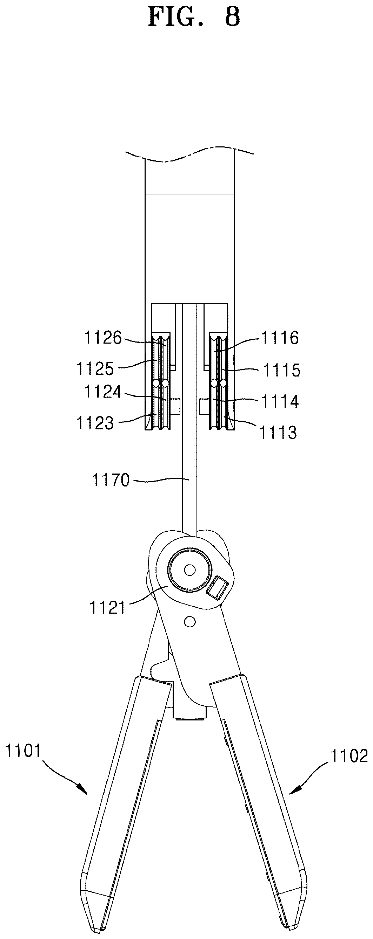

Here, FIG. 3 illustrates a state in which the end tool hub 1160 and a pitch hub 1150 are coupled, and FIG. 4 illustrates a state in which the end tool hub 1160 and pitch hub 1150 are removed. FIG. 5 illustrates a state in which a first jaw 1101 and a second jaw 1102 are removed, and FIG. 6 illustrates a state in which the first jaw 1101, the second jaw 1102, the pulley 1111, the pulley 1121, and the like are removed. FIG. 7 is a view mainly illustrating the wires, and FIG. 8 is a view mainly illustrating the pulleys.

Referring to FIGS. 2 to 24 and the like, the end tool 1100 of the fourth embodiment of the present disclosure includes a pair of jaws for performing a grip motion, that is, the first jaw 1101 and the second jaw 1102. Here, each of the first jaw 1101 and the second jaw 1102, or a component encompassing the first jaw 1101 and the second jaw 1102 may be referred to as the jaw 1103.

In an embodiment, the end tool 1100 may include the pulley 1111, a pulley 1113, a pulley 1114, a pulley 1115, and a pulley 1116 associated with a rotational motion of the first jaw 1101. In an embodiment, the end tool 1100 may include the pulley 1121, a pulley 1123, a pulley 1124, a pulley 1125, and a pulley 1126, which are associated with a rotational motion of the second jaw 1102.

Here, the pulleys facing each other are illustrated in the drawings as being provided parallel to each other, but the concept of the present disclosure is not limited thereto, and each of the pulleys may be variously provided with a position and a size suitable for the configuration of the end tool.

In an embodiment, the end tool 1100 according to an embodiment of the present disclosure may include the end tool hub 1160 and the pitch hub 1150.

A first rotation shaft 1141 to be described later may be inserted through the end tool hub 1160, and the pulley 1111 and the pulley 1121 axially coupled to the first rotation shaft 1141 and at least some of the first jaw 1101 and the second jaw 1102 coupled to the pulley 1111 and the pulley 1121 may be accommodated inside the end tool hub 1160. Here, in an embodiment of the present disclosure, a wire guide portion 1168 serving as an auxiliary pulley is provided in the end tool hub 1160. That is, a first wire guide portion 1168a and a second wire guide portion 1168b for guiding paths of the wire 305 and the wire 302 may be provided in the end tool hub 1160. The wire guide portions 1168 of the end tool hub 1160 may serve as auxiliary pulleys and change the paths of the wires, and the first wire guide portion 1168a and the second wire guide portion 1168b of the end tool hub 1160 serving as auxiliary pulleys will be described in more detail later.

In an embodiment, the first pitch pulley portion 1163a and the second pitch pulley portion 1163b, which serve as end tool pitch pulleys, may be provided at one end portion of the end tool hub 1160. The wire 303 and the wire 304, which are pitch wires, are respectively coupled to the first pitch pulley portion 1163a and the second pitch pulley portion 1163b, which serve as end tool pitch pulleys, and a pitch motion is performed while the end tool hub 1160 rotates around a third rotation shaft 1143.

The third rotation shaft 1143 and a fourth rotation shaft 1144 may be inserted through the pitch hub 1150, and the pitch hub 1150 may be axially coupled to the end tool hub 1160 by the third rotation shaft 1143. Accordingly, the end tool hub 1160 may be configured to perform a pitch rotation around the third rotation shaft 1143 with respect to the pitch hub 1150.

In an embodiment, the pitch hub 1150 may internally accommodate at least some of the pulley 1113, the pulley 1114, the pulley 1123, and the pulley 1124 that are axially coupled to the third rotation shaft 1143. In an embodiment, the pitch hub 1150 may internally accommodate at least some of the pulley 1115, the pulley 1116, the pulley 1125, and the pulley 1126 that are axially coupled to the fourth rotation shaft 1144.

One end portion of the pitch hub 1150 is connected to the end tool hub 1160, and another end portion of the pitch hub 1150 is connected to the connection part 400.

Here, the end tool 1100 according to an embodiment of the present disclosure may include the first rotation shaft 1141, the third rotation shaft 1143, and the fourth rotation shaft 1144. As described above, the first rotation shaft 1141 may be inserted through the end tool hub 1160, and the third rotation shaft 1143 and the fourth rotation shaft 1144 may be inserted through the pitch hub 1150.

The first rotation shaft 1141, the third rotation shaft 1143, and the fourth rotation shaft 1144 may be arranged sequentially from the distal end 1104 toward the proximal end 1105 of the end tool 1100. Accordingly, starting from the distal end 1104, the first rotation shaft 1141 may be referred to as a first pin, the third rotation shaft 1143 may be referred to as a third pin, and the fourth rotation shaft 1144 may be referred to as a fourth pin.

Here, the first rotation shaft 1141 may function as an end tool jaw pulley rotation shaft, the third rotation shaft 1143 may function as an end tool pitch rotation shaft, and the fourth rotation shaft 1144 may function as an end tool pitch auxiliary rotation shaft of the end tool 1100.

Here, each of the rotation shafts may include two shafts of a first sub-shaft and a second sub-shaft. Alternatively, it may be said that each of the rotation shafts is provided by being divided into two parts.

For example, the first rotation shaft 1141 may include two shafts of a first sub-shaft 1141a and a second sub-shaft 1141b. In an embodiment, the third rotation shaft 1143 may include two shafts of a first sub-shaft 1143a and a second sub-shaft 1143b. The fourth rotation shaft 1144 may include two shafts of a first sub-shaft and a second sub-shaft.

Each of the rotation shafts is provided by being divided into two parts as described above to allow the guide tube 1170 to be described later to pass through the end tool hub 1160 and the pitch hub 1150. That is, the guide tube 1170 may pass between the first sub-shaft and the second sub-shaft of each of the rotation shafts. This will be described in more detail later. Here, the first sub-shaft and the second sub-shaft may be disposed on the same axis or may be disposed to be offset to a certain extent.

In an embodiment, it is illustrated in the drawings that each of the rotation shafts is provided by being divided into two parts, but the concept of the present disclosure is not limited thereto. That is, each of the rotation shafts is provided to be curved in the middle such that an escape path for the guide tube 1170 is provided.

Each of the rotation shafts 1141, 1143, and 1144 may be fitted into one or more pulleys, which will be described in detail later.

In an embodiment, the end tool 1100 may further include an actuation rotation shaft 1145. For example, the first jaw 1101 and the second jaw 1102 may be axially coupled to each other by the actuation rotation shaft 1145, and in this state, an actuation motion may be performed while the first jaw 1101 and the second jaw 1102 rotate around the actuation rotation shaft 1145. Here, the actuation rotation shaft 1145 may be positioned closer to the distal end 1104 than the first rotation shaft 1141 is.

Here, in the end tool 1100 according to an embodiment of the present disclosure, the first rotation shaft 1141, which is a yaw rotation shaft, and the actuation rotation shaft 1145 are provided separately rather than as the same shaft. That is, by forming the first rotation shaft 1141, which serves as a rotation shaft of the jaw pulleys 1111 and 1121 and as a rotation shaft of a yaw motion, and the actuation rotation shaft 1145, which serves as a rotation shaft of the second jaw 1102 with respect to the first jaw 1101 and as a rotation shaft of an actuation motion, to be spaced apart from each other by a certain distance, a space may be secured in which the guide tube 1170 and the blade wire 307 accommodated therein can be gently bent. The actuation rotation shaft 1145 will be described in detail later.

The pulley 1111 functions as an end tool first jaw pulley, and the pulley 1121 functions as an end tool second jaw pulley. The pulley 1111 may be referred to as a first jaw pulley, and the pulley 1121 may be referred to as a second jaw pulley. These two components may collectively be referred to as end tool jaw pulleys or simply jaw pulleys.

The pulley 1111 and the pulley 1121, which are end tool jaw pulleys, are configured to face each other, and are configured to be rotatable independently of each other around the first rotation shaft 1141 which is an end tool jaw pulley rotation shaft. In this case, the pulley 1111 and the pulley 1121 are provided to be spaced apart by a certain distance, and a blade assembly accommodation portion may be accommodated therebetween. In an embodiment, at least a portion of a blade assembly to be described later may be positioned in the blade assembly accommodation portion. In other words, the blade assembly including the guide tube 1170 may be positioned between the pulley 1111 and the pulley 1121.

Here, since the pulley 1111 is connected to the first jaw 1101, when the pulley 1111 rotates around the first rotation shaft 1141, the first jaw 1101 may also rotate around the first rotation shaft 1141 together with the pulley 1111.

In an embodiment, since the pulley 1121 is connected to the second jaw 1102, when the pulley 1121 rotates around the first rotation shaft 1141, the second jaw 1102 connected to the pulley 1121 may rotate around the first rotation shaft 1141.

In an embodiment, a yaw motion and an actuation motion of the end tool 1100 are performed in response to the rotation of the pulley 1111 and the pulley 1121. That is, when the pulley 1111 and the pulley 1121 rotate in the same direction around the first rotation shaft 1141, the yaw motion is performed as the first jaw 1101 and the second jaw 1102 rotate the first rotation shaft 1141. In an embodiment, when the pulley 1111 and the pulley 1121 rotate in opposite directions around the first rotation shaft 1141, the actuation motion is performed as the first jaw 1101 and the second jaw 1102 rotate around the actuation rotation shaft 1145.

The pulley 1113 and the pulley 1114 function as end tool first jaw pitch main pulleys, and the pulley 1123 and the pulley 1124 function as end tool second jaw pitch main pulleys. These components may collectively be referred to as end tool jaw pitch main pulleys.

The pulley 1115 and the pulley 1116 function as end tool first jaw pitch sub-pulleys, and the pulley 1125 and the pulley 1126 function as end tool second jaw pitch sub-pulleys. These components collectively may be referred to as end tool jaw pitch sub-pulleys.

Hereinafter, components associated with the rotation of the pulley 1111 will be described.

The pulley 1113 and the pulley 1114 function as end tool first jaw pitch main pulleys. That is, the pulley 1113 and the pulley 1114 function as main rotation pulleys for a pitch motion of the first jaw 1101. Here, the wire 301, which is a first jaw wire, is wound around the pulley 1113, and the wire 305, which is a first jaw wire, is wound around the pulley 1114.

The pulley 1115 and the pulley 1116 function as end tool first jaw pitch sub-pulleys. That is, the pulley 1115 and the pulley 1116 function as sub-rotation pulleys for a pitch motion of the first jaw 1101. Here, the wire 301, which is a first jaw wire, is wound around the pulley 1115, and the wire 305, which is a first jaw wire, is wound around the pulley 1116.

Here, the pulley 1113 and the pulley 1114 are disposed on one side of the pulley 1111 to face each other. Here, the pulley 1113 and the pulley 1114 are configured to be rotatable independently of each other around the third rotation shaft 1143 serving as an end tool pitch rotation shaft. In an embodiment, the pulley 1115 and the pulley 1116 are disposed on one side of the pulley 1113 and one side of the pulley 1114, respectively, to face each other. Here, the pulley 1115 and the pulley 1116 are configured to be rotatable independently of each other around the fourth rotation shaft 1144 serving as an end tool pitch auxiliary rotation shaft. Here, in the drawings, the pulley 1113, the pulley 1115, the pulley 1114, and the pulley 1116 are all illustrated as being rotatable around a Y-axis direction, but the concept of the present disclosure is not limited thereto, and the rotation axes of the respective pulleys may be provided in various directions according to configurations thereof.

The wire 301, which is a first jaw wire, is sequentially wound to make contact with at least portions of the pulley 1115, the pulley 1113, and the pulley 1111. In an embodiment, the wire 305 connected to the wire 301 by the fastening member 323 is sequentially wound to make contact with at least portions of the pulley 1111, the first wire guide portion 1168a of the end tool hub 1160, the pulley 1114, and the pulley 1116.

In other words, the wire 301 and the wire 305, which are the first jaw wires, are sequentially wound to make contact with at least portions of the pulley 1115, the pulley 1113, the pulley 1111, the first wire guide part 1168a of the end tool hub 1160, the pulley 1114, and the pulley 1116, and are configured to move along the above pulleys while rotating the above pulleys.

Accordingly, when the wire 301 is pulled in the direction of an arrow 301 of FIG. 7, the fastening member 323 to which the wire 301 is coupled and the pulley 1111 coupled to the fastening member 323 are rotated in a counterclockwise direction. In an embodiment, when the wire 305 is pulled in the direction of an arrow 305 of FIG. 7, the fastening member 323 to which the wire 305 is coupled and the pulley 1111 coupled to the fastening member 323 are rotated in the clockwise direction in the FIG. 7.

Next, components associated with the rotation of the pulley 1121 will be described.

The pulley 1123 and the pulley 1124 function as end tool second jaw pitch main pulleys. That is, the pulley 1123 and the pulley 1124 function as main rotation pulleys for a pitch motion of the second jaw 1102. Here, the wire 306, which is a second jaw wire, is wound around the pulley 1123, and the wire 302, which is a second jaw wire, is wound around the pulley 1124.

The pulley 1125 and the pulley 1126 function as end tool second jaw pitch sub-pulleys. That is, the pulley 1125 and the pulley 1126 function as sub-rotation pulleys for a pitch motion of the second jaw 1102. Here, the wire 306, which is a second jaw wire, is wound around the pulley 1125, and the wire 302, which is a second jaw wire, is wound around the pulley 1126.

Here, the pulley 1123 and the pulley 1124 are disposed on one side of the pulley 1121 to face each other. Here, the pulley 1123 and the pulley 1124 are configured to be rotatable independently of each other around the third rotation shaft 1143 serving as an end tool pitch rotation shaft. In an embodiment, the pulley 1125 and the pulley 1126 are disposed on one side of the pulley 1123 and one side of the pulley 1124, respectively, to face each other. Here, the pulley 1125 and the pulley 1126 are configured to be rotatable independently of each other around the fourth rotation shaft 1144 serving as an end tool pitch auxiliary rotation shaft. Here, in the drawings, the pulley 1123, the pulley 1125, the pulley 1124, and the pulley 1126 are all illustrated as being configured to rotate around the Y-axis direction, but the concept of the present disclosure is not limited thereto, and the rotating axes of the respective pulleys may be provided in various directions according to configurations thereof.

The wire 306, which is a second jaw wire, is sequentially wound to make contact with at least portions of the pulley 1125, the pulley 1123, and the pulley 1121. In an embodiment, the wire 302 connected to the wire 306 by the fastening member 326 is sequentially wound to make contact with at least portions of the pulley 1121, the second wire guide portion 1168b of the end tool hub 1160, the pulley 1124, and the pulley 1126.

In other words, the wire 306 and the wire 302, which are the second jaw wires, are sequentially wound to make contact with at least portions of the pulley 1125, the pulley 1123, the pulley 1121, the second wire guide portion 1168b of the end tool hub 1160, the pulley 1124, and the pulley 1126, and are configured to move along the above pulleys while rotating the above pulleys.

Accordingly, when the wire 306 is pulled in the direction of an arrow 306 of FIG. 7, the fastening member 326 to which the wire 306 is coupled and the pulley 1121 coupled to the fastening member 326 are rotated in a clockwise direction in FIG. 7. In an embodiment, when the wire 302 is pulled toward an arrow 302 of FIG. 7, the fastening member 326 to which the wire 302 is coupled and the pulley 1121 coupled to the fastening member 326 may be rotated in the counterclockwise direction in FIG. 7.

Hereinafter, a pitch motion of the present disclosure will be described in more detail.

In an embodiment, when the wire 301 is pulled in the direction of the arrow 301 of FIG. 7, and simultaneously, the wire 305 is pulled in the direction of the arrow 305 of FIG. 7 (that is, when opposite strands of the first jaw wire are pulled), as shown in FIG. 6, since the wires 301 and 305 are wound around lower portions of the pulley 1113 and the pulley 1114 rotatable around the third rotation shaft 1143, which is an end tool pitch rotation shaft, the pulley 1111 to which the wires 301 and 305 are fixedly coupled and the end tool hub 1160 to which the pulley 1111 is coupled rotate as a whole in the counterclockwise direction around the third rotation shaft 1143, and as a result, the end tool 1100 may rotate downward to perform the pitch motion. At this time, since the wires 302 and 306, which are fixedly coupled to the second jaw 1102, are wound around upper portions of the pulley 1123 and the pulley 1124 rotatable around the third rotation shaft 1143, the wires 302 and 306 are released in the opposite directions of the arrows 302 and 306, respectively.

In an embodiment, when the wire 302 is pulled in the direction of the arrow 302 of FIG. 7, and simultaneously, the wire 306 is pulled in the direction of the arrow 306 of FIG. 7, as shown in FIG. 6, since the wires 302 and 306 are wound around the upper portions of the pulley 1123 and the pulley 1124 rotatable around the third rotation shaft 1143, which is an end tool pitch rotation shaft, the pulley 1121 to which the wires 302 and 306 are fixedly coupled and the end tool hub 1160 to which the pulley 1121 is coupled rotate as a whole in the clockwise direction around the third rotation shaft 1143, and as a result, the end tool 1100 may rotate upward to perform the pitch motion. At this time, since the wires 301 and 305, which are fixedly coupled to the first jaw 1101, are wound around lower portions of the pulley 1113 and the pulley 1114 rotatable around the third rotation shaft 1143, the wires 302 and 306 are moved in the opposite directions of the arrows 301 and 305, respectively.

In an embodiment, the end tool hub 1160 of the end tool 1100 of the surgical instrument for electrocautery 10 of the present disclosure may further include the first pitch pulley portion 1163a and the second pitch pulley portion 1163b serving as end tool pitch pulleys, the manipulation part 200 may further include the pulley 231 and a pulley 232, which are manipulation part pitch pulleys, and the power transmission part 300 may further include the wire 303 and the wire 304 serving as pitch wires.

For example, the end tool hub 1160 including the first pitch pulley portion 1163a and the second pitch pulley portion 1163b may be configured to rotate around the third rotation shaft 1143 serving as an end tool pitch rotation shaft. In an embodiment, the wires 303 and 304 may serve to connect the first and second pitch pulley portions 1163a and 1163b of the end tool 1100 to the pulleys 231 and 232 of the manipulation part 200.

Thus, when the pulleys 231 and 232 of the manipulation part 200 rotate, the rotation of the pulleys 231 and 232 is transmitted to the end tool hub 1160 of the end tool 1100 through the wires 303 and 304, causing the end tool hub 1160 to rotate as well, and as a result, the end tool 1100 performs a pitch motion while rotating.

That is, the surgical instrument for electrocautery 10 according to an embodiment of the present disclosure includes the first and second pitch pulley portions 1163a and 1163b of the end tool 1100, the pulleys 231 and 232 of the manipulation part 200, and the wires 303 and 304 of the power transmission part 300 in order to transmit driving force for a pitch motion, and thus, the driving force for the pitch motion of the manipulation part 200 is more completely transmitted to the end tool 1100, thereby improving operation reliability.

### (Blade wire and guide tube)

Hereinafter, the blade wire 307 and the guide tube 1170 of the present disclosure will be described in more detail.

The guide tube 1170 according to the present disclosure is configured to surround the blade wire 307 in a certain section, and at this time, the blade wire 307 is movable inside the guide tube 1170. In other words, in a state in which in which the blade wire 307 is inserted into the guide tube 1170, the blade wire 307 is movable relative to the guide tube 1170.

Here, the guide tube 1170 serves to guide the path of the blade wire 307 by preventing the blade wire 307 from being curved in an unintended direction when the blade wire 307 is pushed or pulled. A cutting motion may be smoothly performed by the guide tube 1170.

In an embodiment, one end portion of the guide tube 1170 may be fixedly coupled to an actuation hub 1190 to be described later. Here, the actuation hub 1190 may serve as a first coupling portion. In an embodiment, another end portion of the guide tube 1170 may be fixedly coupled to a second coupling portion (not shown) in the connection part 400. As described above, since opposite end portions of the guide tube 1170 are fixedly coupled to certain points (the first coupling portion and the second coupling portion), respectively, the entire length of the guide tube 1170 may remain constant. Accordingly, a length of a portion of the blade wire 307 inserted into the guide tube 1170 may also remain constant.

In an embodiment, the guide tube 1170 according to the present disclosure may include a flexible material and configured to be bendable. Accordingly, when the end tool 1100 performs a yaw motion around the first rotation shaft 1141 or a pitch motion around the third rotation shaft 1143, the guide tube 1170 may be bent while being deformed in shape corresponding thereto. In an embodiment, when the guide tube 1170 is bent, the blade wire 307 placed therein is also bent.

Here, although the length of the guide tube 1170 is constant, the relative position and distance of the first coupling portion (i.e., the actuation hub 1190) and the second coupling portion (not shown) may be changed as the end tool 1100 is pitch-rotated or yaw-rotated. Accordingly, a space for the guide tube 1170 to move in response to the change in distance is required. To this end, a pitch slit 1164 and a yaw slit 1165 may be provided in the end tool hub 1160 to form spaces for movement of the guide tube 1170. Such a configuration of the end tool hub 1160 will be described in detail later.

In an embodiment, as described above, the blade wire 307 is inserted through the guide tube 1170, and the blade wire 307 is movable inside the guide tube 1170 relative to the guide tube 1170. That is, when the blade wire 307 is pulled in a state in which the guide tube 1170 is fixed, the blade 1175 connected to the blade wire 307 moves toward the proximal end 1105, and when the blade wire 307 is pushed, the blade 1175 connected to the blade wire 307 moves toward the distal end 1104.

This will be described below in more detail.

The most reliable way to perform a cutting motion using the blade 1175 is to push and pull the blade 1175 with the blade wire 307. In an embodiment, in order for the blade wire 307 to push and pull the blade 1175, the guide tube 1170 that can guide the path of the blade wire 307 needs to be provided. When the guide tube 1170 does not guide the path of the blade wire 307 (i.e., does not hold the blade wire 307), a phenomenon may occur in which cutting is not performed and a middle portion of the blade wire 307 is curved even when the blade wire 307 is pushed. Accordingly, in order to reliably perform a cutting motion using the blade 1175, it is essential to include the blade wire 307 and the guide tube 1170.

For example, since the blade wire 307 is required to bend in accordance with the pitch and yaw motions, the blade wire 307 needs to include a flexible material. However, when attempting to transmit a pushing force from one end to the opposite end through a wire made of a flexible material, the bending of the wire causes loss in transmission distance, thereby making it very difficult to transmit the force effectively (e.g., this situation may be analogized to pushing an object on the floor with a stick versus attempting to push the object with a rope). In other words, a movement distance on a load-applying side is reduced due to a decrease in a path length caused by bending, and accordingly, a movement distance on a load-receiving side becomes even smaller. Accordingly, in order to prevent such a loss in movement distance, it is necessary to avoid shortening of the path length, and this is achieved by fixing the path length on both sides using the guide tube 1170.

In some embodiments, to drive a cutting motion using the blade wire 307, the cutting should be performed while pushing the blade wire 307, and in this case, in order for the blade wire 307 to receive a force, a relatively stiff (i.e., not easily bendable) wire should be used as the blade wire 307. However, the stiff (i.e., not easily bendable) wire may have a small bendable range and may be permanently deformed when a force equal to or greater than a certain degree is applied.

In other words, in the case of a stiff (i.e., not easily bendable) wire, there is a minimum radius of curvature that may be bent and spread without permanent deformation. In other words, when the wire or the guide tube is curved below a specific radius of curvature, both the wire and the guide tube may undergo permanent deformation while being bent, thereby restricting the capacity to perform cutting while moving backward and forward. Thus, it is necessary to keep the blade wire 307 curved while having a gentle curvature.

Thus, in order to prevent the blade wire 307 from being rapidly bent while passing through the pulleys, a space, in which the blade wire 307 can be gently bent, is required between the jaw 1103 (i.e., the actuation rotation shaft 1145) and the end tool hub 1160 (i.e., the first rotation shaft 1141 that is a yaw rotation shaft).

To this end, according to the present disclosure, the first rotation shaft 1141, which is a yaw rotation shaft, and the actuation rotation shaft 1145 are separately provided, and the first rotation shaft 1141 and the actuation rotation shaft 1145 are spaced apart from each other by a certain distance, thereby forming a space in which the blade wire 307 and the guide tube 1170 can be gently bent.

As described above, since the blade wire 307 and the guide tube 1170 need to be connected to the blade 1175 through the end tool hub 1160, and a space in which the blade wire 307 and the guide tube 1170 can be bent in the end tool hub 1160 is necessary, in the present disclosure, 1) spaces, through which the blade wire 307/the guide tube 1170 can pass and simultaneously are bendable, that is, the pitch slit 1164 and the yaw slit 1165, are provided in the end tool hub 1160, 2) each of the rotation shafts is provided by being divided into two parts, and 3) a pitch round portion 1166 and a yaw round portion 1167 are additionally provided to guide the bending of the blade wire 307 and the guide tube 1170.

In other words, when one end portion of the guide tube 1170 is fixed in the connection part 400, and another end portion thereof moves while performing pitch and yaw motions, the guide tube 1170 is curved in a direction, in which the gentlest curvature (hereinafter, referred to as "maximum gentle curvature") can be achieved in response to a change in a distance between opposite end portions thereof. As such, by achieving the maximum gentle curvature of the natural state, the motion of the blade wire 307 is smooth and the permanent deformation does not occur.

Thus, in order to secure the maximum gentle curvature, the pitch slit 1164 and the yaw slit 1165 are provided on the path of the guide tube 1170, and furthermore, the pitch round portion 1166 and the yaw round portion 1167 may be additionally provided in the end tool hub 1160. Accordingly, the guide tube 1170 may take a shape closely approximating the maximum gentle curvature (even when the guide tube 1170 does not exactly follow the maximum gentle curvature).

Hereinafter, the end tool hub 1160 will be described in more detail.

### (End tool hub)

FIG. 9 is a perspective view illustrating the end tool hub of the surgical instrument for electrocautery of FIG. 2. FIGS. 10 and 11 are cutaway perspective views of the end tool hub of FIG. 9. FIGS. 12 and 13 are perspective views illustrating the end tool hub of FIG. 9. FIG. 14 is a side view illustrating the end tool hub of FIG. 9 and the guide tube. FIG. 15 is a plan view illustrating the end tool hub of FIG. 9 and the guide tube.

Referring to FIGS. 9 to 15, the end tool hub 1160 includes a body portion 1161, a first jaw pulley coupling portion 1162a, a second jaw pulley coupling portion 1162b, the first pitch pulley portion 1163a, the second pitch pulley portion 1163b, the pitch slit 1164, the yaw slit 1165, the pitch round portion 1166, the yaw round portion 1167, and the wire guide portion 1168. In an embodiment, the wire guide portion 1168 includes the first wire guide portion 1168a and the second wire guide portion 1168b.

The first jaw pulley coupling portion 1162a and the second jaw pulley coupling portion 1162b may be provided in the end tool hub 1160 at the distal end side. Here, the first jaw pulley coupling portion 1162a and the second jaw pulley coupling portion 1162b are configured to face each other, and the pulley 1111 and the pulley 1121 are accommodated therein. Here, the first jaw pulley coupling portion 1162a and the second jaw pulley coupling portion 1162b may be configured to be approximately parallel to a plane perpendicular to the first rotation shaft 1141 that is a yaw rotation shaft.

The first jaw pulley coupling portion 1162a and the second jaw pulley coupling portion 1162b are connected to each other by the body portion 1161. That is, the first jaw pulley coupling portion 1162a and the second jaw pulley coupling portion 1162b, which are parallel to each other, are coupled by the body portion 1161 provided in a direction approximately perpendicular to the first jaw pulley coupling portion 1162a and the second jaw pulley coupling portion 1162b, such that the first jaw pulley coupling portion 1162a, the second jaw pulley coupling portion 1162b, and the body portion 1161 form an approximately U-shape, in which the pulley 1111 and the pulley 1121 are accommodated.

In other words, it may be said that the first jaw pulley coupling portion 1162a and the second jaw pulley coupling portion 1162b are configured to extend in an X-axis direction from the body portion 1161.

Here, the pulley 1111, which is a first jaw pulley, is disposed close to the first jaw pulley coupling portion 1162a of the end tool hub 1160, and the pulley 1121, which is a second jaw pulley, is disposed close to the second jaw pulley coupling portion 1162b of the end tool hub 1160, and thus the yaw slit 1165 may be provided between the first jaw pulley coupling portion 1162a and the second jaw pulley coupling portion 1162b. In an embodiment, at least a portion of the blade assembly to be described later may be positioned in the yaw slit 1165. In other words, it may be said that at least a portion of the guide tube 1170 of the blade assembly may be positioned between the first jaw pulley coupling portion 1162a and the second jaw pulley coupling portion 1162b. As such, by positioning the blade assembly including the guide tube 1170 between the pulley 1111, which is a first jaw pulley, and the pulley 1121, which is a second jaw pulley, the end tool 1100 is capable of performing a cutting motion using the blade 1175 in addition to the pitch and yaw motions. This will be described in more detail later.

In an embodiment, a through hole is provided in the first jaw pulley coupling portion 1162a such that the first rotation shaft 1141 passes through the first jaw pulley coupling portion 1162a and the pulley 1111 and axially couples the first jaw pulley coupling portion 1162a to the pulley 1111. In an embodiment, a through hole is provided in the second jaw pulley coupling portion 1162b such that the first rotation shaft 1141 passes through the second jaw pulley coupling portion 1162b and the pulley 1121 and axially couples the second jaw pulley coupling portion 1162b to the pulley 1121.

Here, as described above, the first rotation shaft 1141, which is a yaw rotation shaft, may be provided by being divided into two parts of the first sub-shaft 1141a and the second sub-shaft 1141b, and the guide tube 1170 may pass between the first sub-shaft 1141a and the second sub-shaft 1141b of the first rotation shaft 1141.

In an embodiment, the yaw slit 1165 may be provided between the first jaw pulley coupling portion 1162a and the second jaw pulley coupling portion 1162b. Since the yaw slit 1165 is provided in the end tool hub 1160 as described above, the guide tube 1170 may pass through the inside of the end tool hub 1160.

In other words, the first rotation shaft 1141 is vertically separated into two parts without passing through the end tool hub 1160, and the yaw slit 1165 may be provided on a plane perpendicular to the first rotation shaft 1141 in the vicinity of the first rotation shaft 1141. Accordingly, the guide tube 1170 is movable (i.e., movable left and right) in the yaw slit 1165 while passing through the vicinity of the first rotation shaft 1141.

In an embodiment, the yaw round portion 1167 may be further provided in the body portion 1161. The yaw round portion 1167 may be provided to be rounded so as to have a predetermined curvature. For example, when viewed from a plane perpendicular to the first rotation shaft 1141 that is a yaw rotation shaft, the yaw round portion 1167 may be provided to be rounded so as to have a predetermined curvature. For example, the yaw round portion 1167 may be provided in a fan shape, and may be provided along a path in which the guide tube 1170 is bent on an XY plane. The yaw round portion 1167 may serve to guide the path of the guide tube 1170 when the end tool 1100 yaw-rotates.

The wire guide portion 1168, which guides a path of the wire passing through the inside of the end tool hub 1160, is provided at one side of the body portion 1161. Here, the wire guide portion 1168 includes the first wire guide portion 1168a and the second wire guide portion 1168b. Here, the first wire guide portion 1168a may be provided on an inner side surface of the first jaw pulley coupling portion 1162a. In an embodiment, the second wire guide portion 1168b may be provided on an inner side surface of the second jaw pulley coupling portion 1162b.

Here, the wire guide portion 1168 may be provided in a cylindrical shape with a cross section that is approximately semi-circular. In an embodiment, the semi-circular portion may be disposed to protrude toward the pulley 1111 and the pulley 1121. In other words, it may be said that the wire guide portion 1168 is configured to protrude toward a space provided by the first jaw pulley coupling portion 1162a, the second jaw pulley coupling portion 1162b, and the body portion 1161. In other words, it may be said that, in the wire guide portion 1168, a region adjacent to the first jaw pulley coupling portion 1162a and the second jaw pulley coupling portion 1162b is provided to have a cross section that is curved with a predetermined curvature.

Alternatively, in other words, it may be also said that the wire guide portion 1168 functions as a kind of pulley member, which guides the paths of the wire 305 and the wire 302 by winding the wire 305 and the wire 302 around an outer circumferential surface thereof. However, here, the wire guide portion 1168 is not a member that rotates around a predetermined axis as the original meaning pulley does, and it may be said that the wire guide portion 1168 is provided to be fixed as a portion of the end tool hub 1160 and performs some functions similar to those of a pulley as a wire wound therearound.

Here, the wire guide portion 1168 is illustrated in the drawing as being provided in a cylindrical shape with a cross section that is approximately semi-circular. That is, at least a portion of the cross section of the wire guide portion 1168 on the XY plane is illustrated as having a certain arc shape. However, the concept of the present disclosure is not limited thereto, and the cross section may be provided to have a predetermined curvature, such as an elliptical or parabolic shape, or may be shaped as a polygonal column with corners rounded to a certain extent. The cross section may thus be provided in various shapes and sizes suitable for guiding the paths of the wire 305 and the wire 302.

Here, a guide groove for guiding the paths of the wire 305 and the wire 302 well may be further provided in a portion of the wire guide portion 1168, which is in contact with the wire 305 and the wire 302. The guide groove may be provided in the form of a groove recessed to a certain depth from a protruding surface of the wire guide portion 1168.

Here, although the guide groove is illustrated in the drawing as being provided in the entire arc surface of the wire guide portion 1168, the concept of the present disclosure is not limited thereto, and the guide groove may be provided only in a portion of the arc surface of the wire guide portion 1168 as necessary.

As described above, by further forming the guide groove in the wire guide portion 1168, unnecessary friction between the wires is reduced, so that durability of the wires may be improved.

The first pitch pulley portion 1163a and the second pitch pulley portion 1163b, which serve as end tool pitch pulleys, may be provided on the end tool hub 1160 at the proximal end side. Here, the first pitch pulley portion 1163a and the second pitch pulley portion 1163b may be configured to face each other. Here, the first pitch pulley portion 1163a and the second pitch pulley portion 1163b may be configured to be approximately parallel to a plane perpendicular to the third rotation shaft 1143, which is a pitch rotation shaft.

For example, one end portion of the end tool hub 1160 is provided in a disk shape similar to a pulley, and grooves around which a wire may be wound may be provided on an outer circumferential surface of the one end portion, thereby forming the first pitch pulley portion 1163a and the second pitch pulley portion 1163b. The wire 303 and the wire 304, which are described above, are coupled to the first pitch pulley portion 1163a and the second pitch pulley portion 1163b, which serve as end tool pitch pulleys, and a pitch motion is performed while the end tool hub 1160 rotates around the third rotation shaft 1143.

In an embodiment, although not shown in the drawings, the pitch pulley may be provided as a separate member from the end tool hub 1160 and coupled to the end tool hub 1160.

The first pitch pulley portion 1163a and the second pitch pulley portion 1163b may be connected to each other by the body portion 1161. That is, as the first pitch pulley portion 1163a and the second pitch pulley portion 1163b, which are parallel to each other, are coupled to each other by the body portion 1161 provided in a direction approximately perpendicular to the first pitch pulley portion 1163a and the second pitch pulley portion 1163b, the first pitch pulley portion 1163a, the second pitch pulley portion 1163b, and the body portion 1161 may form an approximately U-shape.

In other words, it may be said that the first pitch pulley portion 1163a and the second pitch pulley portion 1163b are configured to extend from the body portion 1161 in the X-axis direction.

In an embodiment, a through hole is provided in the first pitch pulley portion 1163a so that the third rotation shaft 1143 may pass through the first pitch pulley portion 1163a. In an embodiment, a through hole is provided in the second pitch pulley portion 1163b so that the third rotation shaft 1143 may pass through the second pitch pulley portion 1163b.

In this case, as described above, the third rotation shaft 1143, which is a pitch rotation shaft, may be provided by being divided into two parts of the first sub-shaft 1143a and the second sub-shaft 1143b, and the guide tube 1170 may pass between the first sub-shaft 1143a and the second sub-shaft 1143b of the third rotation shaft 1143.

The pitch slit 1164 may be provided between the first pitch pulley portion 1163a and the second pitch pulley portion 1163b. Since the pitch slit 1164 is provided in the end tool hub 1160 as described above, the guide tube 1170 may pass through the inside of the end tool hub 1160.

In other words, the third rotation shaft 1143 is horizontally separated into two parts without passing through the end tool hub 1160, and the pitch slit 1164 may be provided on a plane perpendicular to the third rotation shaft 1143 in the vicinity of the third rotation shaft 1143. Accordingly, the guide tube 1170 is movable (movable up and down) in the pitch slit 1164 while passing through the vicinity of the third rotation shaft 1143.

In an embodiment, the pitch round portion 1166 may be further provided in the body portion 1161. The pitch round portion 1166 may be provided to be rounded to have a predetermined curvature. For example, when viewed from a plane perpendicular to the third rotation shaft 1143, which is a pitch rotation shaft, the pitch round portion 1166 may be provided to be rounded to have a predetermined curvature. For example, the pitch round portion 1166 may be provided in a fan shape, and provided along a path in which the guide tube 1170 is bent on the XZ plane. The pitch round portion 1166 may serve to guide the path of the guide tube 1170 when the end tool 1100 pitch-rotates.

Here, the pitch slit 1164 and the yaw slit 1165 may be provided to be connected to each other. Accordingly, the guide tube 1170 and the blade wire 307 positioned therein may be disposed to completely pass through the inside of the end tool hub 1160. In an embodiment, the blade 1175 coupled to one end portion of the blade wire 307 may linearly reciprocate inside the first jaw 1101 and the second jaw 1102.

As described above, since the blade wire 307 and the guide tube 1170 need to be connected to the blade 1175 through the end tool hub 1160, and a space in which the blade wire 307 and the guide tube 1170 can be bent in the end tool hub 1160 is necessary, in the present disclosure, 1) spaces, through which the blade wire 307/the guide tube 1170 can pass and simultaneously are bendable, that is, the pitch slit 1164 and the yaw slit 1165, are provided in the end tool hub 1160, 2) the rotation shafts are each provided by being divided into two parts, and 3) the pitch round portion 1166 and the yaw round portion 1167 are additionally provided to guide the bending of the blade wire 307/the guide tube 1170.

Hereinafter, the role and function of the wire guide portion 1168 will be described in more detail.

The wire guide portion 1168 may be in contact with the wire 305 and the wire 302 and may change the arrangement paths of the wire 305 and the wire 302 to a certain extent, thereby serving to increase rotation radii of the first jaw 1101 and the second jaw 1102.

That is, when the auxiliary pulleys are not disposed, each of the pulley 1111, which is a first jaw pulley, and the pulley 1121, which is a second jaw pulley, may rotate up to a right angle, but, according to an embodiment of the present disclosure, by additionally providing the wire guide portion 1168 in the end tool hub 1160, the maximum rotation angle of each pulley may be increased.

This enables a motion in which two jaws of the end tool 1100 have to be spread apart for an actuation motion while the two jaws are yaw-rotated together by 90°. In other words, the range of yaw rotation in which an actuation motion is possible may be increased through the configuration of the wire guide portion 1168 of the end tool hub 1160. In other words, the range of yaw rotation in which an actuation motion is possible may be increased through the configuration of the wire guide portion 1168 of the end tool hub 1160.

Furthermore, by forming the wire guide portion 1168 in the already-existing end tool hub 1160 without adding a separate structure such as an auxiliary pulley, it is possible to increase the range of rotation without requiring additional components and manufacturing processes.

As described above, since there is no need to additionally dispose a separate structure for increasing the rotation angle, the number of components can be reduced and the manufacturing process can be simplified. In addition, the length of the end tool is shortened by the size of the auxiliary pulley, thereby reducing the length of the end tool during a pitch motion and making it easier to perform surgical operations in a narrow space.

This will be described below in more detail.

In the end tool 1100 of the surgical instrument according to an embodiment of the present disclosure, the arrangement paths of the wires may be changed without a separate structure by forming the wire guide portion 1168 capable of changing the path of the wire on an inner side wall of the end tool hub 1160. As described above, by forming the wire guide portion 1168 in the end tool hub 1160 to change the arrangement paths of the wire 305 and the wire 302 to a certain extent, tangential directions of the wire 305 and the wire 302 are changed, and accordingly, rotation angles of the fastening members 323 and 326 that couple the respective wires to the pulleys may be increased.

That is, the fastening member 326 that couples the wire 302 to the pulley 1121 is rotatable until being positioned on a common internal tangent of the pulley 1121 and the wire guide portion 1168. Similarly, the fastening member (see 323 of FIG. 6) that couples the wire 305 to the pulley 1111 is rotatable until being positioned on a common internal tangent of the pulley 1111 and the wire guide portion 1168, so that a rotation angle of the fastening member (see 323 of FIG. 6) may be increased.

In other words, the wire 301 and the wire 305 wound around the pulley 1111 by the wire guide portion 1168 are positioned on one side of a plane perpendicular to the Y-axis and passing through the X-axis. Simultaneously, the wire 302 and the wire 306 wound around the pulley 1121 by the wire guide portion 1168 are positioned on another side of the plane perpendicular to the Y-axis and passing through the X-axis.

In other words, the pulley 1113 and the pulley 1114 are disposed on one side of the plane perpendicular to the Y-axis and passing through the X-axis, and the pulley 1123 and the pulley 1124 are disposed on another side of the plane perpendicular to the Y-axis and passing through the X-axis.

In other words, the wire 305 is positioned on the internal tangent of the pulley 1111 and the wire guide portion 1168, and the rotation angle of the pulley 1111 is increased due to the wire guide portion 1168. In an embodiment, the wire 302 is positioned on the internal tangent of the pulley 1121 and the wire guide portion 1168, and the rotation angle of the pulley 1121 is increased due to the wire guide portion 1168.

Compared to the surgical instrument of the embodiment in which a separate auxiliary pulley is provided, the length of the end tool of the surgical instrument according to the present embodiment, in which no auxiliary pulley is provided and the wire guide portion 1168 capable of changing the path of a wire is provided on an inner side wall of the end tool hub 1160, may be shortened. As the length of the end tool is shortened as described above, it becomes easier for the operator to manipulate the surgical instrument in a narrow surgical space within the human body, and side effects of the surgery may be reduced."

According to the present disclosure as described above, as the rotation radii of the pulley 1111, which is a first jaw pulley, and the pulley 1121, which is a second jaw pulley, increase, the yaw motion range in which normal opening/closing actuation and cutting motions can be performed may be expanded.

### (Actuation hub)

FIG. 16 is a set of a perspective view and a cutaway perspective view illustrating the actuation hub of the surgical instrument for electrocautery of FIG. 2. FIG. 17 is a view illustrating a state in which the guide tube, the blade wire, and the blade are mounted on the actuation hub illustrated in the cutaway perspective view of FIG. 16. FIG. 18 is an exploded perspective view illustrating the end tool of the surgical instrument for electrocautery of FIG. 2.

Referring to FIGS. 16 to 18, the actuation hub 1190 may be provided in a box shape having a hollow therein. In an embodiment, the actuation hub 1190 is coupled to each of the first jaw 1101 and the second jaw 1102. For example, the actuation hub 1190 is axially coupled to the first jaw 1101 by a first actuation rotation shaft 1145a. In an embodiment, the actuation hub 1190 is axially coupled to the second jaw 1102 by a second actuation rotation shaft 1145b. In this case, the first actuation rotation shaft 1145a and the second actuation rotation shaft 1145b may be positioned on the same line in a Z-axis direction.

In an embodiment, a tube seating portion 1190a may be provided inside the actuation hub 1190, and one end portion of the guide tube 1170 may be fixedly coupled to the tube seating portion 1190a.

In an embodiment, a blade accommodation portion 1190b may be provided inside the actuation hub 1190, and the blade 1175 may be accommodated in the blade accommodation portion 1190b.

In an embodiment, a wire through-hole 1190c may be provided between the tube seating portion 1190a and the blade accommodation portion 1190b inside the actuation hub 1190.

That is, the tube seating portion 1190a, the wire through-hole 1190c, and the blade accommodation portion 1190b are sequentially provided inside the actuation hub 1190, and the blade wire 307 may pass through the inside of the actuation hub 1190 to be connected to the blade 1175.

As described above, by providing the actuation hub 1190, to which the guide tube 1170 is coupled, between the first jaw 1101 and the second jaw 1102, the guide tube 1170 may not be curved, or the angle by which the guide tube 1170 is curved may be reduced, even when the first jaw 1101 or the second jaw 1102 rotates around the first rotation shaft 1141 or the actuation rotation shaft 1145.

For example, in a case in which the guide tube 1170 is directly coupled to the first jaw 1101 or the second jaw 1102, when the first jaw 1101 or the second jaw 1102 rotates, one end portion of the guide tube 1170 also rotates together with the first jaw 1101 or the second jaw 1102, causing the guide tube 1170 to be curved.

For example, in a case in which the guide tube 1170 is coupled to the actuation hub 1190, which is independent of the rotation of the jaw 1103, as in the present embodiment, even when the first jaw 1101 or the second jaw 1102 rotates, the guide tube 1170 may not be curved, or the angle by which the guide tube 1170 is curved may be reduced even when the guide tube 1170 is curved.

That is, by changing the direct connection between the guide tube 1170 and the jaw 1103 to an indirect connection by means of the actuation hub 1190, the degree to which the guide tube 1170 is curved due to the rotation of the jaw 1103 may be reduced.

### (First and second jaws and actuation motion)

Hereinafter, a coupling structure of the first jaw 1101 and the second jaw 1102 of the end tool 1100 of the surgical instrument 10 of FIG. 2 will be described in more detail.

Referring to FIGS. 19 to 24 and the like, the first jaw 1101 includes a movable coupling hole 1101c, a jaw pulley coupling hole 1101d, and a shaft pass-through portion 1101e.

The first jaw 1101 is provided in an overall elongated bar shape, and configured to be rotatable together with the pulley 1111 by being coupled to the pulley 1111 at one end portion thereof.

In an embodiment, the movable coupling hole 1101c, the jaw pulley coupling hole 1101d, and the shaft pass-through portion 1101e may be provided in the first jaw 1101 at a side coupled to the pulley 1111, that is, at the proximal end side.

Here, the movable coupling hole 1101c may be provided to have a predetermined curvature, and may be provided in an approximately elliptical shape. A shaft coupling portion 1111a of the pulley 1111, which will be described later, may be fitted into the movable coupling hole 1101c. Here, a short radius of the movable coupling hole 1101c may be configured to be substantially the same as or slightly greater than a radius of the shaft coupling portion 1111a. In an embodiment, a long radius of the movable coupling hole 1101c may be configured to be greater than the radius of the shaft coupling portion 1111a. Thus, in a state in which the shaft coupling portion 1111a of the pulley 1111 is fitted into the movable coupling hole 1101c of the first jaw 1101, the shaft coupling portion 1111a is movable to a certain extent in the movable coupling hole 1101c. This will be described in more detail below.

In an embodiment, the jaw pulley coupling hole 1101d is provided in the form of a cylindrical hole, and a jaw coupling portion 1111b of the pulley 1111, which will be described later, may be fitted into the jaw pulley coupling hole 1101d. Here, a radius of the jaw pulley coupling hole 1101d may be configured to be substantially the same as or slightly greater than a radius of the jaw coupling portion 1111b. Thus, the jaw coupling portion 1111b of the pulley 1111 may be configured to be rotatably coupled to the jaw pulley coupling hole 1101d of the first jaw 1101. This will be described in more detail below.

In an embodiment, the shaft pass-through portion 1101e may be provided in the first jaw 1101 at the distal end side relative to the movable coupling hole 1101c and the jaw pulley coupling hole 1101d. The shaft pass-through portion 1101e may be provided in the form of a hole, and the actuation rotation shaft 1145, which is a jaw rotation shaft, may be inserted through the shaft pass-through portion 1101e.

The second jaw 1102 includes a movable coupling hole 1102c, a jaw pulley coupling hole 1102d, and a shaft pass-through portion 1102e.

The second jaw 1102 is provided in an overall elongated bar shape, and configured to be rotatable together with the pulley 1121 by being coupled to the pulley 1121 at one end portion thereof.

In an embodiment, the movable coupling hole 1102c, the jaw pulley coupling hole 1102d, and the shaft pass-through portion 1102e may be provided in the second jaw 1102 at a side coupled to the pulley 1111, that is, at the proximal end side.

Here, the movable coupling hole 1102c may be provided to have a predetermined curvature, and may be provided in an approximately elliptical shape. A shaft coupling portion 1121a of the pulley 1121, which will be described later, may be fitted into the movable coupling hole 1102c. Here, a short radius of the movable coupling hole 1102c may be configured to be substantially the same as or slightly greater than a radius of the shaft coupling portion 1121a. In an embodiment, a long radius of the movable coupling hole 1102c may be configured to be greater than the radius of the shaft coupling portion 1121a. Thus, in a state in which the shaft coupling portion 1121a of the pulley 1121 is fitted into the movable coupling hole 1102c of the second jaw 1102, the shaft coupling portion 1121a is movable to a certain extent in the movable coupling hole 1102c. This will be described in more detail below.

In an embodiment, the jaw pulley coupling hole 1102d is provided in the form of a cylindrical hole, and a jaw coupling portion 1121b of the pulley 1121, which will be described later, may be fitted into the jaw pulley coupling hole 1102d. Here, a radius of the jaw pulley coupling hole 1102d may be configured to be substantially the same as or slightly greater than a radius of the jaw coupling portion 1121b. Thus, the jaw coupling portion 1121b of the pulley 1121 may be provided to be rotatably coupled to the jaw pulley coupling hole 1102d of the second jaw 1102. This will be described in more detail below.

In an embodiment, the shaft pass-through portion 1102e may be provided in the second jaw 1102 at the distal end side relative to the movable coupling hole 1102c and the jaw pulley coupling hole 1102d. The shaft pass-through portion 1102e may be provided in the form of a hole, and the actuation rotation shaft 1145 may be inserted through the shaft pass-through portion 1102e.

The pulley 1111, which is a first jaw pulley, may include the shaft coupling portion 1111a and the jaw coupling portion 1111b. The pulley 1111 may be provided entirely in the form of a rotatable disk, and the shaft coupling portion 1111a and the jaw coupling portion 1111b may be configured to protrude to a certain extent from one surface of the pulley 1111. As described above, the shaft coupling portion 1111a of the pulley 1111 may be fitted into the movable coupling hole 1101c of the first jaw 1101, and the jaw coupling portion 1111b of the pulley 1111 may be fitted into the jaw pulley coupling hole 1101d of the first jaw 1101. The pulley 1111 may be configured to rotate around the first rotation shaft 1141, which is an end tool jaw pulley rotation shaft.

In an embodiment, the pulley 1121, which is a second jaw pulley, may include the shaft coupling portion 1121a and the jaw coupling portion 1121b. The pulley 1121 may be provided entirely in the form of a rotatable disk, and the shaft coupling portion 1121a and the jaw coupling portion 1121b may be configured to protrude to a certain extent from one surface of the pulley 1121. As described above, the shaft coupling portion 1112a of the pulley 1112 may be inserted into the movable coupling hole 1102c of the second jaw 1102, and the jaw coupling portion 1112b of the pulley 1112 may be inserted into the jaw pulley coupling hole 1102d of the second jaw 1102. The pulley 1121 may be configured to rotate around the first rotation shaft 1141, which is an end tool jaw pulley rotation shaft.

The coupling relationship between the components described above is as follows.

The first rotation shaft 1141, which is an end tool jaw pulley rotation shaft, is sequentially inserted through the shaft coupling portion 1111a of the pulley 1111, the movable coupling hole 1101c of the first jaw 1101, the movable coupling hole 1102c of the second jaw 1102, and the shaft coupling portion 1121a of the pulley 1121.

The first actuation rotation shaft 1145a is sequentially inserted through the shaft pass-through portion 1101e of the first jaw 1101 and the actuation hub 1190 The second actuation rotation shaft 1145b is sequentially inserted through the shaft pass-through portion 1102e of the second jaw 1102 and the actuation hub 1190.

The shaft coupling portion 1111a of the pulley 1111 is fitted into the movable coupling hole 1101c of the first jaw 1101, and the jaw coupling portion 1111b of the pulley 1111 is fitted into the jaw pulley coupling hole 1101d of the first jaw 1101.

At this time, the jaw pulley coupling hole 1101d of the first jaw 1101 and the jaw coupling portion 1111b of the pulley 1111 are rotatably coupled to each other along an axis, and the movable coupling hole 1101c of the first jaw 1101 and the shaft coupling portion 1111a of the pulley 1111 are movably coupled to each other (here, "movably coupled" means that the shaft coupling portion 1111a of the pulley 1111 is coupled so as to be movable to a certain extent within the movable coupling hole 1101c of the first jaw 1101).

The shaft coupling portion 1121a of the pulley 1121 is fitted into the movable coupling hole 1102c of the second jaw 1102, and the jaw coupling portion 1121b of the pulley 1121 is fitted into the jaw pulley coupling hole 1102d of the second jaw 1102.

At this time, the jaw pulley coupling hole 1102d of the second jaw 1101 and the jaw coupling portion 1121b of the pulley 1121 are rotatably coupled to each other along an axis, and the movable coupling hole 1102c of the second jaw 1102 and the shaft coupling portion 1121a of the pulley 1121 are movably coupled to each other.

Here, the pulley 1111 and the pulley 1121 rotate around the first rotation shaft 1141, which is an end tool jaw pulley rotation shaft. In an embodiment, the first jaw 1101 and the second jaw 1102 rotate around the actuation rotation shaft 1145. That is, the pulley 1111 and the first jaw 1101 have different axes of rotation. Similarly, the pulley 1121 and the second jaw 1102 have different axes of rotation.

That is, although the rotation angle of the first jaw 1101 is limited to a certain extent by the movable coupling hole 1101c, the first jaw 1101 essentially rotates around the actuation rotation shaft 1145, which is a jaw rotation shaft. Similarly, although the rotation angle of the second jaw 1102 is limited to a certain extent by the movable coupling hole 1102c, the second jaw 1102 essentially rotates around the actuation rotation shaft 1145, which is a jaw rotation shaft.

An amplification of the grip force resulting from the coupling relationship between the above-described components will now be described.

In the surgical instrument 10 according to an embodiment of the present disclosure, the coupling structure between the first jaw 1101 and the second jaw 1102 forms an X-shaped structure, and thus, when the first jaw 1101 and the second jaw 1102 rotate in a direction of approaching each other (i.e., when the first jaw 1101 and the second jaw 1102 are closed), the grip force in the closing direction increases further. This will be described below in more detail.

As described above, in the opening and closing motions of the first jaw 1101 and the second jaw 1102, there are two shafts that serve as their respective centers of rotation. That is, the first jaw 1101 and the second jaw 1102 perform the opening and closing motion around two shafts including the first rotation shaft 1141 and the actuation rotation shaft 1145. At this time, the centers of rotation of the first jaw 1101 and the second jaw 1102 become the actuation rotation shaft 1145, and the centers of rotation of the pulley 1111 and the pulley 1121 become the first rotation shaft 1141. At this time, the first rotation shaft 1141 serves as a shaft whose position remains relatively fixed, whereas the actuation rotation shaft 1145 serves as a shaft that moves linearly in position. In other words, when the pulley 1111 and the pulley 1121 rotate while the position of the first rotation shaft 1141 remains fixed, the actuation rotation shaft 1145, which serves as the rotation shaft of the first jaw 1101 and the second jaw 1102 moves backward and forward, thereby opening and closing the first jaw 1101 and the second jaw 1102. This will be described below in more detail.

In FIG. 23, r1 is a distance from the jaw coupling portion 1121b of the pulley 1121 to the shaft coupling portion 1121a, and a length thereof is constant. Thus, a distance from the first rotation shaft 1141 inserted into the shaft coupling portion 1121a to the jaw coupling portion 1121b is also constant as r1.

In an embodiment, r2 of FIG. 23 is a distance from the jaw pulley coupling hole 1102d of the second jaw 1102 to the shaft pass-through portion 1102e, and a length thereof is constant. Thus, a distance from the jaw coupling portion 1121b of the pulley 1121 inserted into the jaw pulley coupling hole 1102d to the actuation rotation shaft 1145 inserted into the shaft pass-through portion 1102e is also constant as r2.

That is, the lengths of r1 and r2 remain constant. Accordingly, when the pulley 1111 and the pulley 1121 rotate in the directions of an arrow B1 of FIG. 22 and an arrow B2 of FIG. 23, respectively, around the first rotation shaft 1141 to perform a closing motion, the first jaw 1101 and the second jaw 1102 rotate around the actuation rotation shaft 1145 as the angle between r1 and r2 changes while the lengths of r1 and r2 remain constant, and at this time, the actuation rotation shaft 1145 itself also linearly moves (i.e., moves forward/backward) by as much as an arrow C1 of FIG. 22 and an arrow C2 of FIG. 23.

That is, assuming that the position of the first rotation shaft 1141, which is an end tool jaw pulley rotation shaft, is fixed, when the first jaw 1101 and the second jaw 1102 are closed, a force is applied in a direction in which the actuation rotation shaft 1145, which is a jaw rotation shaft, moves forward (i.e., toward the distal end). As a result, the grip force in the closing direction of the first jaw 1101 and the second jaw 1102 increases further.

In other words, since the lengths of r1 and r2 remain constant while the second jaw 1102 rotates around the actuation rotation shaft 1145, when the pulley 1121 rotates around the first rotation shaft 1141, the angle between r1 and r2 changes while the lengths of r1 and r2 remain constant. That is, θ2, which is an angle between r1 and r2 in a state in which the second jaw 1102 is open as shown in FIG. 23A, is greater than θ1, which is an angle between r1 and r2 in a state in which the second jaw 1102 is closed as shown in FIG. 23B.

Thus, when the second jaw 1102 rotates from the open state to the closed state, the angle between r1 and r2 changes, and a force is applied in a direction in which the actuation rotation shaft 1145 moves forward.

In this case, since the first rotation shaft 1141 is a shaft whose position is relatively fixed, the actuation rotation shaft 1145 moves forward in the direction of the arrow C1 of FIG. 22 and the direction of the arrow C2 of FIG. 23, and the grip force is further increased in a direction in which the second jaw 1102 is closed.

In other words, when the pulley 1111 and the pulley 1121 rotate around the first rotation shaft 1141, which is a shaft whose relative position is fixed, the angle θ between r1 and r2 changes while the distance between r1 and r2 remains constant. In an embodiment, when the angle θ changes as described above, the first jaw 1101 and the second jaw 1102 push or pull the actuation rotation shaft 1145, and thus the actuation rotation shaft 1145 moves forward or backward. In this case, when the first jaw 1101 and the second jaw 1102 rotate in the direction of closing, the grip force is further increased as the actuation rotation shaft 1145 moves forward in the directions of the arrow C1 of FIG. 22 and the arrow C2 of FIG. 23. In an embodiment, when the first jaw 1101 and the second jaw 1102 rotate in the direction of opening, the actuation rotation shaft 1145 moves backward in a direction opposite to the arrow C1 of FIG. 22 and the arrow C2 of FIG. 23.

With this configuration, the grip force becomes stronger when the first jaw 1101 and the second jaw 1102 are closed, thereby allowing a surgical operator to perform the actuation motion more powerfully with less force.

### (Components associated with cautery and cutting)

Subsequently, referring to FIGS. 2 to 24 and the like, the end tool 1100 according to an embodiment of the present disclosure may include the first jaw 1101, the second jaw 1102, a first electrode 1151, a second electrode 1152, the guide tube 1170, and the blade 1175 in order to perform cauterizing and cutting motions.

Here, components related to the driving of the blade, such as the guide tube 1170 and the blade 1175, may be collectively referred to as a blade assembly. In an embodiment of the present disclosure, by positioning the blade assembly including the guide tube 1170 and the blade 1175 between the pulley 1111, which is a first jaw pulley, and the pulley 1121, which a second jaw pulley, the end tool 1100 is capable of performing a cutting motion using the blade 1175 in addition to the pitch and yaw motions. This will be described in more detail.

As described above, the first jaw 1101 is connected to the first jaw pulley 1111 and rotates around the first rotation shaft 1141 together with the first jaw pulley 1111 when the first jaw pulley 1111 rotates around the first rotation shaft 1141.

In an embodiment, the first electrode 1151 may be provided on a surface of the first jaw 1101 facing the second jaw 1102. In an embodiment, the second electrode 1152 may be provided on a surface of the second jaw 1102 facing the first jaw 1101.

At this time, a slit 1151a may be provided in the first electrode 1151, and the blade 1175 may move along the slit 1151a. In an embodiment, a slit 1152a may be provided in the second electrode 1152, and the blade 1175 may move along the slit 1152a.

In an embodiment, although not shown in the drawings, a spacer (not shown) may be provided between the first jaw 1101 and the first electrode 1151, and a spacer (not shown) may be provided between the second jaw 1102 and the second electrode 1152. The spacers (not shown) may include an insulating material such as ceramic. Alternatively, the first jaw 1101 and the second jaw 1102 may themselves be made of a nonconductor such that the first electrode 1151 and the second electrode 1152 may be maintained to be insulated from each other without a separate insulator until the first electrode 1151 and the second electrode 1152 are in contact with each other.

In an embodiment, although not shown in the drawings, one or more sensors (not shown) may be further provided on at least one of the first jaw 1101 or the second jaw 1102. The sensor (not shown) may be configured to measure at least some of current, voltage, resistance, impedance, and temperature during the cautery by locating tissue between the first jaw 1101 and the second jaw 1102 and passing a current through the first electrode 1151 and the second electrode 1152.

Alternatively, without providing a separate sensor, monitoring and control of at least some of current, voltage, resistance, impedance, and temperature may be directly performed by a generator (not illustrated) that supplies power to the electrodes.

An edge portion provided sharply and configured to cut tissue may be provided in one region of the blade 1175. The tissue positioned between the first jaw 1101 and the second jaw 1102 may be cut as at least a portion of the blade 1175 moves between the distal end 1104 and the proximal end 1105 of the end tool 1100.

Here, the end tool 1100 of the surgical instrument for electrocautery 10 according to an embodiment of the present disclosure includes the guide tube 1170 and the blade 1175 positioned between the pulley 1111 and the pulley 1121. In an embodiment, by providing the guide tube 1170 and the blade 1175, a multi-joint and multi-degree-of-freedom surgical instrument capable of performing pitch, yaw, and actuation motions may also be capable of performing cauterization and cutting. This will be described below in more detail.

To date, various types of surgical instruments for electrocautery have been developed. Among the various types of surgical instruments for electrocautery, a blood vessel resection device called "Advanced Energy Device" or "Vessel Sealer" has a sensing function added to the existing bipolar cautery method, so that power of different polarities may be supplied to two electrodes, and after denaturing a vessel with the heat generated therefrom for hemostasis, the stanched part may be cut with a blade. At this time, the impedance of the tissue (or blood vessel) while the current is flowing is measured to determine whether the cauterization is completed, and when the cauterization is completed, the current supply is automatically stopped, and the tissue is cut with the blade.

In the case of such a bipolar-type blood vessel resection device, it is essential to have a blade to cut the tissue after cauterization, and the end tool needs to be equipped with a mechanism for facilitating a linear motion of the blade, and thus joint movements such as pitch/yaw movements are not possible in most cases.

In an embodiment, there have been attempts to implement joint movements using flexible joints with multiple nodes connected in the bipolar-type blood vessel resection device, but in this case, a rotation angle is limited and it is difficult to achieve accurate motion control of the end tool.

In an embodiment, in the case of a method that utilizes vibration of ultrasonic waves to perform hemostasis and cutting, it is not feasible to provide joints due to the physical properties of ultrasonic waves.

To address these problems, the end tool 1100 of the surgical instrument for electrocautery 10 according to an embodiment of the present disclosure includes the guide tube 1170 positioned between the pulley 1111 and the pulley 1121, and the blade 1175 that moves between a first position and a second position in response to the movement of the blade wire 307 positioned inside the guide tube 1170. In an embodiment, by providing the guide tube 1170 and the blade 1175 as described above, a bipolar-type surgical instrument for cauterizing and cutting tissue may also be configured to perform pitch, yaw, and actuation motions using a pulley/wire mechanism.

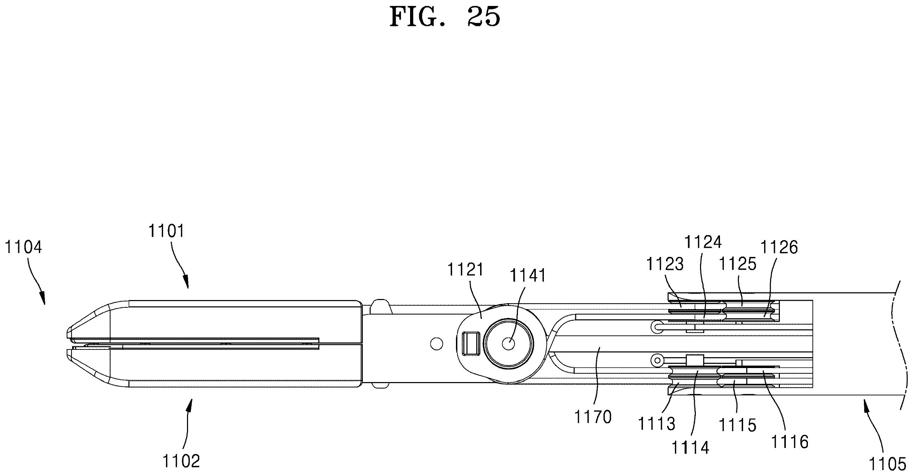

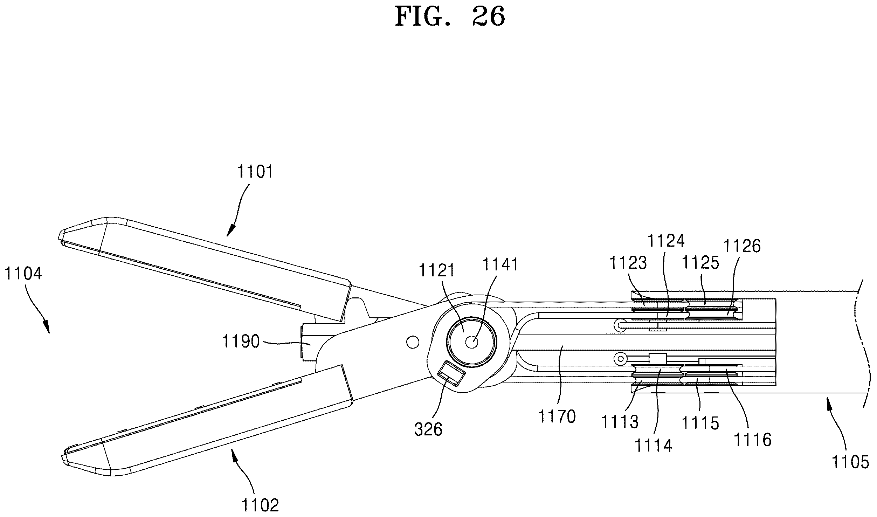

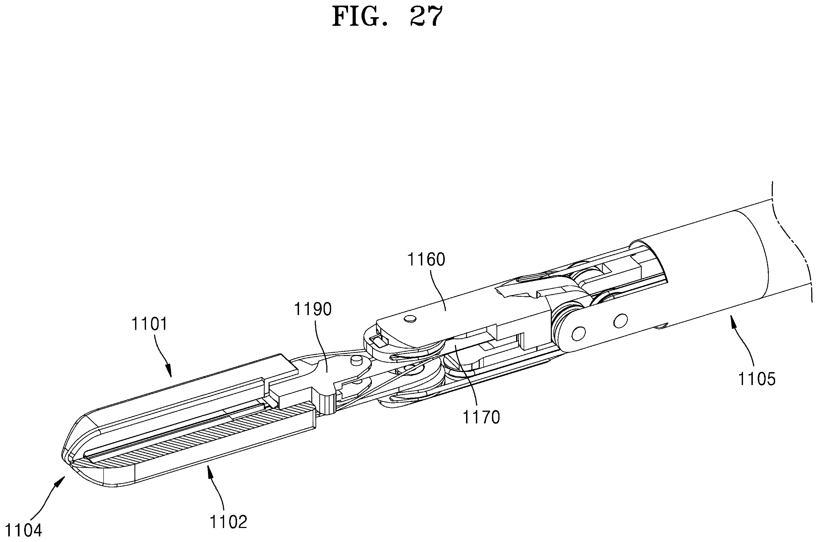

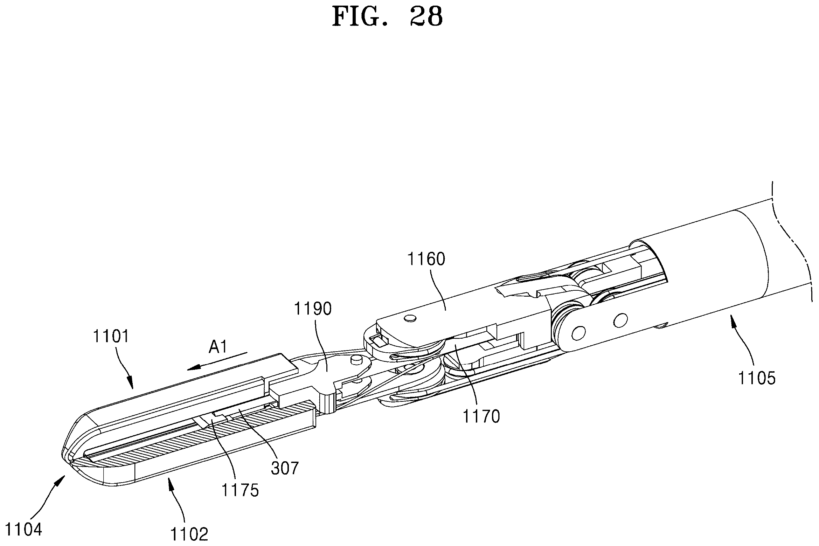

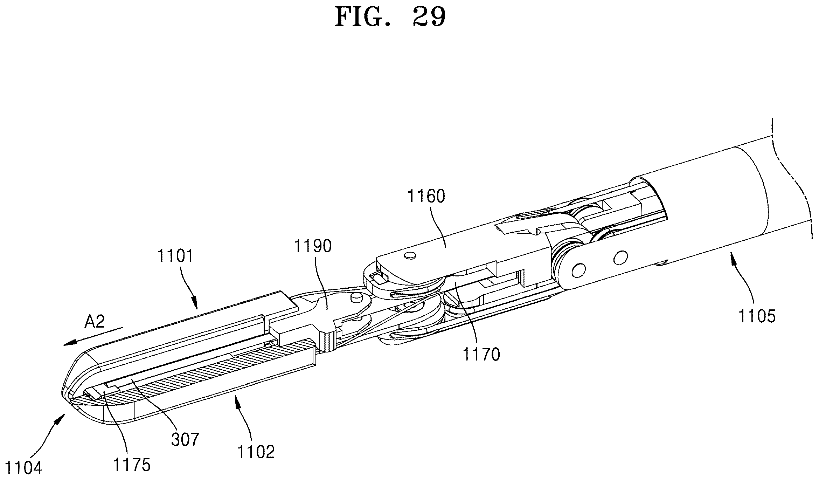

FIG. 25 is a view illustrating a state in which the end tool of the surgical instrument for electrocautery of FIG. 2 is closed, and FIG. 26 is a view illustrating a state in which the end tool of the surgical instrument for electrocautery of FIG. 2 is opened. FIG. 27 is a view illustrating a state in which the blade wire 307 and the blade 1175 are positioned at a first position, FIG. 28 is a view illustrating a state in which the blade wire 307 and the blade 1175 are positioned at a second position, and FIG. 29 is a view illustrating a state in which the blade wire 307 and the blade 1175 are positioned at a third position.

Referring to FIGS. 25 to 29, it may be said that the tissue between the first jaw 1101 and the second jaw 1102 is cut as the cutting motion of FIGS. 27 to 29 is performed in a state in which the first jaw 1101 and the second jaw 1102 are closed as shown in FIG. 25.

Here, the first position shown in FIG. 27 may be defined as a state in which the blade 1175 is maximally drawn in toward the proximal end 1105 of the end tool 1100. Alternatively, the first position may be defined as a state in which the blade 1175 is positioned adjacent to the pulley 1111/pulley 1121.

In an embodiment, the third position shown in FIG. 29 may be defined as a state in which the blade 1175 is maximally withdrawn toward the distal end 1104 of the end tool 1100. Alternatively, the third position may be defined as a state in which the blade 1175 is maximally spaced away from the pulley 1111/pulley 1121.

First, as shown in FIG. 26, a tissue to be cut is positioned between the first jaw 1101 and the second jaw 1102 in a state in which the first jaw 1101 and the second jaw 1102 are opened, and then an actuation motion is performed to close the first jaw 1101 and the second jaw 1102 as shown in FIG. 25.

Next, as shown in FIG. 27, in a state in which the blade wire 307 and the blade 1175 are positioned at the first position, currents of different polarities are applied to the first electrode 1151 and the second electrode 1152 to cauterize the tissue between the first jaw 1101 and the second jaw 1102. At this time, a generator (not shown) configured to supply power to the electrodes may perform monitoring of at least some of current, voltage, resistance, impedance, and temperature by itself, and may stop supplying power when the cauterization is completed.

In a state in which the cautery is completed as described above, when the blade wire 307 moves sequentially in the direction of an arrow A1 of FIG. 17 and an arrow A2 of FIG. 29, the blade 1175 coupled to the blade wire 307 moves from the first position at the proximal end 1105 of the end tool 1100 toward the third position at the distal end 1104 of the end tool 1100, sequentially reaching the positions shown in FIGS. 28 and 29.

As such, the blade 1175 cuts the tissue between the first jaw 1101 and the second jaw 1102 while moving in the X-axis direction.

However, the linear motion of the blade 1175 here does not necessarily refer to a motion in a completely straight line, but should be understood as a motion of the blade 1175 to the extent that the blade 1175 is able to cut the tissue while achieving a linear motion when viewed as a whole, even though the motion is not in a completely straight line, for example, the middle portion of the straight line is bent by a certain angle or there is a section having a gentle curvature in a certain section.

In an embodiment, in this state, when the blade wire 307 is pulled in the opposite direction, the blade 1175 coupled to the blade wire 307 also returns to the first position.

According to the present disclosure, a multi-joint, multi-degree-of-freedom surgical instrument capable of pitch, yaw, and actuation motions can additionally perform cauterizing and cutting motions.

### (Manipulation part)

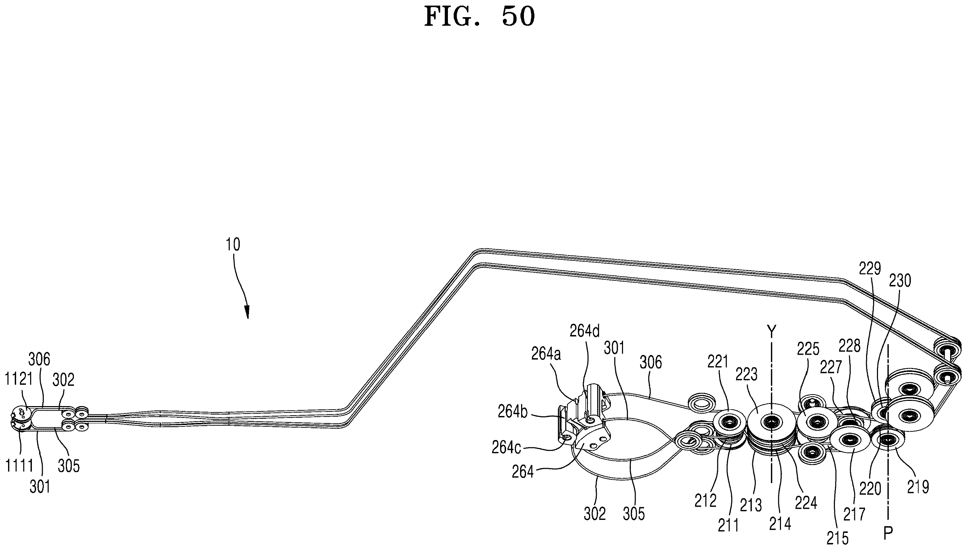

FIG. 47 is a perspective view illustrating the surgical instrument for electrocautery of FIG. 2, and FIGS. 48 and 49 are perspective views illustrating the manipulation part of the surgical instrument for electrocautery of FIG. 2. FIG. 50 is a view schematically illustrating only a configuration of pulleys and wires that constitute joints of the surgical instrument for electrocautery of FIG. 2.

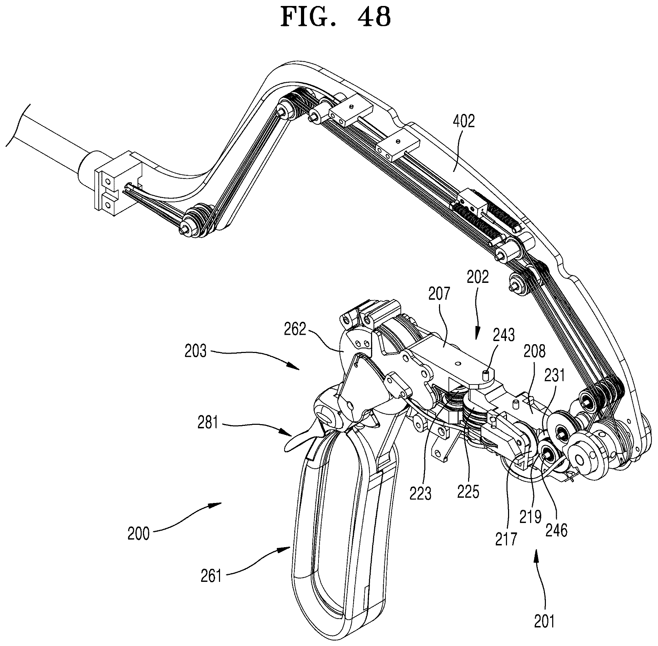

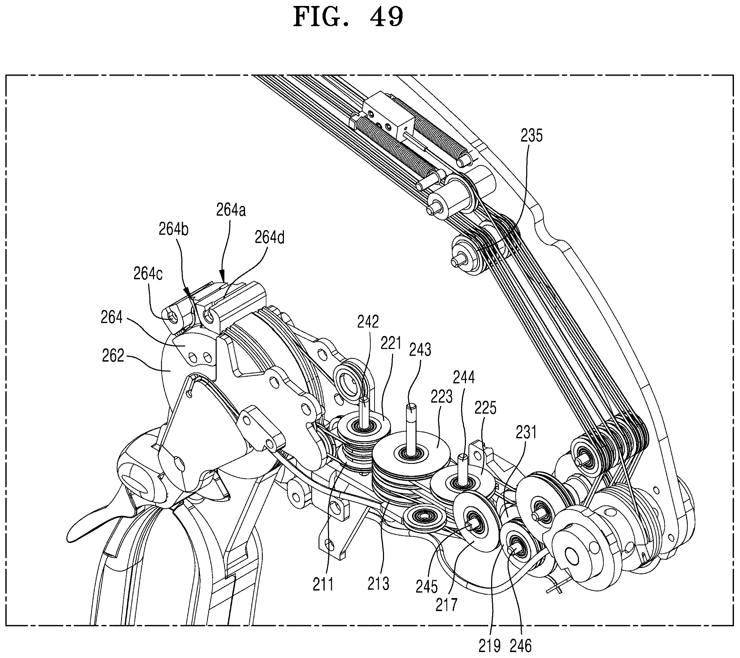

Referring to FIGS. 2 to 24 and FIGS. 47 to 49, the manipulation part 200 of the surgical instrument for electrocautery 10 according to an embodiment of the present disclosure includes a first handle 204 grippable by a user, the actuation-manipulation portion 203 configured to control an actuation motion of the end tool 1100, the yaw-manipulation portion 202 configured to control a yaw motion of the end tool 1100, and a pitch-manipulation portion 201 configured to control a pitch motion of the end tool 1100. Here, it is understood that only the components related to the pitch/yaw/actuation motions of the surgical instrument for electrocautery 10 are illustrated in FIGS. 48 and 49.

In an embodiment, the manipulation part 200 of the surgical instrument for electrocautery 10 further includes the cutting-manipulation portion 280 configured to control the movement of the blade 1175 of the end tool 1100 to perform cutting, and a sealing-manipulation portion 270 configured to control the supply of electric energy to the first electrode 1151 and the second electrode 1152 of the end tool 1100 to perform cauterization.

The manipulation part 200 may include the pulley 211, a pulley 212, a pulley 213, a pulley 214, a pulley 215, a pulley 217, a pulley 218, a pulley 219, and the pulley 220 that are associated with a rotational motion of the first jaw 1101. In an embodiment, the manipulation part 200 may include a pulley 221, a pulley 222, a pulley 223, a pulley 224, a pulley 225, a pulley 227, a pulley 228, a pulley 229, and a pulley 230, which are associated with a rotational motion of the second jaw 1102. In an embodiment, the manipulation part 200 may include a pulley 262 associated with a rotational motion of the first jaw and the second jaw. In an embodiment, the manipulation part 200 may include a pulley 231 associated with a pitch motion. In an embodiment, the manipulation part 200 may include relay pulleys 235 disposed at intervals along the bent portion 402 of the connection part 400.

Here, the pulleys facing each other are illustrated in the drawings as being provided parallel to each other, but the concept of the present disclosure is not limited thereto, and each of the pulleys may be variously provided with a position and a size suitable for the configuration of the manipulation part.

In an embodiment, the manipulation part 200 according to an embodiment of the present disclosure may include a rotation shaft 241, a rotation shaft 242, a rotation shaft 243, a rotation shaft 244, a rotation shaft 245, and a rotation shaft 246. Here, the rotation shaft 241 may function as a manipulation part actuation rotation shaft, and the rotation shaft 242 may function as a manipulation part first yaw sub-rotation shaft. In an embodiment, the rotation shaft 243 may function as a manipulation part yaw main rotation shaft, and the rotation shaft 244 may function as a manipulation part yaw second sub-rotation shaft. In an embodiment, the rotation shaft 245 may function as a manipulation part pitch sub-rotation shaft, and the rotation shaft 246 may function as a manipulation part pitch main rotation shaft.

The rotation shaft 241, the rotation shaft 242, the rotation shaft 243, the rotation shaft 244, the rotation shaft 245, and the rotation shaft 246 may be sequentially disposed from a distal end 205 toward a proximal end 206 of the manipulation part 200.

Each of the rotation shafts 241, 242, 243, 244, 245, and 246 may be fitted into one or more pulleys, which will be described in detail later.

The pulley 262 functions as an actuation pulley of the first and second jaws, and may be referred to as a manipulation part actuation pulley.

The pulley 211 and the pulley 212 function as manipulation part first-jaw first-yaw-sub-pulleys, the pulley 221 and the pulley 222 function as manipulation part second-jaw first-yaw-sub-pulleys, and these components may also be collectively referred to as manipulation part first yaw sub-pulleys.

The pulley 213 and the pulley 214 function as manipulation part first-jaw yaw main pulleys, the pulley 223 and the pulley 224 function as manipulation part second-jaw yaw main pulleys, and these components may also be collectively referred to as manipulation part yaw main pulleys.

The pulley 215 functions as a manipulation part first-jaw second-yaw-sub-pulley, the pulley 225 functions as a manipulation part second-jaw second-yaw-sub-pulley, and these components may also be collectively referred to as manipulation part second yaw sub-pulleys.

The pulley 217 and the pulley 218 function as manipulation part first-jaw pitch sub-pulleys, the pulley 227 and the pulley 228 function as manipulation part second-jaw pitch sub-pulleys, and these components may also be collectively referred to as manipulation part pitch sub-pulleys.

The pulley 219 and the pulley 220 function as manipulation part first-jaw pitch main pulleys, and the pulley 229 and the pulley 230 function as manipulation part second-jaw pitch main pulleys, and these components may also be collectively referred to as manipulation part pitch main pulleys.

The pulley 231 functions as an manipulation part pitch wire main pulley, and may include a pulley (not shown) that functions as a manipulation part pitch wire sub-pulley.

The above components are classified from the perspective of the manipulation part for each motion (pitch/yaw/actuation) as follows.

The pitch-manipulation portion 201 configured to control a pitch motion of the end tool 1100 may include the pulley 217, the pulley 218, the pulley 219, the pulley 220, the pulley 227, the pulley 228, the pulley 229, the pulley 230, and the pulley 231. In an embodiment, the pitch-manipulation portion 201 may include the rotation shaft 245 and the rotation shaft 246. In an embodiment, the pitch-manipulation portion 201 may further include a pitch frame 208.

The yaw-manipulation portion 202 configured to control a yaw motion of the end tool 1100 may include the pulley 211, the pulley 212, the pulley 213, the pulley 214, the pulley 215, the pulley 221, the pulley 222, the pulley 223, the pulley 224, and the pulley 225. In an embodiment, the yaw-manipulation portion 202 may include the rotation shaft 242, the rotation shaft 243, and the rotation shaft 244. In an embodiment, the yaw-manipulation portion 202 may further include a yaw frame 207.

The actuation-manipulation portion 203 configured to control an actuation motion of the end tool 1100 may include the pulley 262 and the rotation shaft 241.

Hereinafter, each component of the manipulation part 200 will be described in more detail.

The first handle 204 may be configured to be gripped by a user with a hand, and in particular, may be configured to allow the user to wrap their palm around the first handle 204. In an embodiment, the actuation-manipulation portion 203 and the yaw-manipulation portion 202 are provided on the first handle 204, and the pitch-manipulation portion 201 is provided on one side of the yaw-manipulation portion 202. In an embodiment, another end portion of the pitch-manipulation portion 201 is connected to the bent portion 402 of the connection part 400.

The actuation-manipulation portion 203 may include an actuation lever 261, the actuation pulley 262, and an actuation restoring elastic member 263.

Here, the actuation lever 261 may be provided in a finger ring shape and may function as a second handle.

Here, the rotation shaft 241, which is an actuation rotation shaft, may be provided to have a certain angle with respect to the XZ plane on which the connection part 400 is provided.

For example, the rotation shaft 241 may be provided in a direction parallel to the Y-axis, and in this state, when the pitch-manipulation portion 201 or the yaw-manipulation portion 202 rotates, a coordinate system of the actuation-manipulation portion 203 may change relatively. The concept of the present disclosure, however, is not limited thereto, and the rotation shaft 241 may be provided in various directions so as to be suitable for a structure of the hand of the user gripping the actuation-manipulation portion 203 according to an ergonomic design.

In some embodiments, the actuation pulley 262 and the actuation lever 261 may be fixedly coupled to each other or may be provided as a single member. Accordingly, the actuation pulley 262 may rotate along with the rotation of the actuation lever 261.

Here, the actuation pulley 262 may be configured as a single pulley or two pulleys fixedly coupled to each other.

The yaw-manipulation portion 202 may include the rotation shaft 242, the rotation shaft 243, the pulleys 213 and 214, which are manipulation part first jaw yaw main pulleys, the pulleys 223 and 224, which are manipulation part second jaw yaw main pulleys, and the yaw frame 207. In an embodiment, the yaw-manipulation portion 202 may further include the pulleys 211 and 212, which are manipulation part first-jaw first-yaw-sub-pulleys provided on one side of the pulley 213 and one side of the pulley 214, respectively, and the pulleys 221 and 222 that are manipulation part second-jaw first-yaw-sub-pulleys provided on one side of the pulley 221 and one side of the pulley 222, respectively. In an embodiment, the yaw-manipulation portion 202 may further include the pulley 215, which is a manipulation part first-jaw second-yaw-sub-pulley provided on another side of the pulley 213 and the pulley 214, and the pulley 225 that is a manipulation part second-jaw second-yaw-sub-pulley provided on another side of the pulley 223 and the pulley 224. Here, the pulley 215 and the pulley 225 may be coupled to the pitch frame 208 to be described later.

Here, it is illustrated in the drawings that the yaw-manipulation portion 202 includes the pulleys 213 and 214 and the pulleys 223 and 224, wherein the pulleys 213 and 214 and the pulleys 223 and 224 are each provided with two pulleys configured to face each other and independently rotatable, but the concept of the present disclosure is not limited thereto. That is, one or more pulleys having the same diameter or different diameters may be provided according to the configuration of the yaw-manipulation portion 202.

For example, the rotation shaft 242, which is a manipulation part first yaw sub-rotation shaft, is provided on one side of the actuation-manipulation portion 203 on the first handle 204, and the rotation shaft 243, which is a manipulation part yaw main rotation shaft, is provided on one side of the rotation shaft 242. At this time, the first handle 204 is configured to rotate around the rotation shaft 243.

Here, the rotation shaft 243 may be provided to have a certain angle with respect to the XY plane on which the connection part 400 is provided. For example, the rotation shaft 243 may be provided in a direction parallel to the Z-axis, and in this state, when the pitch-manipulation portion 201 rotates, a coordinate system of the rotation shaft 243 may change relatively as described above. The concept of the present disclosure, however, is not limited thereto, and the rotation shaft 243 may be provided in various directions so as to be suitable for a structure of the hand of the user gripping the manipulation part 200 according to an ergonomic design.

In an embodiment, the pulleys 213 and 214 and the pulleys 223 and 224 are coupled to the rotation shaft 243 so as to be rotatable around the rotation shaft 243. In an embodiment, the wire 301 or the wire 305, which is a first jaw wire, may be wound around the pulleys 213 and 214, and the wire 302 or the wire 306, which is a second jaw wire, may be wound around the pulleys 223 and 224. In this case, the pulleys 213 and 214 and the pulleys 223 and 224 may each be configured as two pulleys configured to face each other and independently rotatable. Accordingly, a wire being wound and a wire being released may be wound around respective separate pulleys so that the wires may perform motions without interference with each other.

The yaw frame 207 rigidly connects the first handle 204, the rotation shaft 242, and the rotation shaft 243 to each other, and the actuation-manipulation portion 203, to which the rotation shaft 241 and the actuation pulley 262 are coupled, is rigidly connected to the yaw frame 207 either directly or through an relay member, thereby allowing the first handle 204, the yaw-manipulation portion 202, and the actuation-manipulation portion 203 to integrally yaw-rotate around the rotation shaft 243.

The pitch-manipulation portion 201 may include the rotation shaft 246, the pulley 219 and the pulley 220, which are manipulation part first jaw pitch main pulleys, the pulleys 229 and 230, which are manipulation part second jaw pitch main pulleys, and the pitch frame 208. In an embodiment, the pitch-manipulation portion 201 may further include the rotation shaft 245, the pulleys 217 and 218, which are manipulation part first jaw pitch sub-pulleys provided on one side of the pulley 219 and one side of the pulley 220, respectively, and the pulleys 227 and 228, which are manipulation part second jaw pitch sub-pulleys provided on one side of the pulley 229 and one side of the pulley 230, respectively. The pitch-manipulation portion 201 may be connected to the bent portion 402 of the connection part 400 through the rotation shaft 246.

For example, the pitch frame 208 is a base frame of the pitch-manipulation portion 201, and the rotation shaft 243 is rotatably coupled to one end portion thereof. That is, the yaw frame 207 is configured to rotate around the rotation shaft 243 with respect to the pitch frame 208.

As described above, since the yaw frame 207 connects the first handle 204, the rotation shaft 243, the rotation shaft 241, and the rotation shaft 242 to each other, and the yaw frame 207 is also axially coupled to the pitch frame 208, when the pitch frame 208 is pitch-rotated around the rotation shaft 246, the yaw frame 207 connected to the pitch frame 208, the first handle 204, the rotation shaft 241, the rotation shaft 242, and the rotation shaft 243 are pitch-rotated together with the pitch frame 208. That is, when the pitch-manipulation portion 201 rotates around the rotation shaft 246, the actuation-manipulation portion 203 and the yaw-manipulation portion 202 are rotated together with the pitch-manipulation portion 201. In other words, when a user pitch-rotates the first handle 204 around the rotation shaft 246, the actuation-manipulation portion 203, the yaw-manipulation portion 202, and the pitch-manipulation portion 201 are moved together with the first handle 204.

The pulleys 219 and 220 and the pulleys 229 and 230 are coupled to the rotation shaft 246 so as to be rotatable around the rotation shaft 246 of the pitch frame 208.

Here, the pulley 219 and the pulley 220 may be configured to face each other so as to be independently rotatable. Accordingly, a wire being wound and a wire being released may be wound around respective separate pulleys so that the wires may perform motions without interference with each other. Similarly, the pulley 229 and the pulley 230 may also be configured to face each other so as to be independently rotatable. Accordingly, a wire being wound and a wire being released may be wound around respective separate pulleys so that the wires may perform motions without interference with each other.

Next, a motion of each of the wires 303 and 304, which are pitch wires, is described as follows.

A pulley 1131, which is an end tool pitch pulley, is fixedly coupled to the end tool hub 1180 in the end tool 1100, and the pulley 231 and the pulley 232(not shown), which are manipulation part pitch pulleys, are fixedly coupled to the pitch frame 208 in the manipulation part 200. In an embodiment, these pulleys are connected to each other by the wires 303 and 304, which are pitch wires, so that a pitch motion of the end tool 1100 may be performed more easily according to the pitch manipulation of the manipulation part 200. Here, the wire 303 is fixedly coupled to the pitch frame 208 via the pulley 231, and the wire 304 is fixedly coupled to the pitch frame 208 via the pulley 232(now shown). That is, the pitch frame 208 and the pulleys 231 and 232 are rotated together around the rotation shaft 246 by the pitch rotation of the manipulation part 200, and as a result, the wires 303 and 304 are also moved, and thus, a driving force of additional pitch rotation may be transmitted separately from the pitch motion of the end tool by the wire 301, the wire 302, the wire 305, and the wire 306, which are jaw wires.

A connection relationship of each of the first handle 204, the pitch-manipulation portion 201, the yaw-manipulation portion 202, and the actuation-manipulation portion 203 is summarized as follows. The rotation shaft 241, the rotation shaft 242, the rotation shaft 243, the rotation shaft 244, the rotation shaft 245, and the rotation shaft 246 may be provided on the first handle 204. In this case, since the rotation shafts 242 and 243 are directly provided on the first handle 204, the first handle 204 and the yaw-manipulation portion 202 may be directly connected to each other. In an embodiment, since the pitch-manipulation portion 201 is provided on one side of the yaw-manipulation portion 202 so as to be connected to the yaw-manipulation portion 202, the pitch-manipulation portion 201 is not directly connected to the first handle 204, and the pitch-manipulation portion 201 and the first handle 204 may be configured to be indirectly connected to each other via the yaw-manipulation portion 202. In an embodiment, since the actuation-manipulation portion 203 is provided on another side of the yaw-manipulation portion 202 so as to be connected to the yaw-manipulation portion 202, the actuation-manipulation portion 203 may be configured to be indirectly connected to the first handle 204 through the yaw-manipulation portion 202, rather than being directly connected to the first handle 204.

Continuing to refer to the drawings, in the surgical instrument for electrocautery 10 according to an embodiment of the present disclosure, the pitch-manipulation portion 201 and the end tool 1100 may be provided on the same or parallel axis (the X-axis). That is, the rotation shaft 246 of the pitch-manipulation portion 201 is provided at one end portion of the bent portion 402 of the connection part 400, and the end tool 1100 is provided at another end portion of the connection part 400.

In an embodiment, one or more relay pulleys 235 configured to change or guide paths of the wires may be disposed at intervals along the connection part 400, particularly in the bent portion 402. By positioning at least some of the wires to wound around the relay pulleys 235 to guide the paths of the wires, these wires may be placed along a bent shape of the bent portion 402.

Here, in the drawings, it is illustrated that the connection part 400 is provided to be curved with a predetermined curvature by having the bent portion 402, but the concept of the present disclosure is not limited thereto, and the connection part 400 may be provided linearly or to be bent one or more times as necessary, and even in this case, it may be said that the pitch-manipulation portion 201 and the end tool 1100 are provided on substantially the same axis or parallel axes. In an embodiment, although FIG. 2 illustrates that each of the pitch-manipulation portion 201 and the end tool 1100 is provided on an axis parallel to the X-axis, the concept of the present disclosure is not limited thereto, and the pitch-manipulation portion 201 and the end tool 1100 may be provided on different axes.

### (Actuation, yaw, and pitch motions)

Actuation, yaw, and pitch motions in the present embodiment will be described as follows.

First, the actuation motion is described as follows.

When a user inserts his or her finger into a finger ring provided on the actuation lever 261 and rotates the actuation lever 261 using his or her finger, the actuation pulley 262, which is fixedly coupled to the actuation lever 261, rotates around the rotation shaft 241.

In this case, the wires 301 and 305, each having one end portion fixedly coupled to and wound around the pulley 262, and the wires 302 and 306, each having one end portion fixedly coupled to and wound around the same pulley 262, move as the pulley 262 rotates. Here, although the wires 301, 302, 305, and 306 are all coupled to one actuation pulley 262, the movement of each wire differs depending on the direction in which each wire is wound around the pulley 262 as the pulley rotates. This will be described in detail later.

In an embodiment, a rotating force is transmitted to the end tool 1100 through the power transmission part 300, and two jaws 1103 of the end tool 1100 perform an actuation motion.

Here, as described above, the actuation motion refers to a motion in which the two jaws 1101 and 1102 are splayed or closed while being rotated in opposite directions. That is, when the actuation lever 261 of the actuation-manipulation portion 203 rotates in a direction close to the first handle 204, the first jaw 1101 rotates in the counterclockwise direction, and the second jaw 1102 rotates in the clockwise direction, thereby closing the end tool 1100. In an embodiment, when the actuation lever 261 of the actuation-manipulation portion 203 rotates in a direction away from the first handle 204, the first jaw 1121 rotates in the clockwise direction, and the second jaw 1122 rotates in the counterclockwise direction, thereby opening the end tool 1100.

Next, the yaw motion is described as follows.

When a user rotates the first handle 204 around the rotation shaft 243 while holding the first handle 204, the actuation-manipulation portion 203 and the yaw-manipulation portion 202 are yaw-rotated around the rotation shaft 243. That is, when the actuation pulley 262, to which the wires 301 and 305 are fixedly coupled, rotates around the rotation shaft 243, the wires 301 and 305 wound around the pulleys 213 and 214 are moved. At this time, one of the wires 301 and 305 is wound around the pulley 213 or the pulley 214, and another one of the wires 301 and 305 is unwound from the pulley 213 or the pulley 214. Similarly, since the wire 302 and the wire 306 are also fixedly coupled to the actuation pulley 262, when the actuation pulley 262 rotates around the rotation shaft 243, the wire 302 and the wire 306 wound around the pulley 223 and the pulley 224 will move. At this time, one of the wires 302 and 306 is wound around the pulley 223 or 224, and another one of the wires 302 and 306 is unwound from the pulley 223 or 224, and at this time, the wires 301 and 305 connected to the first jaw 1101 and the wires 302 and 306 connected to the second jaw 1102 are wound around the pulleys 213 and 214 and the pulleys 223 and 224 so that the first jaw 1101 and the second jaw 1102 rotate in the same direction during the yaw rotation. In an embodiment, as a rotating force is transmitted to the end tool 1100 through the power transmission part 300, a yaw motion in which two jaws 1103 of the end tool 1100 are rotated in the same direction is performed.

At this time, since the yaw frame 207 connects the first handle 204, the rotation shaft 241, the rotation shaft 242, and the rotation shaft 243 to each other, the first handle 204, the yaw-manipulation portion 202, and the actuation-manipulation portion 203 are rotated together around the rotation shaft 243.

Next, the pitch motion is described as follows.

When a user rotates the first handle 204 around the rotation shaft 246 while holding the first handle 204, the actuation-manipulation portion 203, the yaw-manipulation portion 202, and the pitch-manipulation portion 201 are pitch-rotated around the rotation shaft 246. That is, when the actuation pulley 262, to which the wires 301 and 305 are fixedly coupled, rotates around the rotation shaft 246, the wires 301 and 305 wound around the pulleys 219 and 220 are moved. Similarly, when the actuation pulley 262, to which the wire 302 and the wire 306 are fixedly coupled, rotates around the rotation shaft 246, the wires 302 and 306 wound around the pulleys 229 and 230 are moved. At this time, as will be described with reference to FIG. 50 or the like, in order to allow the first jaw 1101 and the second jaw 1102 to pitch-rotate, the wires 301 and 305, which are first jaw wires, are moved in the same direction and respectively wound around the pulley 219 and the pulley 220, which are manipulation part pitch main pulleys, and the wires 302 and 306, which are second jaw wires, are moved in the same direction and respectively wound around the pulley 229 and the pulley 230, which are manipulation part pitch main pulleys. In an embodiment, a rotating force is transmitted to the end tool 1100 through the power transmission part 300, and two jaws 1103 of the end tool 1100 perform the pitch motion.

At this time, since the pitch frame 208 is connected to the yaw frame 207, and the yaw frame 207 connects the first handle 204, the rotation shaft 241, the rotation shaft 242, and the rotation shaft 243 to each other, when the pitch frame 208 rotates around the rotation shaft 246, the yaw frame 207, the first handle 204, the rotation shaft 241, the rotation shaft 242, and the rotation shaft 243 connected to the pitch frame 208 are rotated together with the pitch frame 208. That is, when the pitch-manipulation portion 201 rotates around the rotation shaft 246, the actuation-manipulation portion 203 and the yaw-manipulation portion 202 are rotated together with the pitch-manipulation portion 201.

In summary, in the surgical instrument for electrocautery 10 according to an embodiment of the present disclosure, the pulleys are provided on respective joint points (an actuation joint, a yaw joint, and a pitch joint), the wires (the first jaw wire or the second jaw wire) are wound around the pulleys, and the rotational manipulations (actuation rotation, yaw rotation, and pitch rotation) of the manipulation part cause the movement of each wire, which in turn induces the desired motion of the end tool 1100. Furthermore, the auxiliary pulley may be provided at one side of each of the pulleys, and the wire may not be wound several times around one pulley due to the auxiliary pulley.

FIG. 50 is a view schematically illustrating only a configuration of the pulleys and wires constituting joints of the surgical instrument for electrocautery 10 according to an embodiment of the present disclosure shown in FIG. 2. In FIG. 50, the relay pulleys that are not related to the operation of joints and are used to reroute the wire are omitted.

Referring to FIG. 50, the manipulation part 200 may include the pulley 211, the pulley 212, the pulley 213, the pulley 214, the pulley 215, the pulley 217, the pulley 218, the pulley 219, and the pulley 220, which are associated with a rotational motion of the first jaw 1101.

In an embodiment, the manipulation part 200 may include the pulley 221, the pulley 222, the pulley 223, the pulley 224, the pulley 225, the pulley 227, the pulley 228, the pulley 229, and the pulley 230, which are associated with a rotational motion of the second jaw 1122. In an embodiment, the manipulation part 200 may include the pulley 262 associated with a rotational motion of the first jaw and the second jaw. (the arrangement and structure of each of the pulleys of the manipulation part 200 are the same in principle as the arrangement and structure of each of the pulleys of the end tool 1100, and thus specific designations of some reference numerals are omitted in the drawings).

The pulleys 211 and 212 and the pulleys 221 and 222 may be configured to be rotatable independently of each other around the same shaft, that is the rotation shaft 242. Here, the pulley 211 and the pulley 212 may be provided as two pulleys that are configured to face each other so as to be independently rotatable. Similarly, the pulleys 221 and 222 may be provided as two pulleys configured to face each other and provided to be independently rotatable, and in this case, the two pulleys may be provided to have different diameters.

The pulleys 213 and 214 and the pulleys 223 and 224 may be configured to be rotatable independently of each other around the same shaft, that is the rotation shaft 243. In this case, the pulleys 213 and 214 and the pulleys 223 and 224 may each be provided as two pulleys configured to face each other and configured to be independently rotatable.

The pulleys 215 and 225 may be configured to be rotatable independently of each other around the same shaft, which is the rotation shaft 244.

The pulleys 217 and 218 and the pulleys 227 and 228 may be configured to be rotatable independently of each other around the same shaft, that is the rotation shaft 245. In this case, the pulleys 217 and 218 may be provided to have different diameters. In an embodiment, the pulleys 227 and 228 may be configured to have different diameters.

The pulleys 219 and 220 and the pulleys 229 and 230 may be configured to be rotatable independently of each other around the same shaft, that is the rotation shaft 246.

The wire 301 is wound around the pulley 262 after sequentially passing through the pulley 219, the pulley 217, the pulley 215, the pulley 213, and the pulley 211 of the manipulation part 200, and then is coupled to the pulley 262 by a coupling member 264a. In an embodiment, the wire 305 sequentially passes through the pulley 220, the pulley 218, the pulley 214, and the pulley 212 of the manipulation part 200 and is coupled to the pulley 262 by a coupling member 264c. Here, the coupling members 264a and 264c may be directly coupled to the pulley 262, or may be coupled to a separate coupling member 264, which is in turn coupled to the pulley 262, such that the coupling members 264a and 264c are ultimately coupled to the pulley 262. Thus, when the pulley 262 rotates, the wires 301 and 305 are wound around or unwound from the pulley 262, and accordingly, the first jaw 1101 rotates.

The wire 306 is wound around the pulley 262 after sequentially passing through the pulley 229, the pulley 227, the pulley 225, the pulley 223, and the pulley 221 of the manipulation part 200, and then is coupled to the pulley 262 by a fastening member 264d. In an embodiment, the wire 302 sequentially passes through the pulley 230, the pulley 228, the pulley 224, and the pulley 222 of the manipulation part 200 and is coupled to the pulley 262 by a coupling member 264b. Here, the coupling members 264b and 264d may be directly coupled to the pulley 262, or may be coupled to the separate coupling member 264, which is in turn coupled to the pulley 262, such that the coupling members 264b and 264d are ultimately coupled to the pulley 262. Thus, when the pulley 262 rotates, the wire 302 and the wire 306 are wound around or unwound from the pulley 262, and accordingly, the second jaw 1102 rotates.

### (Conceptual diagram of pulleys and wires)

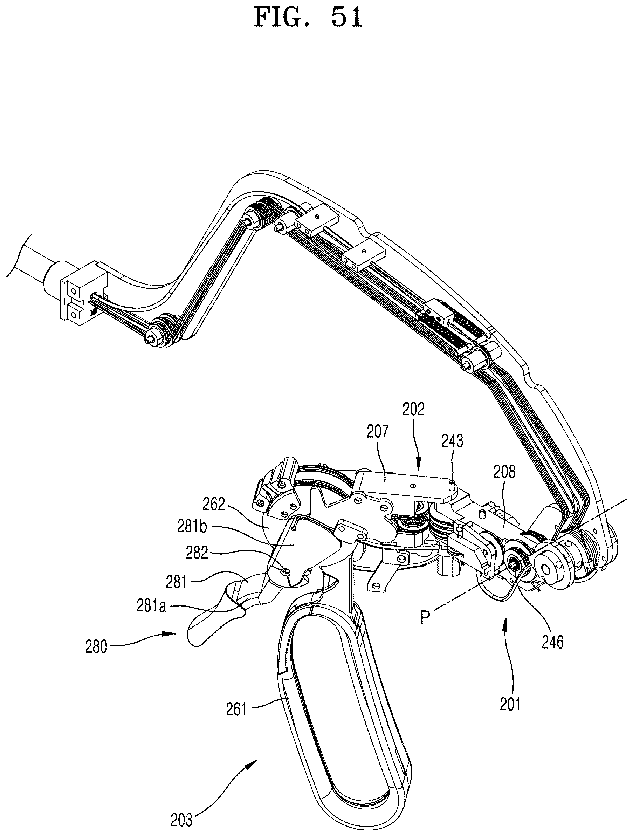

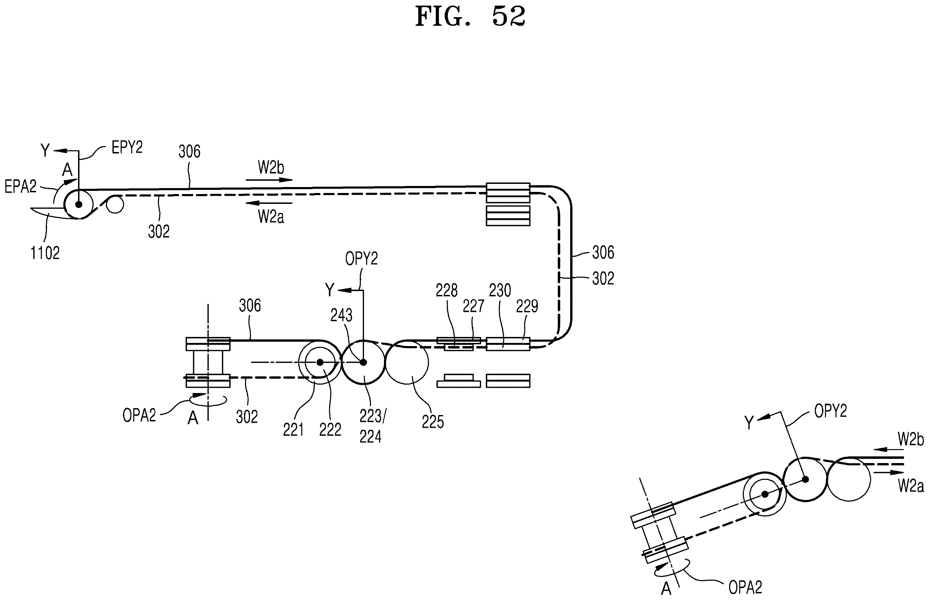

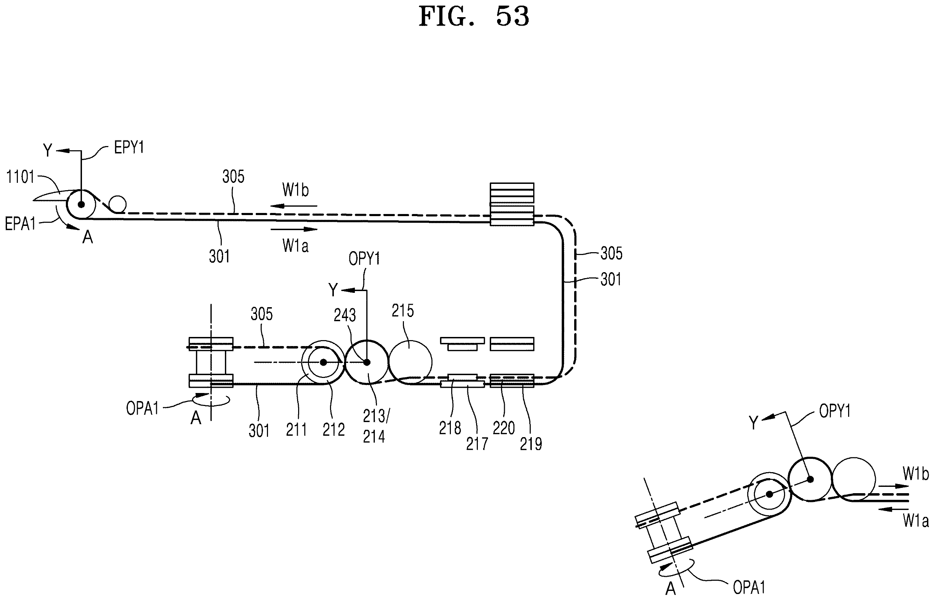

FIGS. 52 and 53 are diagrams illustrating a configuration of pulleys and wires, which are associated with an actuation motion and a yaw motion of the surgical instrument for electrocautery 10 according to an embodiment of the present disclosure shown in FIG. 2, separately for each of the first jaw and the second jaw. FIG. 52 is a diagram illustrating only pulleys and wires related to the second jaw, and FIG. 53 is a diagram illustrating only pulleys and wires related to the first jaw. In an embodiment, FIG. 51 is a perspective view illustrating a yaw motion of the surgical instrument of FIG. 2.

First, a wire operation in an actuation motion will be described.

Referring to FIG. 53, when the actuation lever 261 rotates around the rotation shaft 241 in the direction of an arrow OPA1, the pulley 262 connected to the actuation lever 261 is rotated, and the wires 301 and 305 wound around the pulley 262 are moved in directions W1a and W1b, respectively, and as a result, the first jaw 1101 of the end tool 1100 is rotated in the direction of an arrow EPA1.

Referring to FIG. 52, when the actuation lever 261 rotates around the rotation shaft 241 in the direction of an arrow OPA2, the pulley 262 connected to the actuation lever 261 is rotated, and thus opposite strands of the wires 302 and 306 wound around the pulley 262 are moved in directions W2a and W2b, respectively, and as a result, the second jaw 1102 of the end tool 1100 is rotated in the direction of an arrow EPA2. Thus, when the user operates the actuation lever 261 in a direction closer to the first handle 204, a motion in which the first jaw 1101 and the second jaw 1102 of the end tool move closer to each other is performed.

Next, a wire operation in a yaw motion will be described.

First, since the rotation shaft 243 is connected to the rotation shafts 241 and 242 by the yaw frame (see 207 of FIG. 48), the rotation shaft 243 and the rotation shafts 241 and 242 are integrally rotated together.

Referring to FIG. 53, when the first handle 204 rotates around the rotation shaft 243 in the direction of an arrow OPY1, the pulley 262, the pulley 211, the pulley 212, the pulley 213, and the pulley 214 and the wires 301 and 305 wound therearound are rotated as a whole around the rotation shaft 243, and as a result, the wires 301 and 305 wound around the pulleys 213 and 214 are moved in the directions W1a and W1b, respectively, which in turn causes the first jaw 1101 of the end tool 1100 to rotate in the direction of an arrow EPY1.

Referring to FIG. 52, when the first handle 204 rotates around the rotation shaft 243 in the direction of an arrow OPY2, the pulley 262, the pulley 221, the pulley 222, the pulley 223, and the pulley 224 and the wires 302 and 306 wound therearound are rotated as a whole around the rotation shaft 243, and as a result, the wires 302 and 306 wound around the pulleys 223 and 224 are moved in directions opposite to the directions W2a and W2b, respectively, which in turn causes the first jaw 1101 of the end tool 1100 to rotate in the direction of an arrow EPY2.

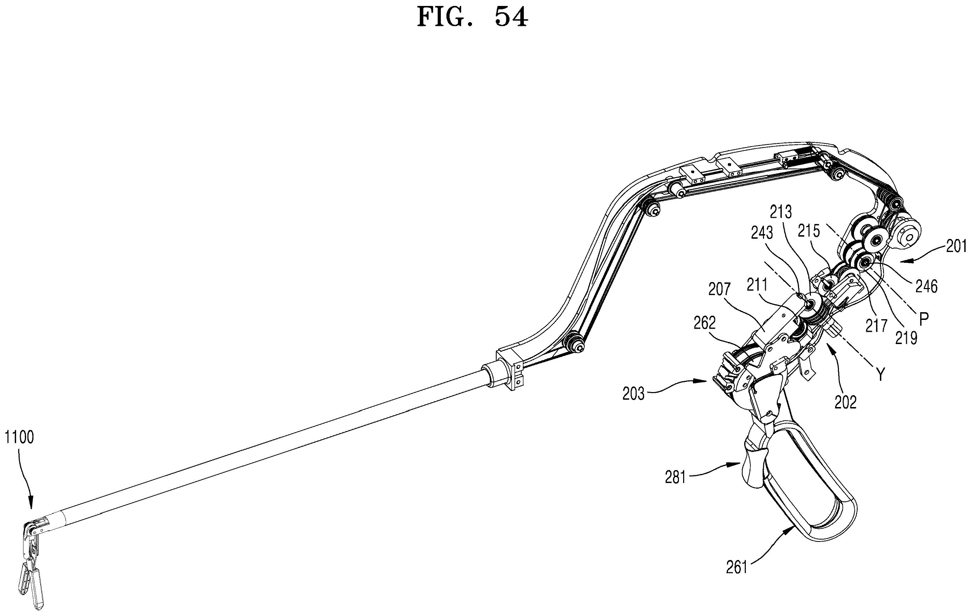

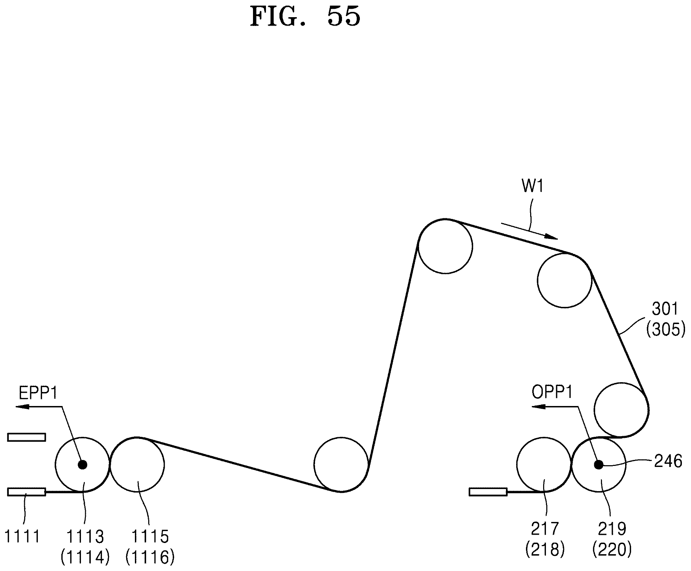

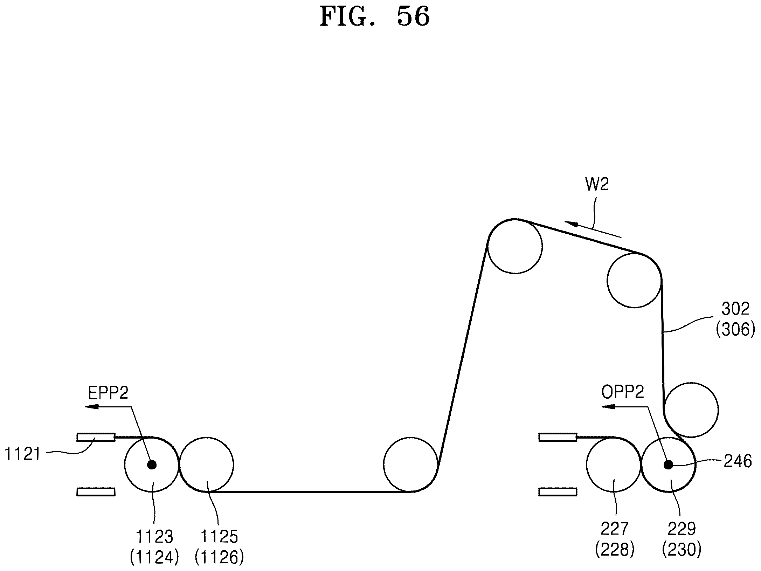

FIGS. 55 and 56 are diagrams illustrating a configuration of pulleys and wires, which are associated with a pitch motion of the surgical instrument for electrocautery 10 according to an embodiment of the present disclosure shown in FIG. 2, separately for each of the first jaw and the second jaw. FIG. 56 is a diagram illustrating only pulleys and wires related to the second jaw, and FIG. 55 is a diagram illustrating only pulleys and wires related to the first jaw. As shown in FIG. 2 and elsewhere herein, there are two pulleys related to the pitch motion, and opposite strands of each wire are wound in the same path, which is illustrated with one line in FIG. 55. In an embodiment, FIG. 54 is a perspective view illustrating a pitch motion of the surgical instrument of FIG. 2.

Referring to FIG. 55, when the first handle 204 rotates around the rotation shaft 246 in the direction of an arrow OPP1, the pulley 262, the pulley 217, the pulley 219, and the like, and the wire 301 and the like wound therearound are rotated as a whole around the rotation shaft 246. At this time, since the wires 301 and 305, which are first jaw wires, are wound around upper portions of the pulley 219 and the pulley 220, the wires 301 and 305 are moved in the direction of an arrow W1. As a result, the first jaw 1101 of the end tool 1100 rotates in the direction of an arrow EPP1.

Referring to FIG. 56, when the first handle 204 rotates around the rotation shaft 246 in the direction of an arrow OPP2, the pulley 262, the pulley 227, the pulley 229, and the like, and the wire 302 and the like wound therearound are rotated as a whole around the rotation shaft 246. At this time, since the wires 302 and 306, which are second jaw wires, are wound around lower portions of the pulley 229 and the pulley 230, the wires 302 and 306 are moved in the direction of an arrow W2. As a result, the second jaw 1102 of the end tool 1100 rotates in the direction of an arrow EPP2.

Thus, the actuation, yaw, and pitch manipulations are manipulatable independent of each other.

### (Actuation lever operation)

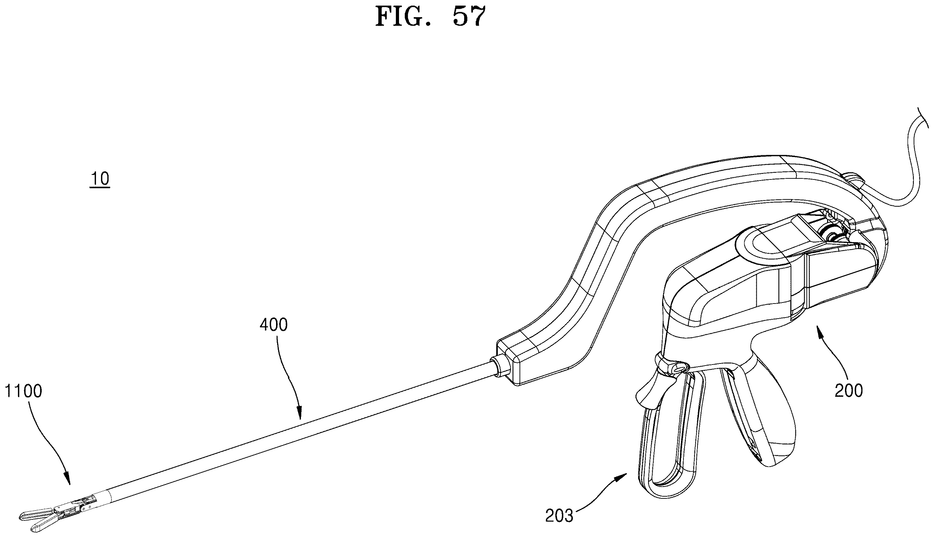

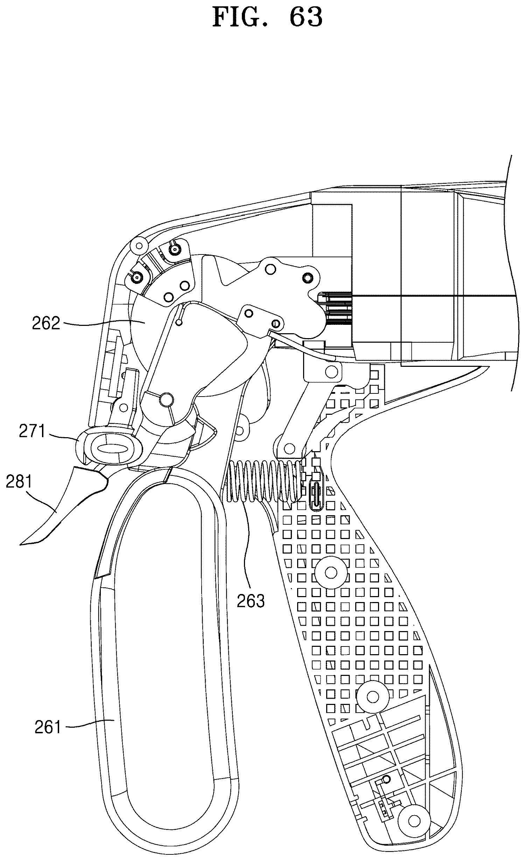

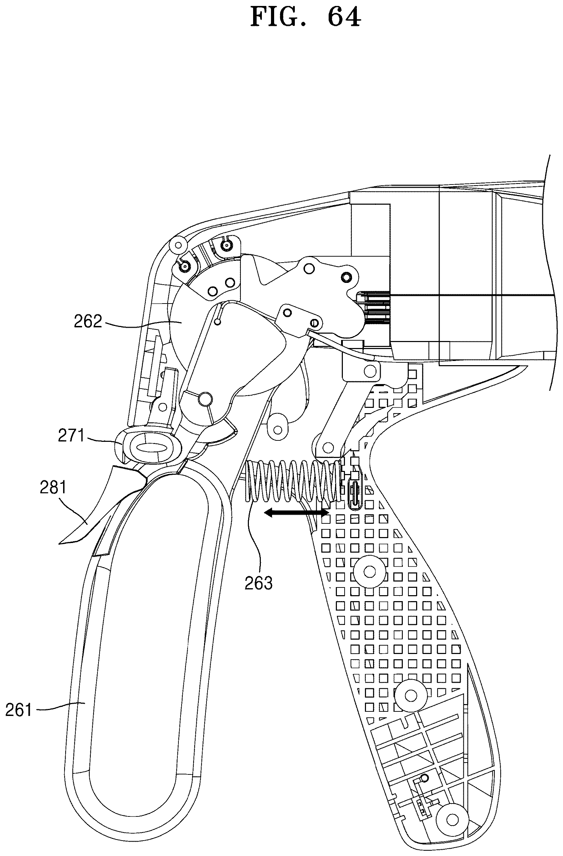

FIGS. 57 to 60 are perspective views illustrating the operation of the actuation lever of the surgical instrument for electrocautery shown in FIG. 2, and FIGS. 61 and 62 are views illustrating the movement of the wires during the operation of the actuation lever of the surgical instrument for electrocautery shown in FIG. 2. FIGS. 63 and 64 are partial cross-sectional views illustrating the actuation restoring elastic member of the surgical instrument for electrocautery shown in FIG. 2.

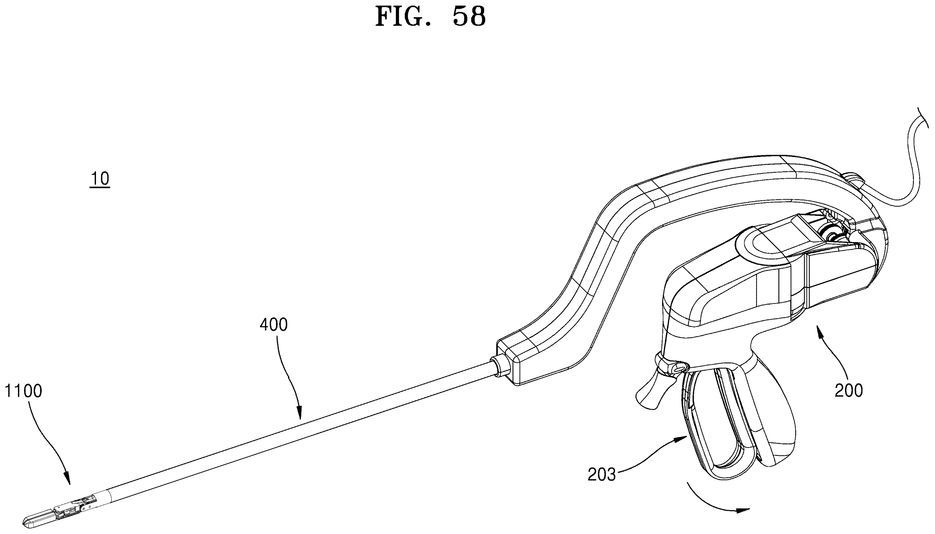

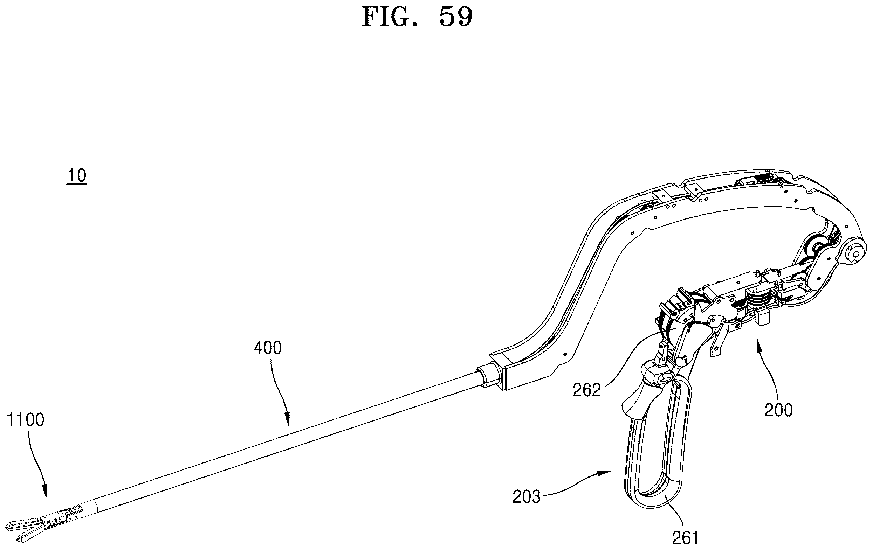

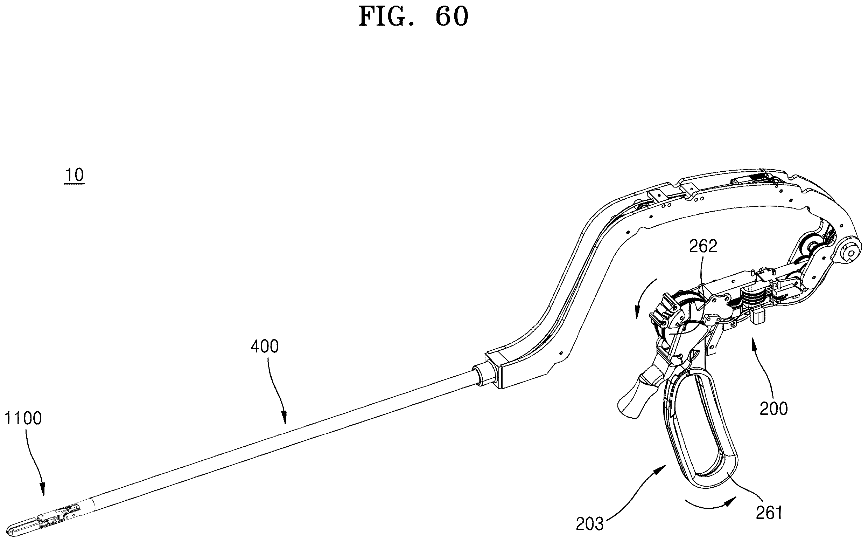

FIG. 57 illustrates a state in which the actuation lever of the surgical instrument for electrocautery shown in FIG. 2 is not operated, and FIG. 58 illustrates a state in which the actuation lever of the surgical instrument for electrocautery shown in FIG. 2 is operated. In an embodiment, FIG. 59 illustrates a state in which a case of the surgical instrument for electrocautery shown in FIG. 57 is removed, with the actuation lever in a non-operating state, and FIG. 60 illustrates a state in which the case of the surgical instrument for electrocautery shown in FIG. 58 is removed, with the actuation lever in an operating state.

Referring to FIGS. 57 to 60, in the surgical instrument for electrocautery 10 according to an embodiment of the present disclosure, the actuation pulley 262 may be rotated by gripping the first handle 204 with the palm and pulling the actuation lever 261 toward the first handle 204 while inserting a finger into the actuation lever 261. That is, an actuation motion may be performed by operating one lever.

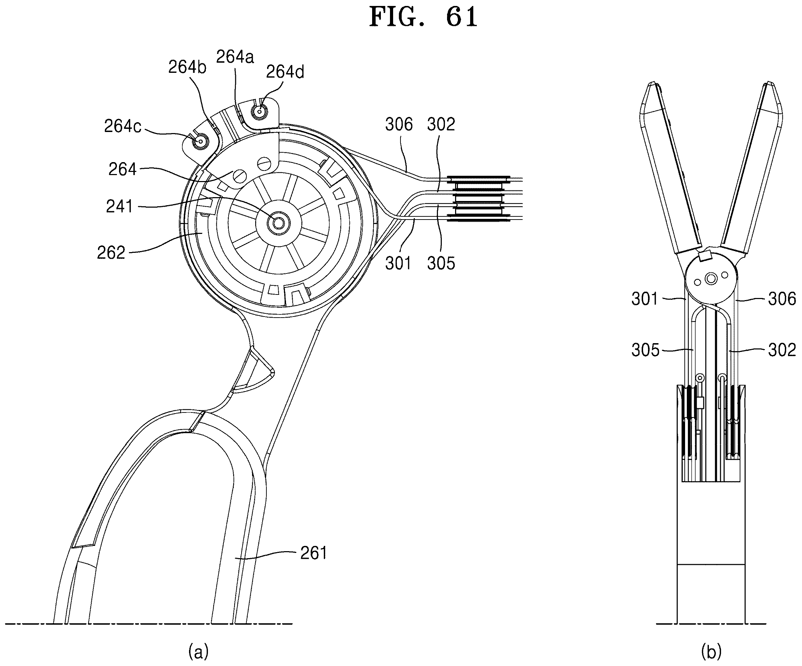

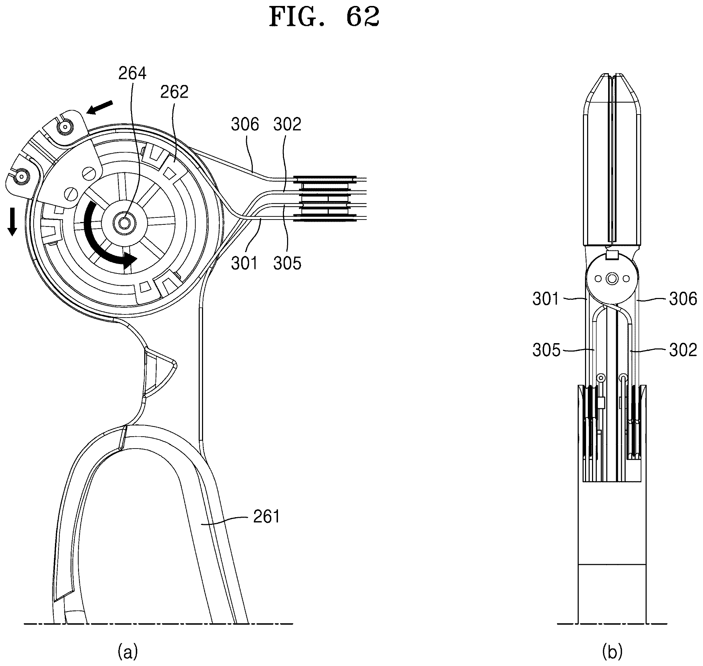

FIG. 61A is a side view illustrating the wires at the actuation pulley 262 of the surgical instrument for electrocautery shown in FIG. 2, and FIG. 61B is a plan view illustrating the wires at the end tool 1100. FIG. 62A is a side view illustrating the movement of the wires at the actuation pulley 262 during the actuation lever operation, and FIG. 62B is a plan view illustrating the movement of the wires at the end tool 1100.

Referring to FIGS. 50, 61, and 62, in the surgical instrument 10 according to an embodiment of the present disclosure, the wires 301 and 305, which are first jaw wires, and the wires 302 and 306, which are second jaw wires, are all coupled to the single actuation pulley 262, and thus, by appropriately positioning each wire, the movement of each wire may be varied solely by the rotation of the single pulley. That is, by rotating the actuation pulley 262 in any one direction through the actuation lever 261, the first jaw 1101 and the second jaw 1102 may rotate in opposite directions. In other words, depending on the rotation of the actuation pulley 262, the first jaw 1101 and the second jaw 1102 may perform opening and closing motions, allowing them to open or close.

In an embodiment, the wires 301 and 305, which are first jaw wires, may be wound in opposite directions around the actuation pulley 262. For example, as shown in FIG. 50 or the like, the wire 301 may be wound around the actuation pulley 262 in the counterclockwise direction, and the wire 305 may be wound around the actuation pulley 262 in the clockwise direction. Similarly, the wires 302 and 306, which are second jaw wires, may be wound in opposite directions around the actuation pulley 262. For example, as shown in FIG. 50, the wire 302 may be wound around the actuation pulley 262 in the clockwise direction, and the wire 306 may be wound around the actuation pulley 262 in the counterclockwise direction.

At this time, when the actuation pulley 262 rotates in the counterclockwise direction, the wire 301 is wound around the actuation pulley 262 and the wire 305 is unwound from the actuation pulley 262. Accordingly, at the first jaw pulley 1111 of the end tool 1100, the wire 301 is unwound, and the wire 305 is wound, causing the first jaw pulley 1111 of the end tool to rotate in the counterclockwise direction.

In an embodiment, when the actuation pulley 262 rotates in the counterclockwise direction, the wire 306 is wound around the actuation pulley 262 and the wire 302 is unwound from the actuation pulley 262. Accordingly, at the second jaw pulley 1121 of the end tool 1100, the wire 306 is unwound, and the wire 302 is wound, causing the second jaw pulley 1121 of the end tool to rotate in the clockwise direction.

Similarly, when the actuation pulley 262 rotates in the clockwise direction, the first jaw pulley 1111 rotates in the clockwise direction and the second jaw pulley 1121 rotates in the counterclockwise direction.

Accordingly, when the actuation pulley 262 rotates, the first jaw pulley 1111 and the second jaw pulley 1121 are rotated in opposite directions, causing the first and second jaws of the end tool 1100 to open or close.

In an embodiment, referring to FIGS. 63 and 64, the surgical instrument for electrocautery 10 according to an embodiment of the present disclosure may include the actuation restoring elastic member 263 between the actuation lever 261 and the first handle 204. For example, a spring-shaped member may be positioned in a separation space between the actuation lever 261 and the first handle 204. When the actuation lever 261 is pulled toward the first handle 204 to perform an actuation motion, the actuation lever 261 moves closer to the first handle 204, thereby compressing the actuation restoring elastic member 263. Accordingly, a restoring force of the actuation restoring elastic member 263 acts on the actuation lever 261, and as a result, a force is applied to the actuation lever 261 in a direction away from the first handle 204. Thus, when the user releases the actuation lever 261, the actuation lever 261 automatically returns to its original position. Thus, as described above, as the actuation pulley 262 rotates in the clockwise direction, the first jaw wire and the second jaw wire are moved, and the first jaw 1101 and the second jaw 1102 are opened.

### (Sealing-manipulation portion)

Hereinafter, the sealing-manipulation portion 270 of the surgical instrument for electrocautery 10 according to an embodiment of the present disclosure will be described.

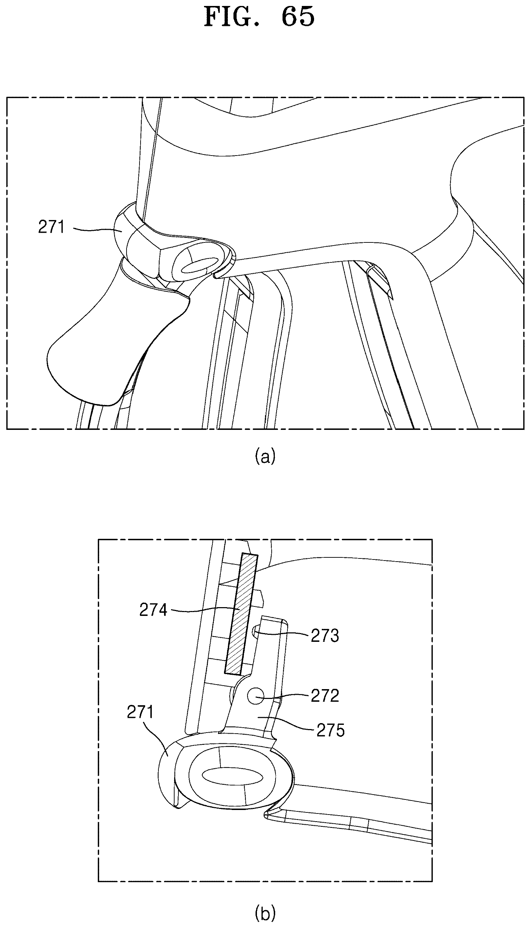

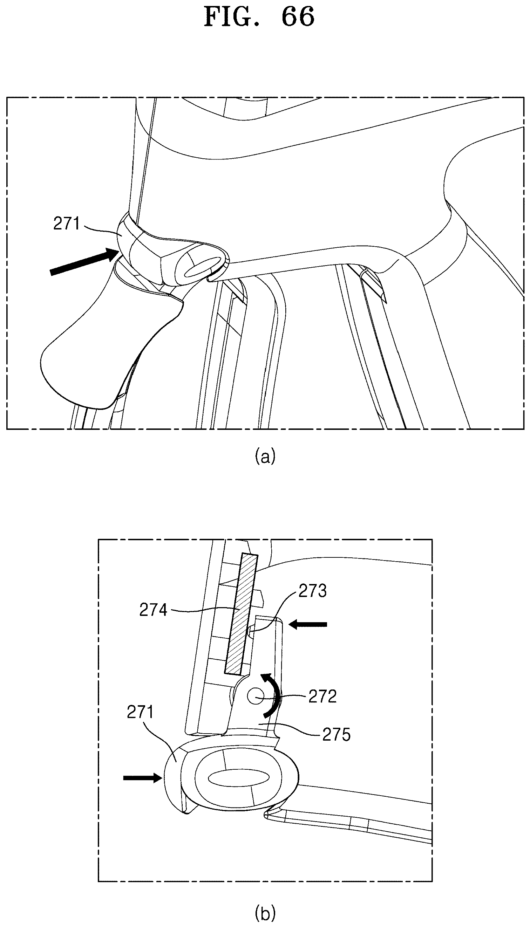

FIGS. 65 and 66 are perspective views illustrating the operation of a sealing button of the surgical instrument for electrocautery shown in FIG. 2. FIG. 65A is a perspective view illustrating the sealing-manipulation portion in a state in which the sealing button is not operated, and FIG. 65B is a partial cross-sectional view illustrating the inside of the sealing-manipulation portion in the state in which the sealing button is not operated. FIG. 66A is a perspective view illustrating the sealing-manipulation portion in a state in which the sealing button is operated, and FIG. 66B is a partial cross-sectional view illustrating the inside of the sealing-manipulation portion in the state in which the sealing button is operated.

Referring to FIGS. 65 and 66, the sealing-manipulation portion 270 may include a body portion 275, a sealing button 271, a sealing rotation shaft 272, and contact portions 273 and 274.

The sealing-manipulation portion 270 may be provided in a region adjacent to the actuation lever 261 of the actuation-manipulation portion 203. In an embodiment, the sealing-manipulation portion 270 may be provided on one side surface of a cutting lever 281 of the cutting-manipulation portion 280, which will be described later. In an embodiment, the user may operate the actuation lever 261 or the cutting lever 281 while holding the first handle 204, and the sealing button 271 may be provided in a position that facilitates its operation. For example, as shown in the drawings, the sealing button 271 may be positioned on the distal end side of the manipulation part 200 and may be provided so that one region of the sealing button 271 is inserted into the sealing-manipulation portion 270 when pressed.

In an embodiment, the body portion 275 may be provided in the shape of an elongated bar. In an embodiment, the sealing button 271 may be provided at one end portion of the body portion 275, and the contact portion 273 may be provided at another end portion of the body portion 275. In an embodiment, the sealing rotation shaft 272 may be coupled to a central portion of the body portion 275. Accordingly, the body portion 275 is rotatable around the sealing rotation shaft 272. In an embodiment, in the sealing-manipulation portion 270, the contact portion 274 may be provided on a surface facing the contact portion 273.

As described above, the contact portion 273 may be provided at one end portion of the body portion 275 with respect to the sealing rotation shaft 272, and the sealing button 271 protruding outward from the manipulation part 200 may be provided at another end portion of the contact portion 273.

Accordingly, when the sealing button 271 is pressed, the body portion 275 may rotate around the sealing rotation shaft 272, causing the contact portion 273 at the end portion of the body portion 275 to move and make contact with the contact portion 274.

As described above, the operation of the sealing button 271 may generate pressure or an electrical signal at the contact portion 274, thereby supplying electrical energy to the first electrode 1151 and the second electrode 1152 to perform cauterization.

### (Cutting-manipulation portion, cutting-relay member, and cutting-relay assembly)

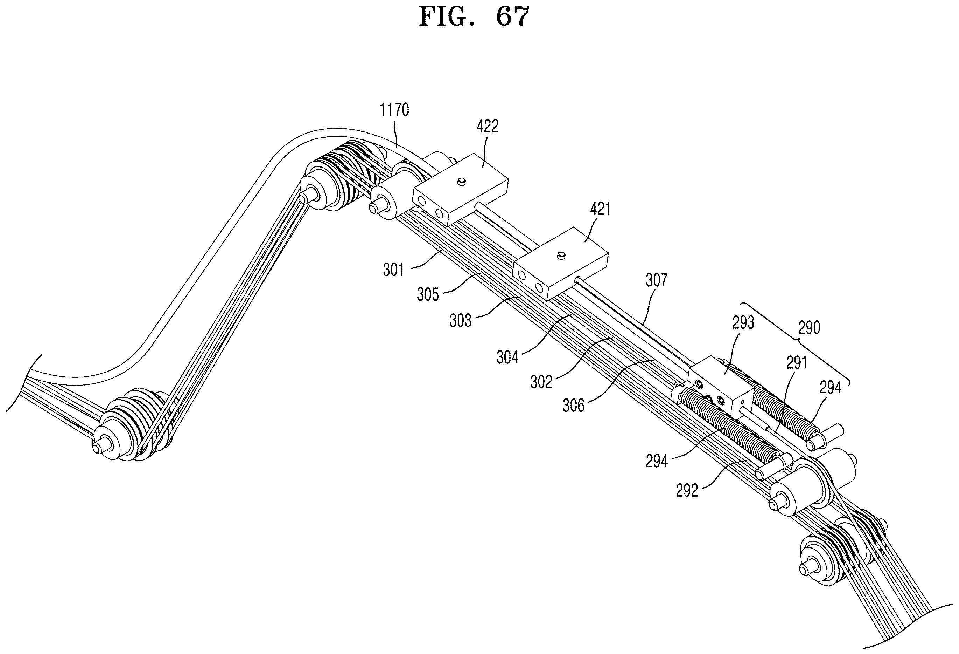

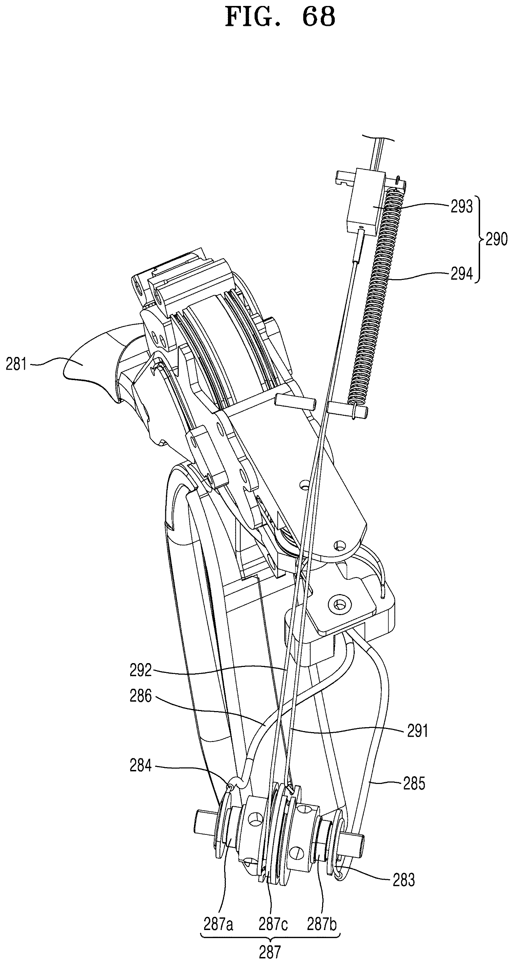

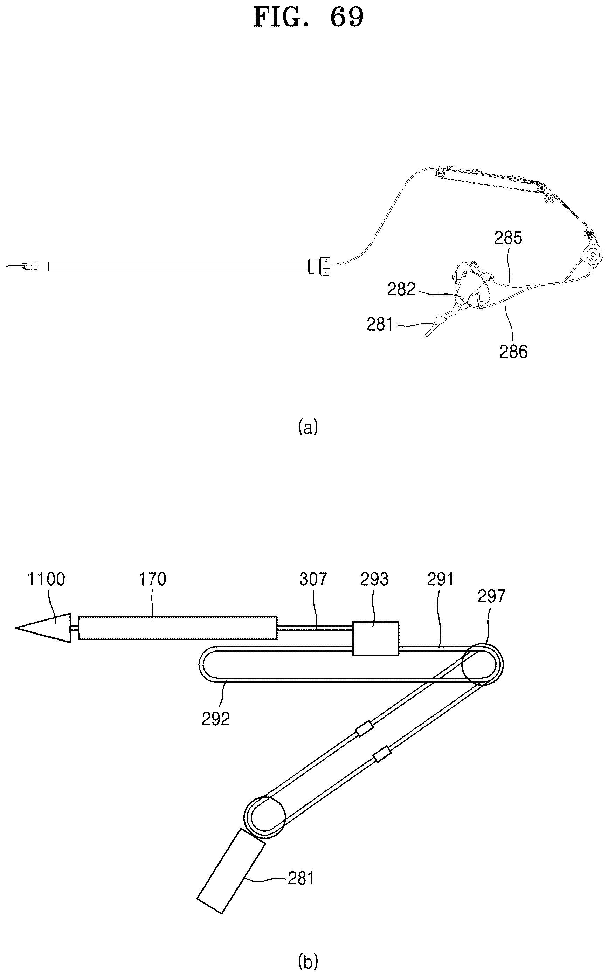

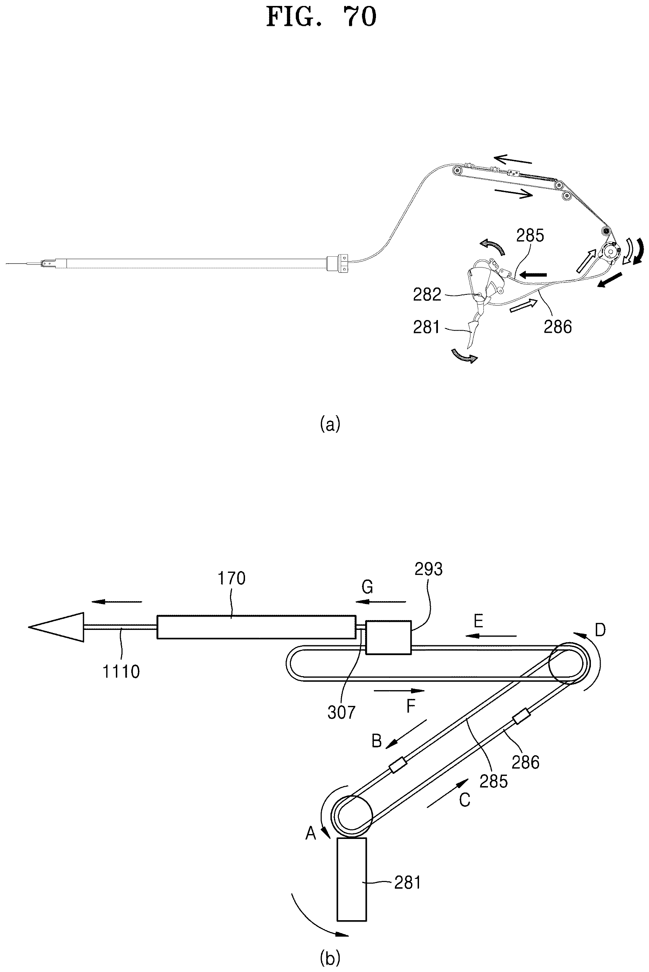

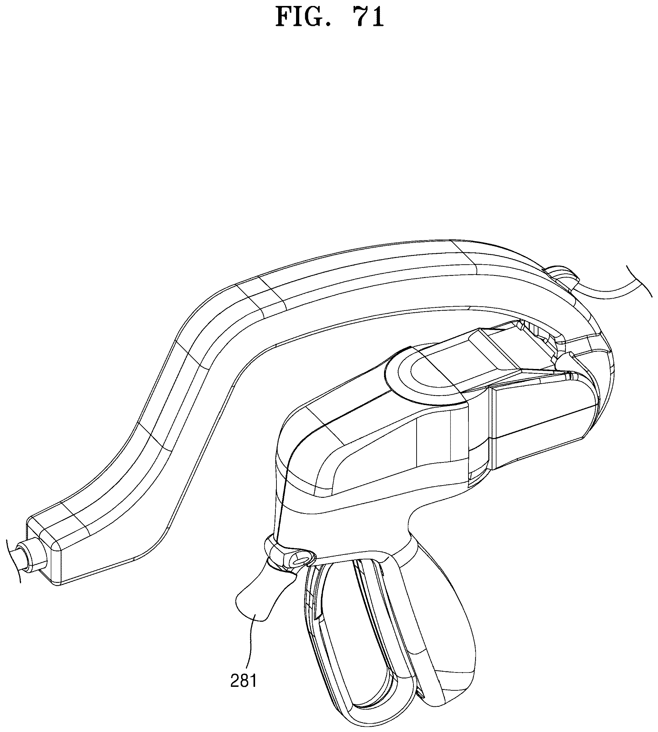

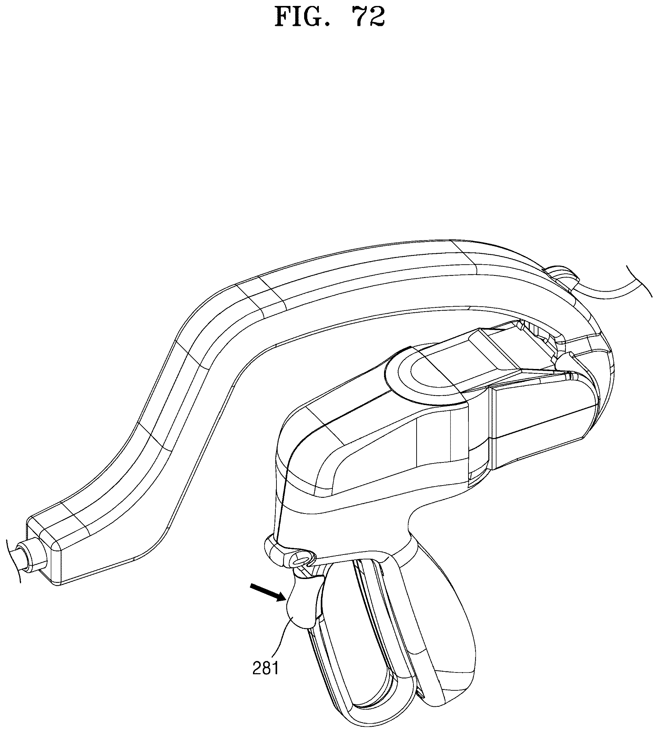

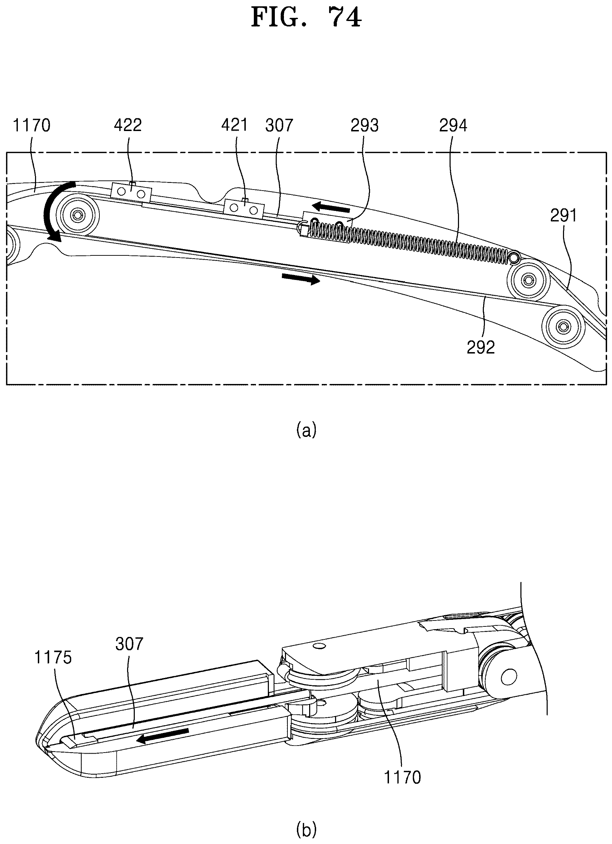

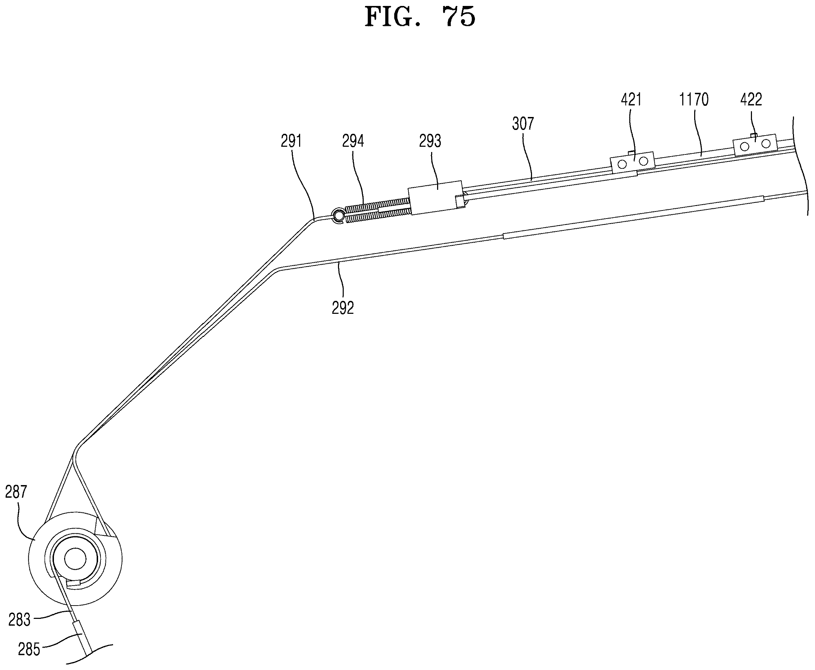

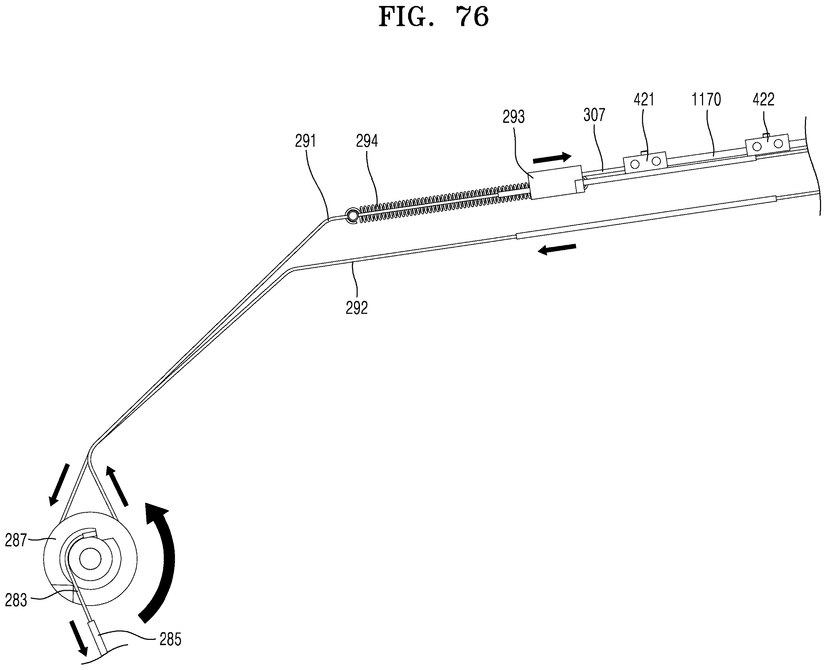

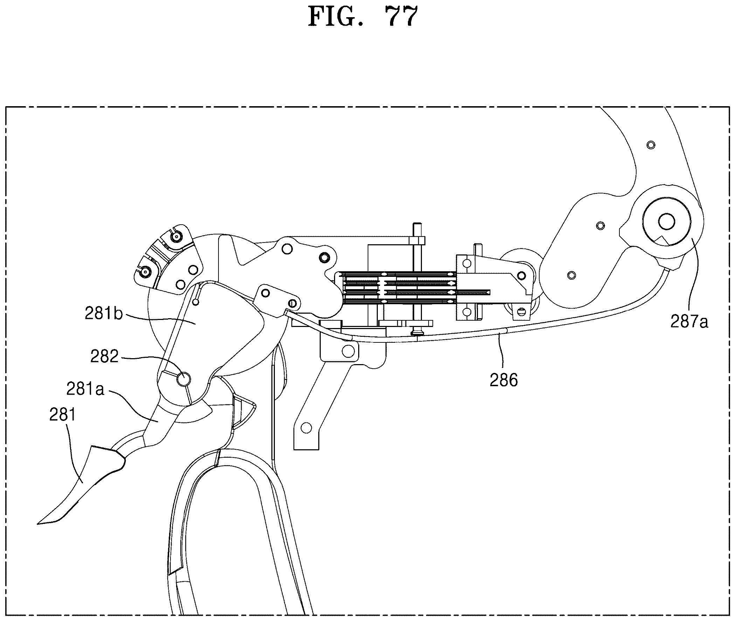

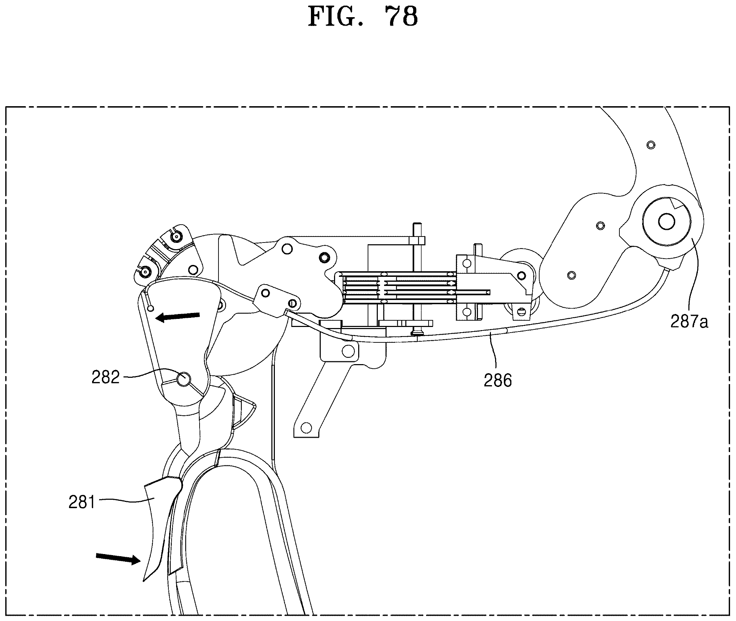

FIG. 67 is a perspective view illustrating a cutting-relay assembly of the surgical instrument for electrocautery shown in FIG. 2, and FIG. 68 is a perspective view illustrating the cutting-manipulation portion and the cutting-relay assembly of the surgical instrument for electrocautery shown in FIG. 2. FIGS. 69 and 70 are views for describing the cutting principle of the surgical instrument for electrocautery shown in FIG. 2. FIGS. 71 and 72 are perspective views illustrating a cutting lever operation of the surgical instrument for electrocautery shown in FIG. 2, and FIGS. 73 and 74 are views illustrating the movement of the cutting-relay assembly and the blade of the end tool during the cutting lever operation of the surgical instrument for electrocautery shown in FIG. 2. FIGS. 75 and 76 are views schematically illustrating only components of the cutting-relay member and the cutting-relay assembly during the cutting lever operation of the surgical instrument for electrocautery shown in FIG. 2. FIGS. 77 and 78 are views illustrating the cutting-manipulation portion and the wires during the cutting lever operation of the surgical instrument for electrocautery shown in FIG. 2.

Referring to FIGS. 67 to 78, the surgical instrument for electrocautery 10 according to an embodiment of the present disclosure may include the cutting-manipulation portion 280, a cutting-relay member 287, and a cutting-relay assembly 290.

Here, the cutting-relay assembly 290 may have a structure configured to convert a rotational motion of the cutting-relay member 287, which will be described later, into a linear motion.

The cutting-relay assembly 290 may be provided in the bent portion 402 of the connection part 400. In an embodiment, the cutting-relay assembly 290 may be provided in the bent portion 402 in which a plurality of wires, a plurality of electrical lines, and the relay pulleys 235 are accommodated.

The cutting-relay assembly 290 according to an embodiment of the present disclosure may include a first cutting-relay wire 291, a second cutting-relay wire 292, a cutting-relay block 293, and a cutting restoring elastic member 294.

Here, the first cutting-relay wire 291 and the second cutting-relay wire 292 may be connected to the cutting-relay member 287 and extend toward a distal end side within the bent portion 402. In an embodiment, each of the first cutting-relay wire 291 and the second cutting-relay wire 292 may have one end portion fixed to the cutting-relay member 287, and another end portion extending from the cutting-relay member 287, passing through the relay pulleys 235, wound around the pulleys in the bent portion 402, and coupled to the cutting-relay block 293. In this case, at least one region of each cutting-relay wire may be wound around the pulleys, such that the cutting-relay wires form a loop-shaped path overall, as shown in FIG. 69.

In an embodiment, one end portion of the first cutting-relay wire 291 may be connected to the cutting-relay member 287, and another end portion thereof may be connected to one side surface of the cutting-relay block 293. One end portion of the second cutting-relay wire 292 may be connected to the cutting-relay member 287, and another end portion thereof may be connected to a side surface of the cutting-relay block 293, which is opposite to the one side surface. In other words, the second cutting-relay wire 292 may be connected to a distal end-side surface of the cutting-relay block 293, and the first cutting-relay wire 291 may be connected to a proximal end-side surface of the cutting-relay block 293.

In another example, the cutting-relay wire may be a single wire, opposite end portions of which are connected to the cutting-relay member 287, and the cutting-relay block 293 may be coupled to a certain portion of the cutting-relay wire between the end portions.

In an embodiment, movement paths of the first cutting-relay wire 291 and the second cutting-relay wire 292, which are connected to the cutting-relay block 293, may include a linear section in which the wires move in a straight-line direction in at least one section. For example, a wire extending between one pulley and another pulley may form a linear section under tension.

When the wire having such a linear section in its movement path moves, the cutting-relay block 293 connected to the wire may also move in the straight-line direction along the movement path of the wire. For example, the movement path provided by the first cutting-relay wire 291 and the second cutting-relay wire 292 may extend in a substantially straight-line direction from a proximal end side to a distal end side of the manipulation part 200, or in the opposite direction. In this case, the cutting-relay block 293, which is coupled to the cutting-relay wires 291 and 292, may move in the straight-line direction toward the distal end side or the proximal end side of the manipulation part 200.

In an embodiment, one end portion of the blade wire 307 may be coupled to the distal end-side surface of the cutting-relay block 293. That is, one end portion of the blade wire 307 may be coupled to the distal end-side surface of the cutting-relay block 293 together with the second cutting-relay wire 292, and may be positioned in parallel with the linear movement section defined by the second cutting-relay wire 292 and the first cutting-relay wire 291. Accordingly, when the cutting-relay block 293 linearly moves toward the distal end side or the proximal end side of the manipulation part in response to the movement of the cutting-relay wires, the blade wire 307 coupled to the cutting-relay block 293 may also linearly move toward the distal end side or the proximal end side of the manipulation part.

In an embodiment, at least a portion of the blade wire 307 may be accommodated within the guide tube 1170. Accordingly, the movement path of the blade wire 307 may be guided by the guide tube 1170.

Here, one end portion of the guide tube 1170 may be fixed to one side surface of the end tool 1100, and another end portion thereof may be fixed by being coupled to fixing members 421 and 422 within the bent portion 402. Here, the fixing members 421 and 422 are fixedly coupled to the bent portion 402 and serve to fix the guide tube 1170. In an embodiment, the fixing member 421 may be coupled to one end portion of the guide tube 1170 to fix the one end portion, and the fixing member 422 may be positioned at a certain distance from the fixing member 421 and may be coupled to another point spaced apart from the one end portion of the guide tube 1170 to fix the guide tube 1170. Accordingly, the guide tube 1170 positioned between the fixing member 421 and the fixing member 422 is fixed in place and does not move, so that the linear section may be maintained.

Accordingly, as the cutting-relay block 293 moves toward the distal end side of the manipulation part 200, the blade wire 307 connected to the cutting-relay block 293 also moves and enters the guide tube 1170, and the blade wire 307 may be guided so as not to bend.

In an embodiment, the cutting-relay assembly 290 may further include the cutting restoring elastic member 294. The cutting restoring elastic member 294 may be connected to the cutting-relay block 293 and apply a force to pull the cutting-relay block 293 toward the proximal end side of the manipulation part. For example, one end portion of the cutting restoring elastic member 294 may be coupled to the proximal end side of the manipulation part, and another end portion thereof may be coupled to the cutting-relay block 293. In this case, when the cutting-relay block 293 moves toward the distal end side of the manipulation part, the elastic member may be extended, and a restoring force may be generated to pull the cutting-relay block 293 toward the proximal end side.

One or more cutting restoring elastic members 294 may be provided and, as illustrated in the drawings, may be disposed on opposite side surfaces of the cutting-relay block 293, one on each side surface. However, the concept of the present disclosure is not limited thereto, and the number or arrangement of the cutting restoring elastic members 294 may vary.

In an embodiment, referring to FIGS. 68 to 78, the cutting-relay member 287 may be provided at a connection point between the manipulation part 200 and the bent portion 402. For example, since the cutting-relay member 287 serves to mediate between the cutting-manipulation portion 280 and the cutting-relay assembly 290, the cutting-relay member 287 may be appropriately provided at any position in the manipulation part 200 or the connection part 400, as needed.

The cutting-relay member 287 of the surgical instrument for electrocautery 10 according to an embodiment of the present disclosure may include a first cutting-relay pulley 287a, a second cutting-relay pulley 287b, and a third cutting-relay pulley 287c.

The first cutting-relay pulley 287a, the second cutting-relay pulley 287b, and the third cutting-relay pulley 287c may be pulleys that rotate around a common rotation axis. The third cutting-relay pulley 287c may be positioned at the center of the cutting-relay member 287, and the first cutting-relay pulley 287a and the second cutting-relay pulley 287b may be positioned on opposite sides of the third cutting-relay pulley 287c. That is, the first cutting-relay pulley 287a, the third cutting-relay pulley 287c, and the second cutting-relay pulley 287b may be stacked in sequence, and a rotation shaft may pass through and couple the pulleys. In an embodiment, the first cutting-relay pulley 287a, the second cutting-relay pulley 287b, and the third cutting-relay pulley 287c may rotate integrally in the same direction.

In an embodiment, the first cutting-relay wire 291 and the second cutting-relay wire 292 of the cutting-relay assembly 290 described above may be connected to the third cutting-relay pulley 287c. For example, one end portion of the first cutting-relay wire 291 and one end portion of the second cutting-relay wire 292 may be wound in opposite directions and fixedly coupled to the third cutting-relay pulley 287c. Accordingly, as the third cutting-relay pulley 287c rotates, one of the first cutting-relay wire 291 and the second cutting-relay wire 292 may be wound around the pulley, while another one thereof may be unwound. That is, as the cutting-relay member 287 rotates, the first and second cutting-relay wires 291 and 292 connected thereto may move in opposite directions.

Accordingly, as described above, when the second cutting-relay wire 292, which is coupled to the distal end side of the cutting-relay block 293, is wound around the third cutting-relay pulley 287c, the first cutting-relay wire 291, which is coupled to the proximal end side of the cutting-relay block 293, is unwound from the third cutting-relay pulley 287c. As a result, the second cutting-relay wire 292 pulls the cutting-relay block 293 toward the distal end side of the manipulation part, so that the cutting-relay block 293 is able to move from the proximal end side to the distal end side.

In an embodiment, when the second cutting-relay wire 292 is unwound from the third cutting-relay pulley 287c, the first cutting-relay wire 291 is wound around the third cutting-relay pulley 287c. Accordingly, the first cutting-relay wire 291 pulls the cutting-relay block 293 toward the proximal end side of the manipulation part, thereby allowing the cutting-relay block 293 to move from the distal end side to the proximal end side.

As such, the cutting-relay block 293 may move within the bent portion 402 toward either the proximal end side or the distal end side of the manipulation part in response to the rotation of the cutting-relay member 287.

Hereinafter, the cutting-manipulation portion 280 configured to rotate the cutting-relay member 287 will be described.

The manipulation part 200 of the surgical instrument for electrocautery 10 according to an embodiment of the present disclosure may include the cutting-manipulation portion 280 configured to rotate the cutting-relay member 287. For example, the cutting-manipulation portion 280 may include the cutting lever 281, a cutting-lever rotation shaft 282, a first cutting-lever wire 283, and a second cutting-lever wire 284.

Referring to FIG. 48 and the like, the cutting lever 281 and the cutting-lever rotation shaft 282 may be provided adjacent to the actuation pulley 262. For example, the cutting-lever rotation shaft 282 may be provided in a region that does not interfere with the rotation of the actuation pulley 262 and the actuation lever 261, and may be a shaft parallel to the rotation shaft 241 of the actuation pulley 262. In an embodiment, the cutting lever 281 may be rotatable around the cutting-lever rotation shaft 282, and one end portion of the cutting lever 281 may rotate in a direction approaching or moving away from the actuation lever 261.

The cutting lever 281 may include a body portion 281a and a wire coupling portion 281b. Here, the body portion 281a may be a portion functioning as a handle, and the wire coupling portion 281b may be a portion which is provided to branch from the body portion 281a and to which the cutting-lever wire, which will be described later, is coupled.

In an embodiment, the body portion 281a, which serves as a handle, may be configured to protrude outward from the manipulation part 200 on one side of the cutting lever 281 with respect to a point where the cutting lever 281 is coupled to the cutting-lever rotation shaft 282, and on the opposite side of the cutting lever 281, a structure may be provided to branch out from opposite sides of the body portion 281a and surround opposite surfaces of the actuation pulley 262.

In other words, the wire coupling portion 281b may be configured to extend from the body portion 281a and may include two branched portions that branch from the body portion 281a with respect to a point where the cutting-lever rotation shaft 282 is provided. That is, one wire coupling portion 281b and another wire coupling portion 281b, which are configured to face each other and branch from the body portion 281a, may be provided. Here, one of the branched wire coupling portions 281b may be configured to partially surround one side surface of the actuation pulley 262, and another one thereof may be configured to partially surround the opposite side surface of the actuation pulley 262. In other words, the actuation pulley 262 may be positioned between the wire coupling portions 281b branched from the cutting lever 281.

In an embodiment, the cutting-lever wires may be coupled to the branched portions at another end portion of the cutting lever 281 described above. For example, the first cutting-lever wire 283 and the second cutting-lever wire 284 may be respectively coupled to the branched end portions of the cutting lever 281.

For example, the first cutting-lever wire 283 may be connected to the first cutting-relay wire 291, and the second cutting-lever wire 284 may be connected to the second cutting-relay wire 292.

In other words, one end portion of each of the first cutting-lever wire 283 and the second cutting-lever wire 284 may be coupled to the cutting lever 281, and another end portion of each of the first cutting-lever wire 283 and the second cutting-lever wire 284 may be coupled to the cutting-relay member 287.

In an embodiment, the cutting-manipulation portion 280 may include cutting-lever tubes 285 and 286 that are configured to accommodate at least some of the cutting-lever wires 283 and 284 therein and to be bendable to a certain extent. This will be described in detail later.

Hereinafter, the principle by which the cutting lever 281 rotates the cutting-relay member 287 in response to the rotation of the cutting lever 281 will be described.

The first cutting-lever wire 283 of the cutting-manipulation portion 280 according to an embodiment of the present disclosure may be wound in a first direction around the cutting lever 281 with respect to the cutting-lever rotation shaft 282, and then wound in the first direction around the cutting-relay member 287. The second cutting-lever wire 284 may be wound in a second direction, which is opposite to the first direction, around the cutting lever 281, and then wound in the second direction around the cutting-relay member 287. In an embodiment, it may be said that the first cutting-lever wire 283 may be wound around one of the wire coupling portions 281b, and the second cutting-lever wire 284 may be wound around the other wire coupling portion 281b.

In an embodiment, one end portion of the first cutting-lever wire 283 may be fixed to one side surface of the cutting lever 281 with respect to a plane perpendicular to the cutting-lever rotation shaft 282, and another end portion of the first cutting-lever wire 283 may be fixed to another side surface of the cutting-relay member 287. One end portion of the second cutting-lever wire 284 may be fixed to another side surface of the cutting lever 281 with respect to the plane perpendicular to the cutting-lever rotation shaft 282, and another end portion of the second cutting-lever wire 284 may be fixed to one side surface of the cutting-relay member 287.

For example, one end portion of the first cutting-lever wire 283 may be coupled to one side of the branched end portion of the cutting lever 281, and another end portion thereof may be coupled to the second cutting-relay pulley 287b. For example, one end portion of the second cutting-lever wire 284 may be coupled to another side of the branched end portion of the cutting lever 281, and another end portion thereof may be coupled to the first cutting-relay pulley 287a.

Accordingly, as the cutting lever 281 operates, when the first cutting-lever wire 283 is wound around the cutting-relay member 287, the second cutting-lever wire 284 may be unwound from the cutting-relay member 287, and when the first cutting-lever wire 283 is unwound from the cutting-relay member 287, the second cutting-lever wire 284 may be wound around the cutting-relay member 287.

That is, according to the operation of the cutting lever 281, the first cutting-lever wire 283 and the second cutting-lever wire 284 may move in opposite directions, thereby rotating the cutting-relay member 287.

As described above, the cutting-lever wires 283 and 284 may transmit the rotation of the cutting lever 281 to the cutting-relay member 287, thereby causing a rotational motion of the cutting-relay member 287. For example, as described above, the cutting-relay assembly 290 may convert the rotational motion of the cutting-relay member 287 into a linear motion of the cutting-relay block 293.

In an embodiment, referring to FIGS. 69 and 70, when the cutting lever 281 rotates in the direction of arrow A, the first cutting-lever wire 283 is moved in the direction of arrow B, and the second cutting-lever wire 284 is moved in the direction of arrow C, thereby rotating the cutting-relay member 287 in the direction of arrow D. At this time, when the cutting-relay member 287 rotates in the direction of arrow D, the first cutting-relay wire 291, which is fixedly coupled to the cutting-relay member 287, is moved in the direction of arrow E, and the second cutting-relay wire 292 is moved in the direction of arrow F. Along with this, the cutting-relay block 293, which is fixedly coupled to the first cutting-relay wire 291, is moved in the direction of arrow G. Accordingly, the blade wire 307 fixedly coupled to the cutting-relay block 293 is also moved in the direction of arrow G. At this time, since the blade wire 307 is accommodated in the guide tube 1170, a movement path of the blade wire 307 is guided by the guide tube 1170.

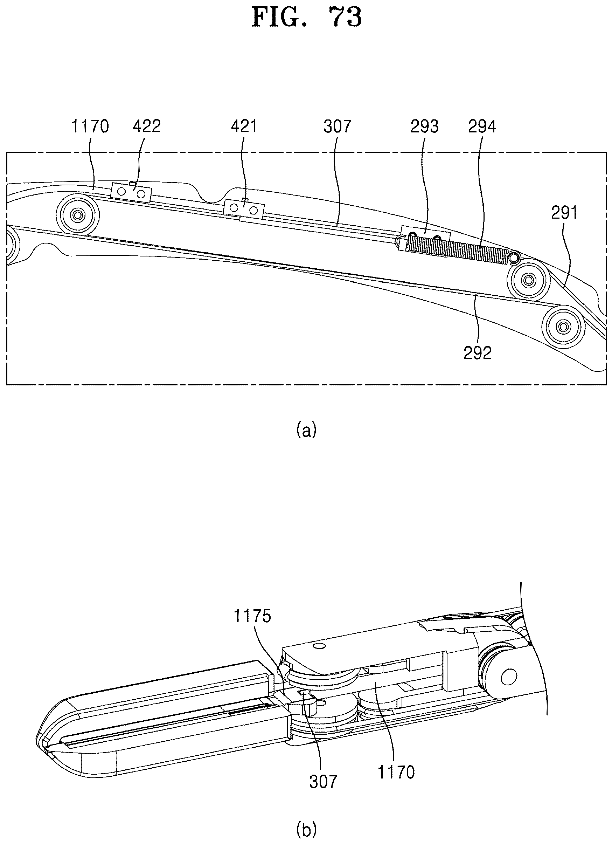

In an embodiment, referring to FIGS. 73 to 76, the cutting-relay assembly 290 may move the cutting-relay block 293 toward the distal end side of the manipulation part in response to the movement of the cutting-relay wires 291 and 292, thereby drawing the blade wire 307 into the guide tube 1170.

Here, as described above, the guide tube 1170 accommodating the blade wire 307 may be connected to the end tool 1100. For example, the blade 1175 may be coupled to an end portion of the blade wire 307.

Accordingly, as the blade wire 307 is moved within the guide tube 1170 by the cutting-relay block 293, the blade wire 307 and the blade 1175 within the end tool 1100 may be moved toward the distal end side of the end tool 1100. That is, through the operation of the cutting lever 281, a cutting motion capable of cutting tissue may be performed by moving the blade 1175 of the end tool 1100.

In an embodiment, the cutting-manipulation portion 280 may further include the cutting-lever tubes 285 and 286, which are configured to accommodate at least portions of the cutting-lever wires 283 and 284 and to be bendable to a certain extent. For example, the first cutting-lever wire 283 may be accommodated in a first cutting-lever tube 285, and the second cutting-lever wire 284 may be accommodated in a second cutting-lever tube 286.

In an embodiment, the cutting-lever wires 283 and 284 may pass through the interiors of the cutting-lever tubes 285 and 286 and be connected to the cutting-relay member 287. For example, the cutting-lever wires 283 and 284 may be configured to be movable along the cutting-lever tubes 285 and 286 within the cutting-lever tubes 285 and 286. For example, when the cutting-lever tubes 285 and 286 are bent to a certain extent, the cutting-lever wires 283 and 284 inside the cutting-lever tubes 285 and 286 may also bend along with the cutting-lever tubes 285 and 286.

In an embodiment, one end portion of each of the cutting-lever tubes 285 and 286 may be fixed to one side of the cutting lever 281, and another end portion of each of the cutting-lever tubes 285 and 286 may be fixed to one side of the cutting-relay member 287. For example, the cutting-lever wires 283 and 284 may be configured to be exposed from the respective end portions of the cutting-lever tubes 285 and 286.

That is, although opposite end portions of each of the cutting-lever tubes 285 and 286 are fixed in position and do not change in length, the cutting-lever wires 283 and 284, which are accommodated in the cutting-lever tubes 285 and 286 and connected to the cutting lever 281 and the cutting-relay member 287, may be movable within the cutting-lever tubes 285 and 286 in response to the rotation of the cutting lever 281.

In an embodiment, referring to FIGS. 77 and 78, the second cutting-lever tube 286 may be fixed to one side of the cutting lever 281 and also to one side of the cutting-relay member 287. Here, the one side of the cutting-relay member 287 to which the second cutting-lever tube 286 is fixed is not a rotating component such as the first cutting-relay pulley 287a, but rather a component fixed to the frame of the manipulation part 200. Accordingly, when the cutting lever 281 rotates as indicated by the arrow, the second cutting-lever wire 284 may move within the second cutting-lever tube 286 and rotate the first cutting-relay pulley 287a.

### (Structure of guide tube)

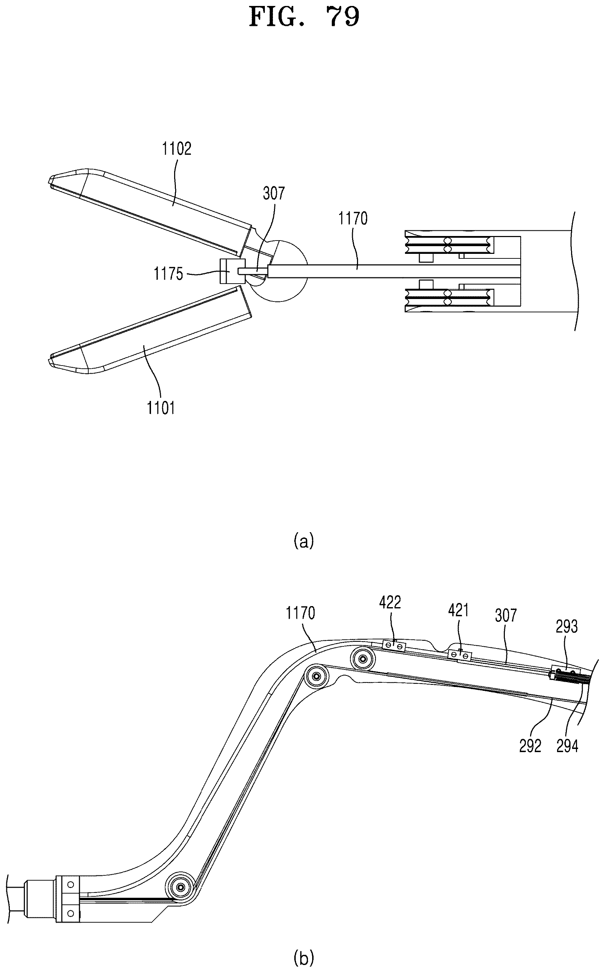

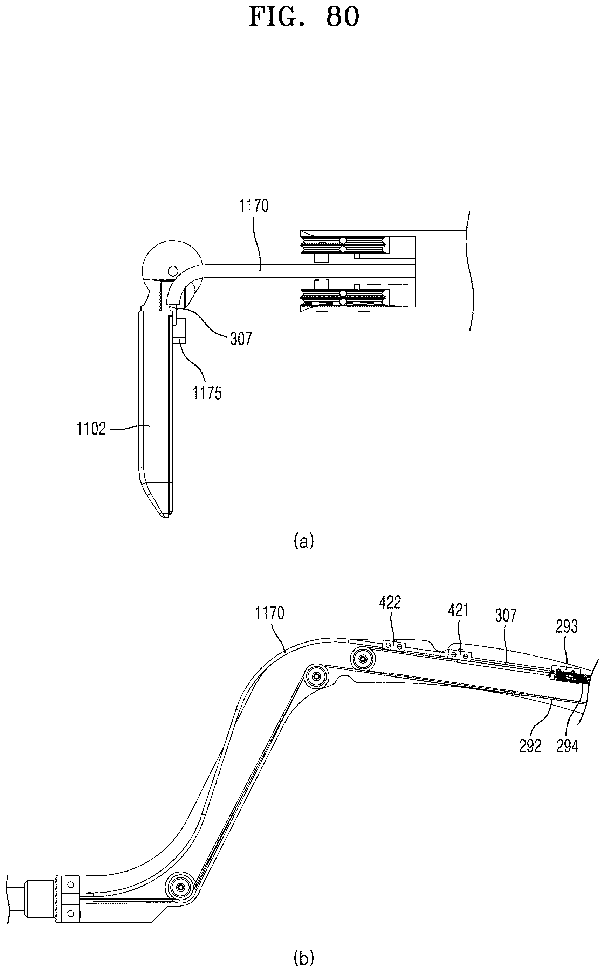

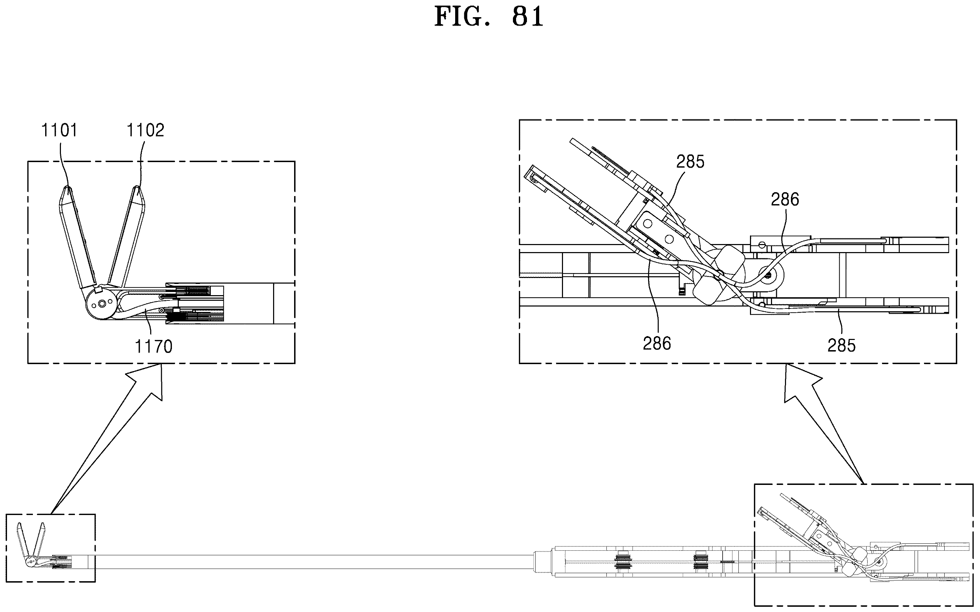

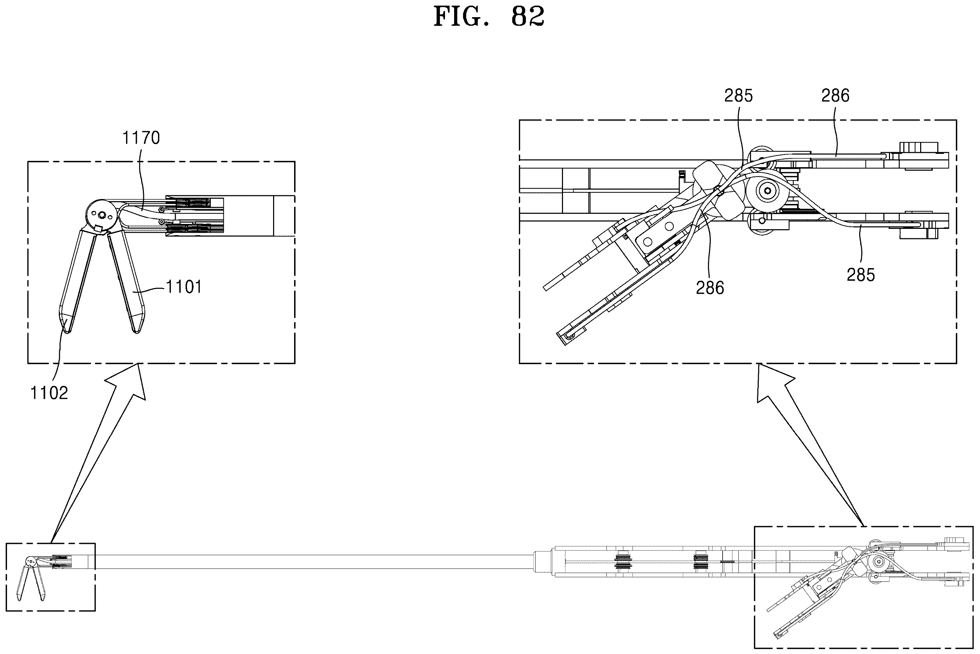

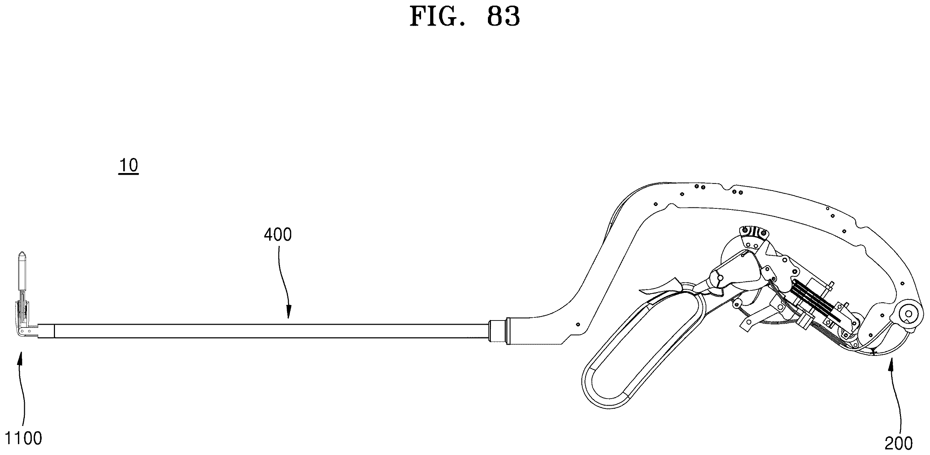

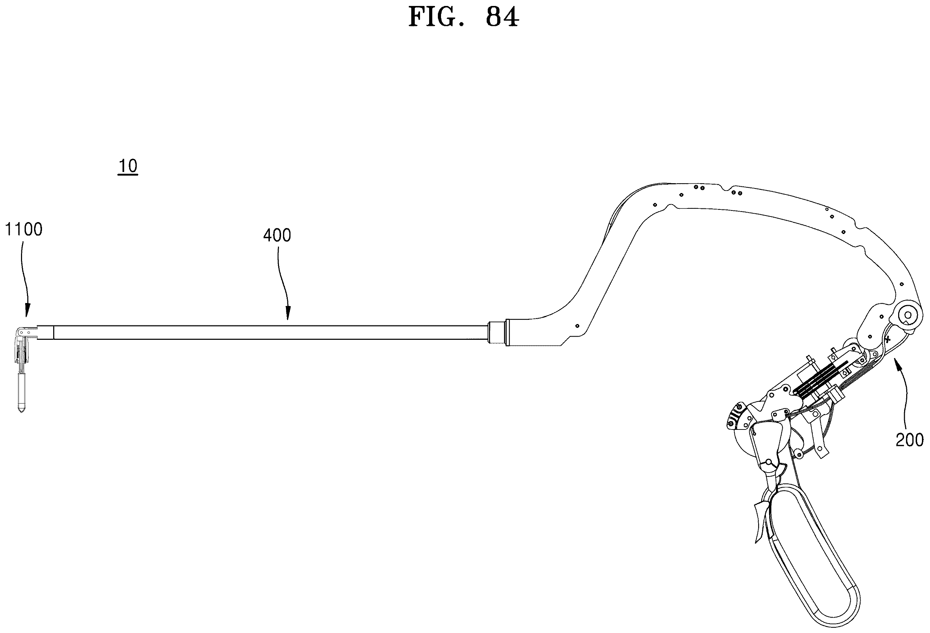

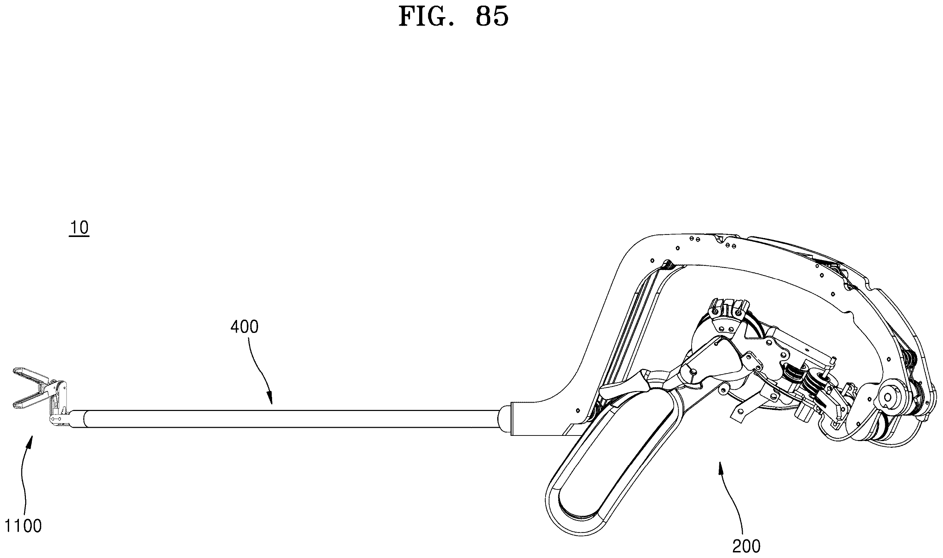

FIGS. 79 and 80 are views illustrating a path of the guide tube in the end tool and the connection part of the surgical instrument for electrocautery shown in FIG. 2. FIG. 81 is a view illustrating a path of the guide tube in the end tool and the manipulation part when the end tool of the surgical instrument for electrocautery shown in FIG. 2 is yaw-rotated by +90°, and FIG. 82 is a view illustrating a path of the guide tube in the end tool and the manipulation part when the end tool of the surgical instrument for electrocautery shown in FIG. 2 is yaw-rotated by -90°. FIGS. 83 and 84 are perspective views illustrating a pitch motion of the surgical instrument for electrocautery shown in FIG. 2, and FIG. 85 is a perspective view illustrating a yaw-pitch combined motion of the surgical instrument for electrocautery shown in FIG. 2.

Referring to FIGS. 79 and 80, one end portion of the guide tube 1170, which accommodates the blade wire 307, may be coupled to the fixing members 421 and 422 of the bent portion 402, and another end portion of the guide tube 1170 may be coupled to the end tool 1100. For example, a portion of the guide tube 1170 between opposite end portions may be provided to be unfixed, such that the portion is movable to a certain extent within the connection part 400. For example, one end of the guide tube 1170 may be fixed to the end tool 1100, and another end thereof may be fixed to a side of the manipulation part 200. The portion of the guide tube 1170 between opposite end portions may be bendable. However, since opposite end portions of the guide tube 1170 are fixed and immovable, it may be said that the total length of the guide tube 1170 between the fixed opposite end portions is constant.

Here, the guide tube 1170 may be provided to have a surplus length within the connection part 400. That is, as shown in FIG. 80, the guide tube 1170 is pulled further toward the end tool 1100 in a neutral state than during a yaw motion of the end tool 1100. Accordingly, the guide tube 1170 needs to be accommodated within the connection part 400 by an amount corresponding to the pulled length.

In an embodiment, referring to FIGS. 81 and 82, the cutting-lever tubes 285 and 286, which accommodate the cutting-lever wires 283 and 284, may cross each other below the yaw rotation shaft 243 of the manipulation part 200. For example, the cutting-lever tubes 285 and 286 may be provided to have a surplus length.

As described above, since one end of each of the cutting-lever tubes 285 and 286 is fixed to the cutting lever 281 and another end thereof is fixed to a fixing portion on one side of the cutting-relay member 287, the total length of each of the cutting-lever tubes 285 and 286 between the opposite end portions of the cutting-lever tubes 285 and 286 remains constant. Accordingly, even when the cutting-lever tubes 285 and 286 are curved or slightly displaced, the length of the cutting-lever wires 283 and 284 accommodated therein remains constant, and the cutting-lever wires 283 and 284 may not move within the cutting-lever tubes 285 and 286.

Accordingly, as illustrated in the drawings, even when the cutting-lever tubes 285 and 286 are pulled or released due to the yaw motion of the end tool 1100 and the rotation of the first handle 204 and the cutting-manipulation portion 280 at the lower end of the yaw rotation shaft 243, the cutting-lever wires 283 and 284 inside the cutting-lever tubes 285 and 286 do not move, and thus do not rotate the cutting-relay member 287. That is, the cutting-relay assembly 290 is not operated, and thus, the blade 1175 in the end tool 1100 does not move.

In other words, the surgical instrument for electrocautery 10 according to an embodiment of the present disclosure may perform a cutting motion independently of a yaw motion of the end tool 1100 without being interfered with by the yaw motion. Similarly, the surgical instrument for electrocautery 10 according to an embodiment of the present disclosure may perform a cutting motion independently of a pitch motion of the end tool 1100 without being interfered with by the pitch motion.

As described with reference to FIG. 2, the actuation-manipulation portion 203, the yaw-manipulation portion 202, and the pitch-manipulation portion 201 are configured such that the respective rotation shafts are positioned at the rear thereof to be identical to the joint configuration of the end tool, so that a user can intuitively perform matching manipulations.

For example, in the surgical instrument for electrocautery 10 according to an embodiment of the present disclosure, the pulleys are provided at respective joint points (the actuation joint, yaw joint, and pitch joint), the wires (the first jaw wire or the second jaw wire) are provided to be wound around the pulleys, the rotational manipulations (actuation rotation, yaw rotation, and pitch rotation) of the manipulation part cause movement of the respective wires, which in turn induces the desired motion of the end tool 1100. Furthermore, the auxiliary pulleys may be provided on one side of the respective pulleys, and these auxiliary pulleys may prevent the wire from being wound around one pulley multiple times, so that the wires wound around the pulley do not come into contact with each other, and paths of the wire being wound around the pulley and the wire being released from the pulley are safely provided, so that safety and efficiency in the transmission of driving force of a wire may be improved.

In an embodiment, as described above, the yaw-manipulation portion 202 and the actuation-manipulation portion 203 are directly provided on the first handle 204. Thus, when the first handle 204 rotates around the rotation shaft 246, the yaw-manipulation portion 202 and the actuation-manipulation portion 203 are also rotated together with the first handle 204. Accordingly, the coordinate systems of the yaw-manipulation portion 202 and the actuation-manipulation portion 203 are not fixed, but continuously change relative to the rotation of the first handle 204. That is, in FIG. 2 or the like, the yaw-manipulation portion 202 and the actuation-manipulation portion 203 are illustrated as being parallel to the z-axis. However, when the first handle 204 rotates, the yaw-manipulation portion 202 and the actuation-manipulation portion 203 are not parallel to the Z-axis any longer. That is, the coordinate systems of the yaw-manipulation portion 202 and the actuation-manipulation portion 203 change according to the rotation of the first handle 204. However, in the present specification, for convenience of description, unless described otherwise, the coordinate systems of the yaw-manipulation portion 202 and the actuation-manipulation portion 203 are described on the basis of a state in which the first handle 204 is positioned perpendicular to the connection part 400 as shown in FIG. 2.

### (Pitch, yaw, and cutting motions of end tool)

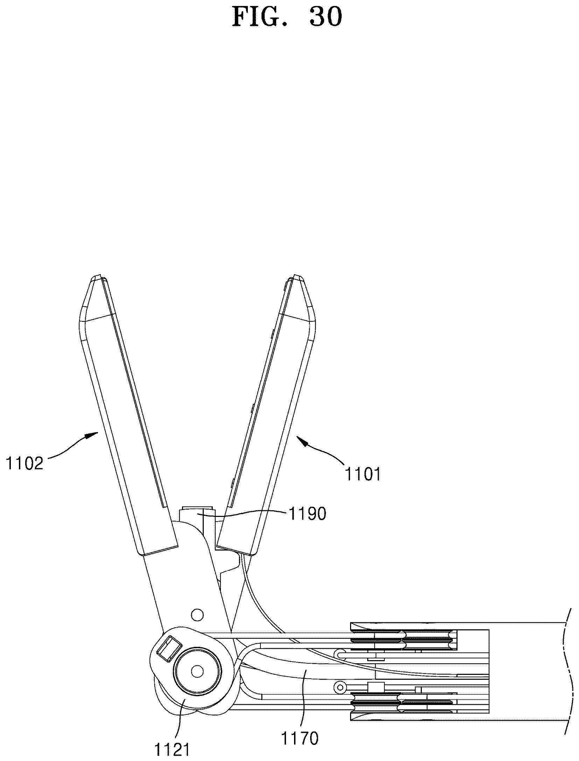

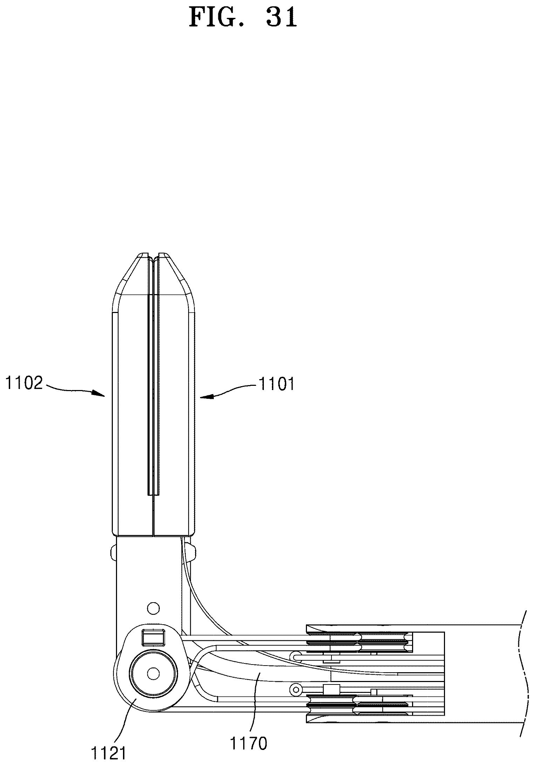

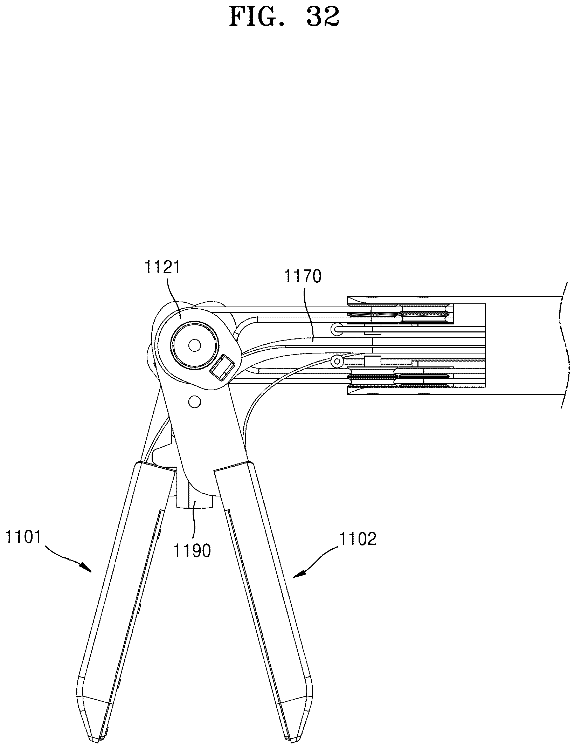

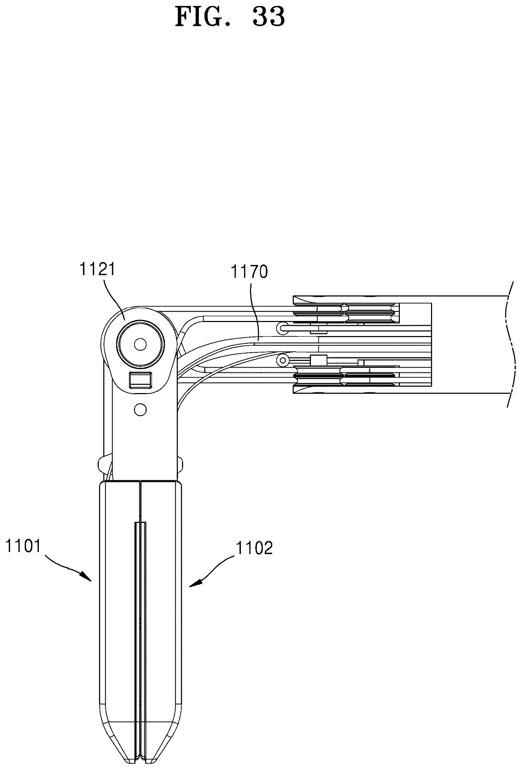

FIGS. 30 and 31 are views illustrating a process of performing an opening and closing motion in a state in which the end tool of the surgical instrument for electrocautery of FIG. 2 is yaw-rotated by +90°. In an embodiment, FIGS. 32 and 33 are views illustrating a process of performing an opening and closing motion in a state in which the end tool of the surgical instrument for electrocautery of FIG. 2 is yaw-rotated by -90°.

As shown in FIGS. 30 to 33, the end tool of the surgical instrument for electrocautery according to an embodiment of the present disclosure is configured to normally perform an opening and closing motion, that is, an actuation motion even in a state in which the jaws are yaw-rotated by +90° or -90°.

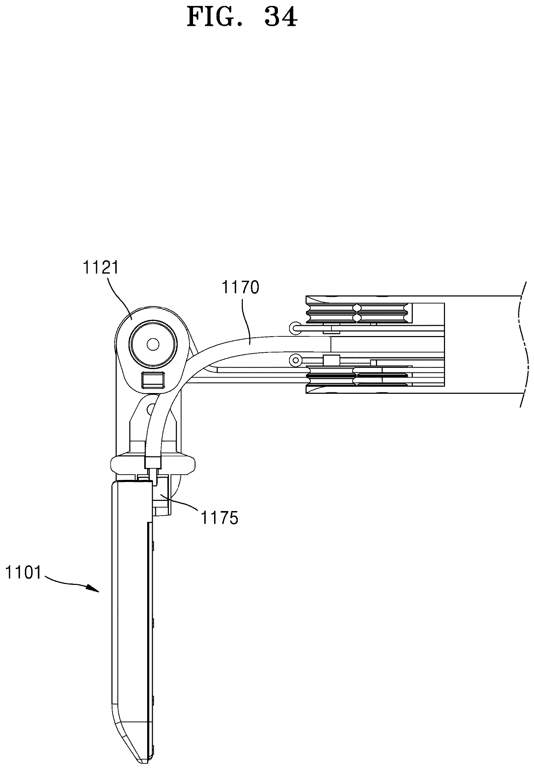

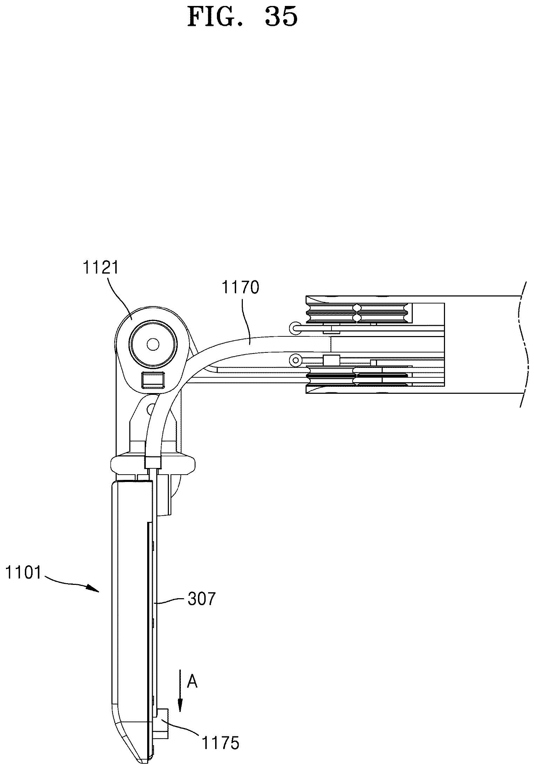

FIGS. 34 and 35 are views illustrating a process of performing a cutting motion in a state in which the end tool of the surgical instrument for electrocautery of FIG. 2 is yaw-rotated by +90°.

As shown in FIGS. 34 and 35, the end tool of the surgical instrument for electrocautery according to an embodiment of the present disclosure is configured to normally perform a cutting motion even in a state in which the jaws are yaw-rotated by +90°.

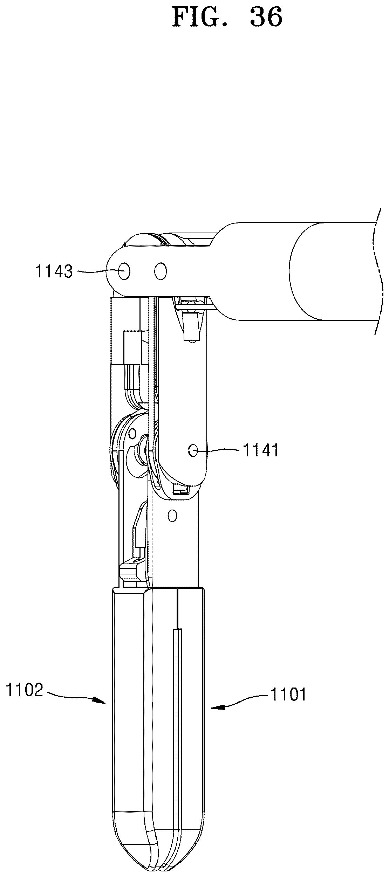

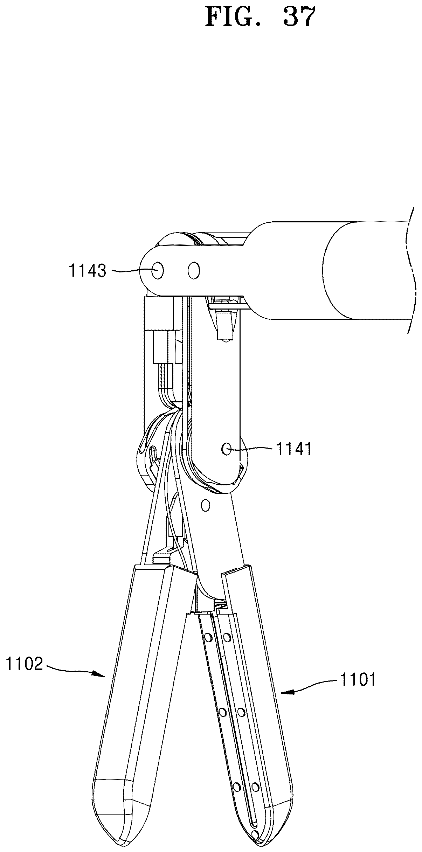

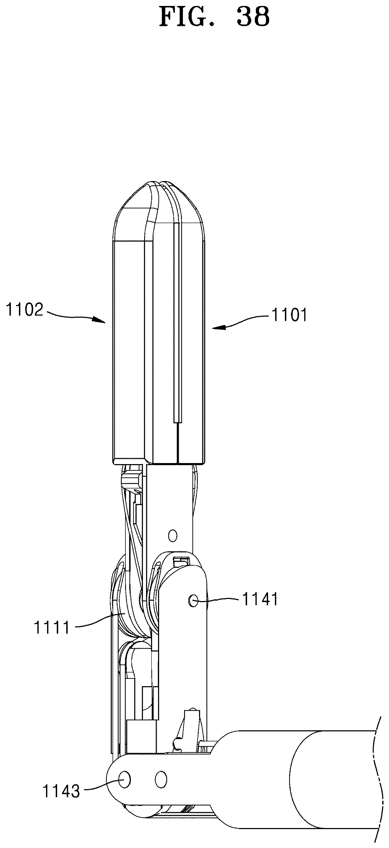

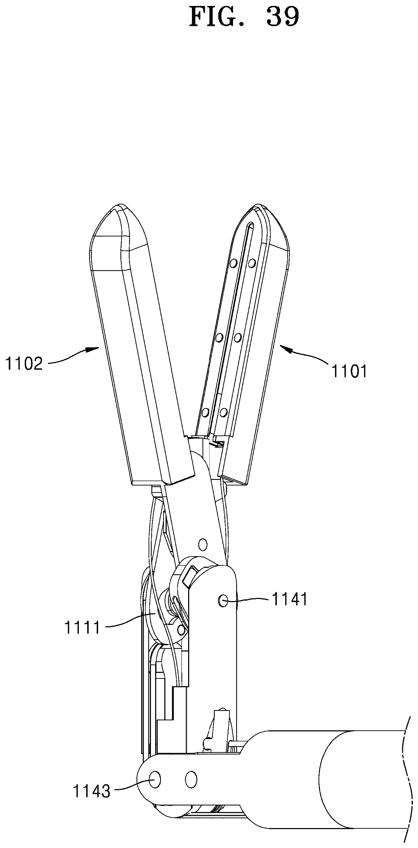

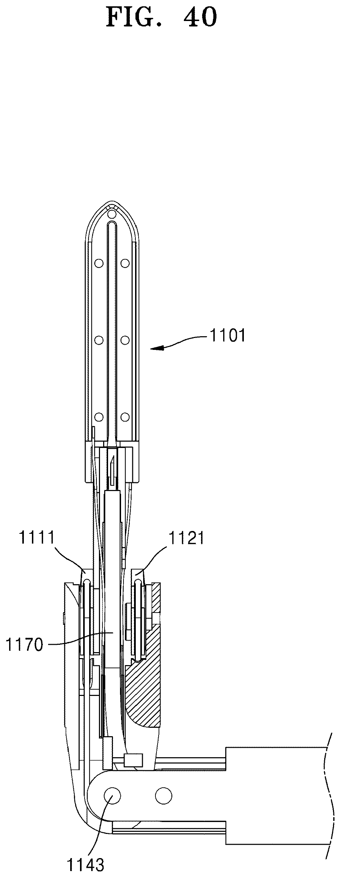

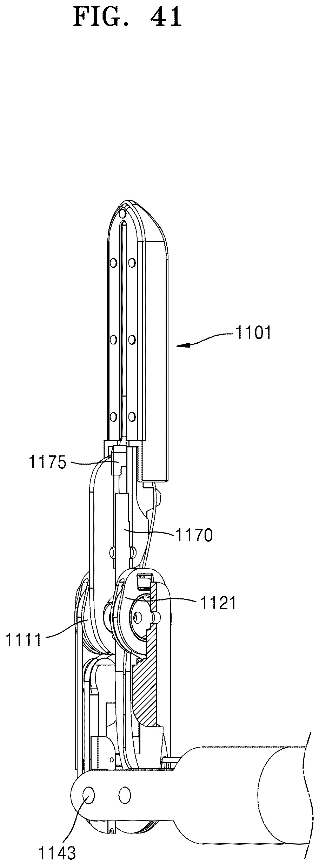

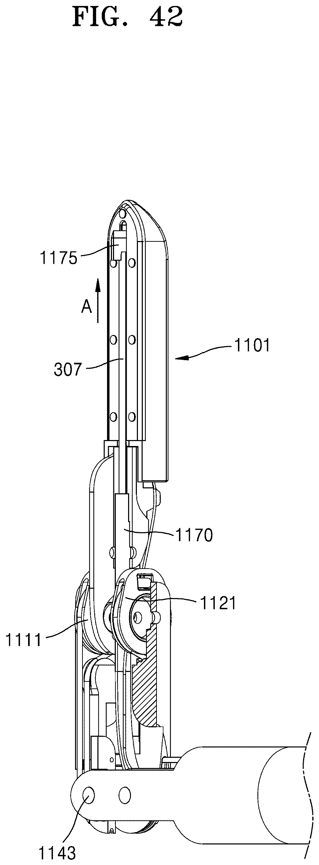

FIGS. 36 and 37 are views illustrating a process of performing an opening and closing motion in a state in which the end tool of the surgical instrument for electrocautery of FIG. 2 is pitch-rotated by +90°. FIGS. 38 and 39 are views illustrating a process of performing an opening and closing motion in a state in which the end tool of the surgical instrument for electrocautery of FIG. 2 is pitch-rotated by -90°. FIG. 40 is a cutaway perspective view of the end tool of the surgical instrument for electrocautery of FIG. 38. FIGS. 41 and 42 are views illustrating a process of performing a cutting motion in a state in which the end tool of the surgical instrument for electrocautery of FIG. 2 is pitch-rotated by -90°.

As shown in FIGS. 36 to 42, the end tool of the surgical instrument for electrocautery according to an embodiment of the present disclosure is configured to normally perform a cutting motion even in a state in which the jaws are pitch-rotated by -90°.

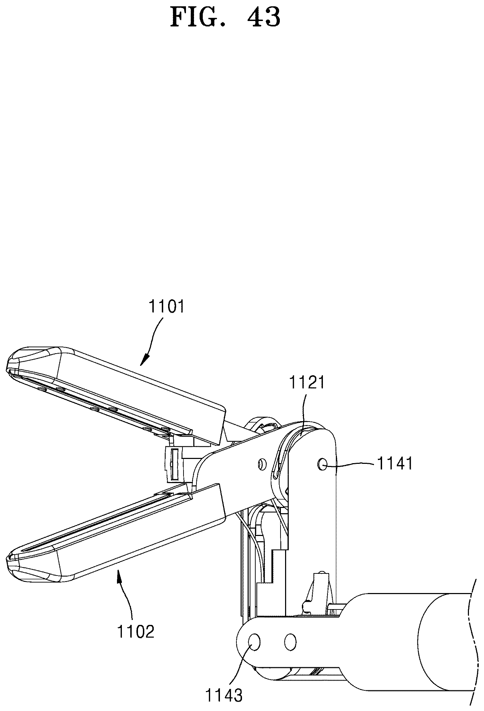

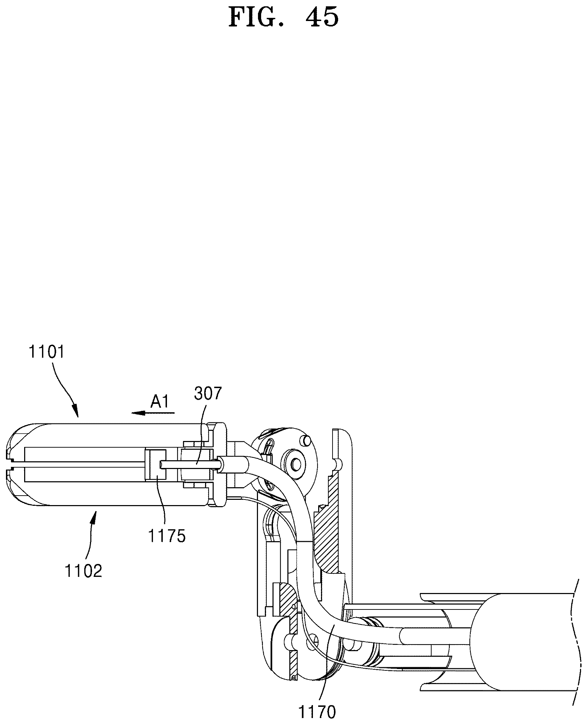

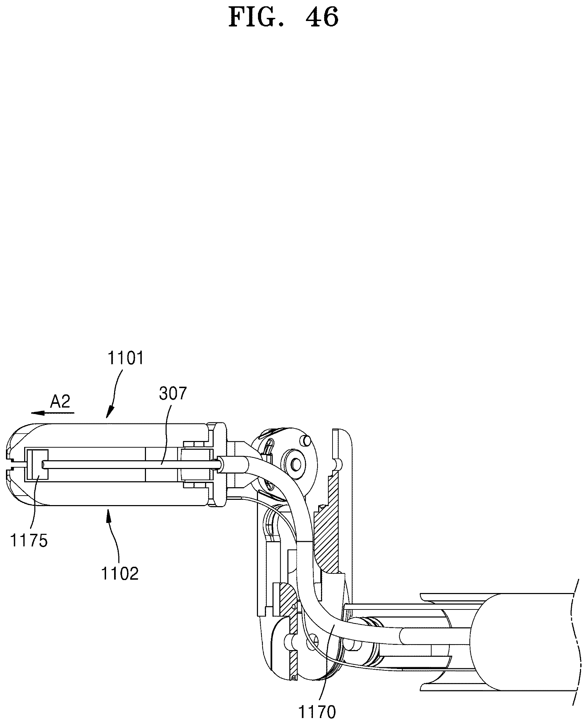

FIG. 43 is a view illustrating a state in which the jaws are pitch-rotated by -90° and simultaneously yaw-rotated by +90°, and FIGS. 45 and 46 are perspective views illustrating a cutting motion of the end tool of the surgical instrument for electrocautery of FIG. 2 and illustrate a state of performing a cutting motion in a state in which the jaws are pitch-rotated by -90° and simultaneously yaw-rotated by +90°.

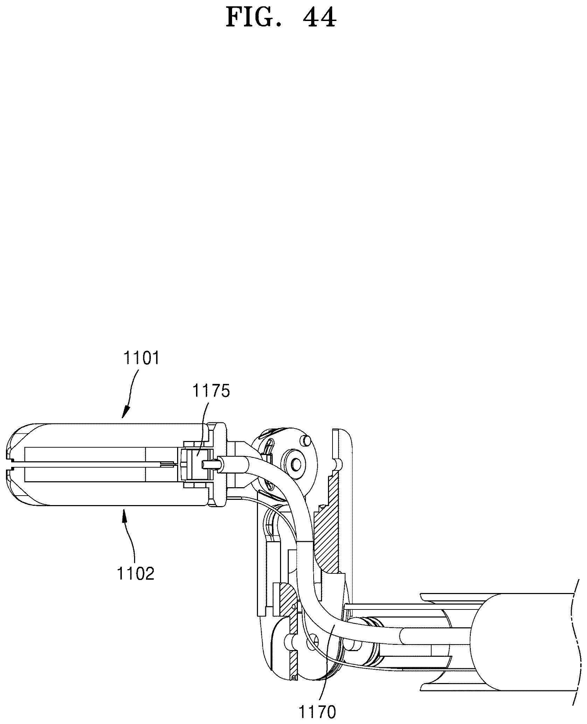

As shown in FIGS. 43 to 46, the end tool of the surgical instrument for electrocautery according to an embodiment of the present disclosure is configured to normally perform a cutting motion even in a state in which the jaws are pitch-rotated by -90° and simultaneously yaw-rotated by +90°.

According to the present disclosure, a manipulation direction of a manipulation part by an operator and an operating direction of an end tool are intuitively aligned with each other, so that the operator's convenience can be improved, and the accuracy, reliability, and speed of surgery can be improved.

The present disclosure relates to a surgical instrument for electrocautery, and more particularly, to a surgical instrument that may be mounted on a robot arm or manually operated for use in laparoscopic surgery or various other surgeries, and that may be used as a surgical instrument for electrocautery with improved insulation performance.

The present disclosure has been described above with a focus on exemplary embodiments. It will be understood by those skilled in the art that various changes in form and details may be made therein without departing from the essential features of the present disclosure. Therefore, the disclosed embodiments should be considered in descriptive sense only and not for purposes of limitation. The scope of the present disclosure is defined not by the detailed description of the disclosure but by the appended claims, and all differences within the scope will be construed as being included in the present disclosure.

## Claims

1. A surgical instrument comprising:
an end tool including a first jaw and a second jaw, each configured to be rotatable, the end tool being configured to be rotatable in two or more directions;
a manipulation part configured to control the rotation of the end tool in the two or more directions;
a power transmission part including a first jaw wire connected to the manipulation part and configured to transmit a rotation of the manipulation part to the first jaw, and a second jaw wire connected to the manipulation part and configured to transmit the rotation of the manipulation part to the second jaw; and
a connection part configured to extend in a first direction (X-axis) and having one end portion to which the end tool is coupled and another end portion to which the manipulation part is coupled, thereby connecting the manipulation part to the end tool,
wherein the end tool further includes:
a first jaw pulley coupled to the first jaw and configured to be rotatable around a first rotation shaft;
a second jaw pulley coupled to the second jaw and configured to be rotatable around a shaft that is substantially identical to or parallel to the first rotation shaft; and
a blade assembly including a blade that moves between a proximal end and a distal end of the end tool, and positioned adjacent to the first jaw pulley or the second jaw pulley,
the power transmission part further includes a blade wire, at least a portion of which is in contact with the blade assembly, to transmit, to the blade, a driving force required to move the blade,
the manipulation part includes a cutting-manipulation portion including a cutting lever configured to rotate around a rotation shaft, and
opposite end portions of the blade wire are respectively connected to the cutting-manipulation portion and the blade, such that a rotational motion of the cutting-manipulation portion is converted into a linear motion of the blade through the blade wire.

2. The surgical instrument of claim 1, wherein
the cutting-manipulation portion includes:
a cutting-relay member configured to rotate around a rotation shaft different from the rotation shaft around which the cutting lever rotates and connected to the cutting lever by a cutting-lever wire; and
a cutting-relay assembly configured to be connected to the cutting-relay member and to convert a rotational motion of the cutting-relay member into a linear motion, and
the rotational motion of the cutting-manipulation portion is converted into a linear motion of the blade wire through the cutting-relay assembly.

3. The surgical instrument of claim 2, wherein the cutting-lever wire generates the rotational motion of the cutting-relay member by transmitting a rotation of the cutting lever to the cutting-relay member.

4. The surgical instrument of claim 3, wherein the cutting-manipulation portion further includes a cutting-lever tube configured to internally accommodate at least a portion of the cutting-lever wire and to be bendable to a certain extent.

5. The surgical instrument of claim 4, wherein
the cutting-lever tube has one end portion fixed to one side of the cutting lever, and another end portion fixed to one side of the cutting-relay member, and
the cutting-lever wire is further configured to be movable along and within the cutting-lever tube.

6. The surgical instrument of claim 3, wherein
the cutting-lever wire includes a first cutting-lever wire and a second cutting-lever wire,
the first cutting-lever wire is wound in the first direction around a cutting-lever rotation shaft and then wound in the first direction around the cutting-relay member, and
the second cutting-lever wire is wound in a second direction, which is a direction opposite to the first direction, around the cutting-lever rotation shaft and then wound in the second direction around the cutting-relay member.

7. The surgical instrument of claim 6, wherein
according to an operation of the cutting lever,
when the first cutting-lever wire is wound around the cutting-relay member, the second cutting-lever wire is unwound from the cutting-relay member, and
when the first cutting-lever wire is unwound from the cutting-relay member, the second cutting-lever wire is wound around the cutting-relay member.

8. The surgical instrument of claim 2, wherein
the cutting-relay assembly includes:
a cutting-relay wire connected to the cutting-relay member; and
a cutting-relay block coupled to the cutting-relay wire at at least one region, and
one end portion of the cutting-relay wire is fixed to the cutting-relay member, and at least one region of the cutting-relay wire is connected to a pulley, thereby forming a loop-shaped path.

9. The surgical instrument of claim 8, wherein the cutting-relay wire has a movement path including at least one linear section along which the cutting-relay wire moves in a straight-line direction.

10. The surgical instrument of claim 9, wherein the cutting-relay block moves within the linear section toward either a distal end side or a proximal end side of the manipulation part.

11. The surgical instrument of claim 10, wherein the blade assembly further includes a guide tube positioned in the connection part and configured to be bendable to a certain extent, and at least a portion of the blade wire is accommodated in the guide tube.

12. The surgical instrument of claim 11, wherein the blade wire passes through an interior of the guide tube, and has one end portion connected to one surface of the cutting-relay block at the distal end side and another end portion connected to the blade.

13. The surgical instrument of claim 11, wherein a movement path of the blade wire is guided by the guide tube.

14. The surgical instrument of claim 11, wherein the guide tube has opposite ends respectively fixed to the end tool and the manipulation part, and is bendable between the opposite ends.

15. The surgical instrument of claim 1, wherein the blade moves linearly in a direction in which the connection part extends.

16. The surgical instrument of claim 1, wherein, the end tool is configured, in response to a control of the manipulation part, to perform a pitch motion, a yaw motion, and an actuation motion, and to linearly move the blade.

17. The surgical instrument of claim 16, wherein the end tool is configured to perform at least one motion among the pitch motion, the yaw motion, and the actuation motion, and to linearly move the blade either independently of or simultaneously with the at least one motion.

18. The surgical instrument of claim 1, further comprising:
a guide tube configured to internally accommodate at least a portion of the blade wire and to be bendable to a certain extent; and
an actuation hub positioned between the first jaw and the second jaw, coupled to the first jaw and the second jaw, and having a hollow provided therein to which one end portion of the guide tube is coupled.

19. The surgical instrument of claim 18, wherein the guide tube and the at least a portion of the blade wire accommodated therein are configured to pass through the hollow provided in the actuation hub.

20. The surgical instrument of claim 18, wherein the guide tube is not directly coupled to the first jaw or the second jaw.

21. The surgical instrument of claim 1, wherein
the first jaw and the second jaw are positioned to cross each other about a first point serving as a rotation center, and
rotation centers of the first jaw pulley and the second jaw pulley are provided at a second point that is positioned to be spaced apart from the first point.

22. The surgical instrument of claim 21, wherein the first point is positioned more distally than the second point on the first jaw and the second jaw.

23. The surgical instrument of claim 21, wherein
the first rotation shaft includes a first sub-shaft and a second sub-shaft,
the first sub-shaft and the second sub-shaft are positioned to be spaced apart from each other to a certain extent, and
the blade assembly is partially accommodated between the first sub-shaft and the second sub-shaft.

24. The surgical instrument of claim 1, wherein
the first jaw and the second jaw perform an actuation motion by rotating around an actuation rotation shaft of the end tool, and
the manipulation part further includes an actuation-manipulation portion configured to control an actuation motion of the end tool,
wherein the actuation-manipulation portion includes:
an actuation pulley configured to rotate around one actuation rotation shaft; and
an actuation lever coupled to the actuation pulley and configured to rotate integrally with the actuation pulley, and
the one actuation rotation shaft of the manipulation part is not parallel to the actuation rotation shaft of the end tool.

25. The surgical instrument of claim 24, wherein
the first jaw wire and the second jaw wire are wound around and fixed to the actuation pulley, and
the first jaw and the second jaw rotate in opposite directions in response to the rotation of the actuation pulley.

26. The surgical instrument of claim 25, wherein
each of the first jaw wire and the second jaw wire includes a pair of wire strands,
one of the pair of wire strands of the first jaw wire has one end portion wound in the first direction around the actuation pulley and another end portion wound in a third direction around the first jaw pulley,
the other of the pair of wire strands of the first jaw wire has one end portion wound in a second direction, which is opposite to the first direction, around the actuation pulley and another end portion wound in a fourth direction, which is opposite to the third direction, around the first jaw pulley,
one of the pair of wire strands of the second jaw wire has one end portion wound in the second direction around the actuation pulley and another end portion wound in the third direction around the second jaw pulley, and
the other of the pair of wire strands of the second jaw wire has one end portion wound in the first direction around the actuation pulley and another end portion wound in the fourth direction around the second jaw pulley.

27. The surgical instrument of claim 1, wherein the manipulation part further includes a sealing-manipulation portion provided on one side of the cutting-manipulation portion and configured to be rotatable around one rotation shaft.

28. The surgical instrument of claim 27, wherein
the sealing-manipulation portion includes a sealing button configured to protrude from one end portion thereof, and
when the sealing button is pressed, a contact portion provided at another end portion of the sealing-manipulation portion comes into contact with another contact portion within the manipulation part, thereby transmitting an electrical signal.

29. The surgical instrument of claim 27, wherein
a first electrode is provided on a surface of the first jaw facing the second jaw, and
a second electrode is provided on a surface of the second jaw facing the first jaw.

30. The surgical instrument of claim 29, wherein cauterization of a tissue is performed as a current flows through the first electrode and the second electrode.

31. The surgical instrument of claim 30, wherein, after completion of the cauterization, the blade wire moves, and accordingly, the blade moves from a proximal end side toward a distal end side of the first jaw to cut the tissue.
